# EUROPÄISCHE PATENTSCHRIFT

(11) **EP 3 931 175 B1**
(45) Veröffentlichungstag und Bekanntmachung des Hinweises auf die Patenterteilung: **10.04.2024**
(21) Anmeldenummer: 20705371.1
(22) Anmeldetag: 21.02.2020
(51) Int. Cl.: C07C 67/54, C07C 67/303, C07C 69/80, C07C 69/75

(54) **VERFAHREN ZUR AUFARBEITUNG VON BENZOLPOLYCARBONSÄUREESTERN UND DEREN VERWENDUNG ZUR HERSTELLUNG VON CYCLOHEXANPOLYCARBONSÄUREESTERN**
METHOD FOR PROCESSING BENZENE POLYCARBOXYLIC ACID ESTERS AND USE OF SAME TO PRODUCE CYCLOHEXANE POLYCARBOXYLIC ACID ESTERS
PROCÉDÉ POUR LE RETRAITEMENT D'ESTERS D'ACIDE BENZÈNEPOLYCARBOXYLIQUE ET LEUR UTILISATION POUR LA PRODUCTION D'ESTERS D'ACIDE CYCLOHEXANEPOLYCARBOXYLIQUE

(30) Priorität: 25.02.2019 EP 19159145
(43) Veröffentlichungstag der Anmeldung: 05.01.2022
(73) Patentinhaber: BASF SE, 67056 Ludwigshafen am Rhein (DE)
(72) Erfinder: MAKARCZYK, Piotr, 67056 Ludwigshafen (DE); BREITSCHEIDEL, Boris, 67056 Ludwigshafen (DE); SZARKA, Zsolt Jozsef, 67056 Ludwigshafen (DE); JUDAT, Sonja, 67056 Ludwigshafen (DE)
(74) Vertreter: BASF IP Association
(86) Internationale Anmeldenummer: PCT/EP2020/054583
(87) Internationale Veröffentlichungsnummer: WO 2020/173818

(56) Entgegenhaltungen:
- EP-A1- 3 214 067
- WO-A1-99/32427
- WO-A1-2010/076192
- GB-A- 1 302 146

## Beschreibung

### HINTERGRUND DER ERFINDUNG

Die vorliegende Erfindung betrifft ein Verfahren zur Aufreinigung von Benzolpolycarbonsäureestern und zur Herstellung von Cyclohexanpolycarbonsäureestern durch Hydrierung der aufgereinigten Benzolpolycarbonsäureester.

### STAND DER TECHNIK

Während Benzolpolycarbonsäureester in der Vergangenheit in großem Umfang als Weichmacher in Kunststoffen, wie beispielsweise PVC, eingesetzt wurden, versucht man diese zunehmend zu ersetzen, da insbesondere die Phthalat-Weichmacher im Verdacht stehen, gesundheitsschädlich zu sein. Dies gilt vor allem für Kunststoffe, die in sensiblen Anwendungsbereichen, wie Kinderspielzeug, Lebensmittelverpackungen oder medizinischen Artikeln, zum Einsatz kommen. Da Cyclohexanpolycarbonsäureester im Gegensatz zu ihren unhydrierten aromatischen Analoga toxikologisch unbedenklich sind, eignen sie sich besonders gut als alternative Weichmacher für diese sensiblen Anwendungen. Somit dienen Benzolpolycarbonsäureester heute auch als wichtige Zwischenstufe zur Herstellung von Cyclohexanpolycarbonsäureestern. Es besteht weiterhin Bedarf an neuen Verfahren, die die Bereitstellung von Cyclohexanpolycarbonsäureestern mit einem geringen Gehalt an Nebenprodukten, insbesondere an schwerer flüchtigen Nebenprodukten, wie Ethern, ermöglichen.

Die Herstellung von Benzolpolycarbonsäureestern erfolgt vielfach als direkte Veresterung von Carbonsäuren mit Alkoholen unter Wasserabspaltung in Gegenwart eines Veresterungskatalysators. Geeignete Veresterungskatalysatoren sind Brönsted-Säuren, wie Schwefelsäure, alpha-Naphthalinsulfonsäure, Phosphorsäure, Methansulfonsäure und Toluolsulfonsäure sowie Lewissäuren, wie Alkoholate, Carboxylate und Chelatverbindungen von Titan, Zirkonium, Zinn, Aluminium und Zink. Beim Einsatz von Brönsted-Säuren als Katalysatoren ist die Reaktionstemperatur der Veresterung nach oben begrenzt. So sollten bei der Veresterung mit Schwefelsäure in der Regel Temperaturen von über 140 °C nicht überschritten werden, um ein Verfärben des Produktes zu vermeiden. Im Einzelfall ist es möglich, die Veresterung bei Temperaturen bis zu 165 °C durchzuführen, um eine akzeptable Produktqualität zu erzielen, wobei allerdings als Nebenreaktion die säurekatalysierte Dehydratisierung des Alkohols zunimmt. Neben der Veresterung kommt es so mit zunehmenden Temperaturen auch zur Bildung der symmetrischen Ether und schließlich unter Wassereliminierung zu Alkenen.

Bei Erhöhung der Temperatur auf etwa 175 °C bis 200 °C nimmt zwar die Umsetzungsgeschwindigkeit erheblich zu, jedoch lassen sich Qualitätsverschlechterungen, bedingt durch vermehrte Nebenreaktionen, wie z. B. die Etherbildung, nicht vermeiden. Die Verwendung von neutralen Katalysatoren, wie z. B. von Titanaten oder Zirkonaten, hat den Vorteil einer geringeren Alkoholzersetzung und Etherbildung. Aufgrund der geringeren Aktivität im Vergleich zu den Brönsted Säuren müssen jedoch in der Regel höhere Veresterungstemperaturen von z. B. über 180 °C angewandt werden. Daher werden diese Katalysatoren meistens bei der Herstellung der langkettigen Benzolpolycarbonsäureester eingesetzt. Aufgrund der hohen Siedepunkte der langkettigen Alkohole ist es möglich, Veresterungstemperaturen von über 200 °C zu erreichen, ohne dass die Notwendigkeit besteht, die Reaktion unter erhöhtem Druck durchzuführen. So reagieren z. B. Titanate von kurzkettigen Alkoholen, wie Titanisopropoxid, rasch mit freien höheren Alkoholen in einer Umesterungsreaktion zu den entsprechenden Orthotitanaten, z. B. mit Isononanol (Trimethyl-3,5,5-hexanol) zu Tetraisononyltitanat, wobei freiwerdendes Isopropanol abdestilliert wird. Die dabei gebildete Lewis-Säure überträgt den Isononanolrest unter Esterbildung mit einer Carboxygruppe der eingesetzten Carbonsäure und reagiert mit freiem Isononanol zurück zum Tetraisononyltitanat. Bei höheren Temperaturen von z. B. über 200 °C nehmen aber auch mit neutralen Katalysatoren, wie den Titanaten, die Dehydratisierung des Alkohols sowie die weiteren Nebenreaktionen, wie die Bildung von Ethern und Alkenen, zu. Alternativ kann die Herstellung von Benzoldicarbonsäureestern durch Umsetzung von entsprechenden Carbonsäureanhydriden mit Alkoholen erfolgen. Wird die Veresterung zweistufig ausgestaltet, kann zumindest auf der ersten Veresterungsstufe zur Bildung der Monoester auf den Einsatz eines Veresterungskatalysators verzichtet werden.

Es ist bekannt, niedriger als der Alkohol siedende Verunreinigungen bereits im Verlauf der Veresterung aus dem Reaktionsgemisch abzutrennen. Die WO 2010/076192 beschreibt ein Verfahren zur Herstellung von Carbonsäureestern durch Umsetzung einer Carbonsäure oder eines Carbonsäureanhydrids mit einem Alkohol, wobei man das bei der Reaktion gebildete Wasser als Alkohol-Wasser-Azeotrop mit dem Brüden abdestilliert, den Brüden zumindest teilweise kondensiert, das Kondensat in eine wässrige Phase und eine organische Phase trennt, aus der organischen Phase niedriger als der Alkohol siedende Komponenten (Leichtsieder) zumindest teilweise entfernt und die an Leichtsiedern abgereicherte organische Phase zumindest teilweise in das Reaktionssystem zurückführt. Bei den Leichtsiedern handelt es sich dabei hauptsächlich um Olefine, die durch Wasserabspaltung aus dem eingesetzten Alkohol gebildet werden. Während die destillative Abtrennung und Ausschleusung der Leichtsieder aus dem Alkoholrückführstrom für die meisten Alkohole möglich ist, verbleiben die höhermolekularen und somit schwerer flüchtigen Nebenkomponenten, wie die Ether, als Verunreinigung im Produkt.

Das bei der großtechnischen Herstellung von Benzolpolycarbonsäureestern anfallende rohe Veresterungsprodukt wird üblicherweise mehreren Aufarbeitungsschritten unterzogen. Dazu zählt die Abtrennung von überschüssigem Alkohol, die in der Regel durch Verdampfen erfolgt, und des zur Veresterung eingesetzten Katalysators. Brönstedsäure-Katalysatoren werden durch Zugabe von wässriger Base, wie NaOH oder KOH, neutralisiert. Neutrale Katalysatoren, wie Titanalkoxide, Zirkonalkoxide, etc., werden ebenfalls durch Zugabe einer wässrigen basischen Lösung hydrolysiert, wobei sich gegebenenfalls schwerlösliche Metallsalze und/oder Metallalkoxide bilden können. Die Abtrennung des deaktivierten Katalysators kann extraktiv erfolgen, wobei das mit der wässrigen Base behandelte rohe Veresterungsprodukt solange stehen gelassen wird, bis dieses durch mechanisches Absetzen in zwei Phasen zerfällt (eine wässrige Phase, die wenigstens einen Teil des hydrolysierten Katalysators enthält und eine organische Phase, die die Hauptmenge des Veresterungsprodukts enthält), die getrennt abgezogen werden können. Die nach der extraktiven Auftrennung erhaltene organische Phase wird gegebenenfalls durch einmaliges oder mehrfaches Waschen mit einer sauren wässrigen Lösung und/oder mit Wasser neutralisiert. Bei der Hydrolyse neutraler Katalysatoren entstandene Feststoffe können von der flüssigen Produktphase z. B. mittels Sedimentation und/oder Filtration abgetrennt werden. Es resultiert ein neutraler, an Alkohol abgereicherter Rohester, der wenigstens einem zusätzlichen Reinigungsschritt unterzogen werden kann, damit das Rohprodukt von Restfeuchte und Restalkohol befreit wird. Dabei werden die leichtsiedenden Komponenten wie Wasser und Alkohol verdampft, während das schwerflüchtige Esterprodukt als Sumpfaustrag oder Rückstand gewonnen wird. Dieser Aufarbeitungsschritt kann z. B. in einem Kessel unter Vakuum, in einer Strippvorrichtung durch Behandlung mit einem Strippmittel, wie Dampf oder Stickstoff, in einem Fallfilmverdampfer, Dünnschichtverdampfer, in einer Vakuumdampfdestillationskolonne oder ähnlichem erfolgen. Der so erhaltene Benzolpolycarbonsäureester kann gewünschtenfalls mit einem festen Adsorptionsmittel, wie Kohle, Kieselgel oder Tonmineralien in Kontakt gebracht werden, z. B. um eine weitere Verbesserung der Farbzahl zu erzielen.

H. Suter beschreibt in Phthalsäureanhydrid und seine Verwendung, Wissenschaftliche Forschungsberichte, Reihe II: Anwendungstechnik und angewandte Wissenschaft, Dr. Dietrich Steinkopf Verlag, Darmstadt, 1972, Seiten 79 - 80 und Abb. 16 ein Verfahren der BASF zur Herstellung von Phthalatweichmachern, wie Di-2-ethylhexylphthalat (DOP), durch Veresterung von Phthalsäureanhydrid mit Alkohol. Besonderheit dieses Verfahrens ist, dass es autokatalytisch, d. h. ohne Zusatz von Säuren, verläuft. Der an Alkohol abgereicherte, neutralisierte Rohester wird in einer als Ausdämpfkolonne bezeichneten Vorrichtung einer Behandlung mit Dampf unter vermindertem Druck zur Verringerung der flüchtigen Anteile unterzogen. Die leichtsiedenden Komponenten Wasserdampf und Alkohol werden am Kopf der Kolonne abgezogen, während das gestrippte Esterprodukt als Sumpfaustrag der Kolonne kontinuierlich entnommen wird. Nach der Kondensation des Brüden wird in einem Phasenseparator die wässrige Phase vom Alkohol abgetrennt und der Alkohol zurück in die Synthese gefahren. Die Abtrennung von weiteren bei der Veresterung gebildeten Nebenkomponenten, wie Alkenen und Ethern, ist nicht beschrieben.

In der GB-A 1,302,146 ist ein Verfahren zur Reinigung hochsiedender Ester mittels Dampfdestillation beschrieben. Die thermische Aufreinigung der Ester erfolgt durch zwei in Reihe geschaltete Destillationskolonnen.

Wie eingangs erwähnt, ist ebenfalls bekannt, Benzolpolycarbonsäureester einer Hydrierung durch Umsetzung mit Wasserstoff in Gegenwart eines Hydrierkatalysators zu unterziehen, um die entsprechenden Cyclohexanpolycarbonsäureester zu erhalten. So beschreibt beispielsweise die EP 0005737 A1 (DE-A 28 23 165) die Hydrierung von aromatischen Carbonsäureestern an geträgerten Ni-, Ru-, Rh- oder Pd-Katalysatoren zu den entsprechenden cycloaliphatischen Carbonsäureestern bei Temperaturen von 70 bis 250 °C und Drücken von 30 bis 200 bar.

In der WO 99/32427 ist ein Verfahren zur Hydrierung von Benzolpolycarbonsäuren oder Derivaten davon, wie z. B. den Estern, unter Verwendung eines Makroporen aufweisenden Katalysators beschrieben. Das Verfahren zeichnet sich durch eine hohe Raum-Zeit-Ausbeute und eine hohe Selektivität aus. In diesem Dokument ist ebenfalls die Verwendung der so erhaltenen Cyclohexanpolycarbonsäurederivate als Weichmacher in Kunststoffen beschrieben. Konkret wird z. B. der Cyclohexan-1,2-dicarbonsäurediisononylester erwähnt.

Es gab bereits Bestrebungen, Cyclohexanpolycarbonsäureester mit einem verringerten Gehalt an bestimmten Nebenprodukten bereit zu stellen.

Die WO 2014/053618 beschreibt ein Verfahren zur Herstellung von Cyclohexanpolycarbonsäuren oder Derivaten davon mit einem geringen Anteil an Nebenkomponenten, insbesondere an Hexahydrophthalid und Isononylalkohol. Gelöst wird diese Aufgabe durch Hydrierung der entsprechenden Benzolpolycarbonsäuren oder deren Derivaten in Gegenwart eines speziellen Schalenkatalysators und bei einer Leerrohrgeschwindigkeit von höchstens 50 m/h.

Die EP 3214067 A1 betrifft ein Verfahren zur Aufreinigung von Cyclohexanpolycarbonsäureester-Rohprodukten, wie sie bei der Hydrierung der entsprechenden Benzolpolycarbonsäureester-Vorläufer erhalten werden, bei dem die auf die Benzolpolycarbonsäureester einwirkende thermische Belastung als auch die Menge der bei dem Verfahren entstehenden Abgase und/oder Abwässer (Kondensate), die der Aufbereitung bzw. der Abfallbeseitigung zugeführt werden müssen, möglichst gering gehalten werden. Diese Aufgabe wird gelöst durch ein Verfahren, bei dem man
i) ein Cyclohexanpolycarbonsäureester-haltiges Rohprodukt bereitstellt,
ii) das in Schritt i) bereitgestellte Rohprodukt einer thermischen Aufreinigung in wenigstens einem Stoffaustauschapparat durch Einleiten eines Gasstroms G1 im Sumpf des wenigstens einen Stoffaustauschapparats unterzieht, unter Erhalt eines an dem Cyclohexanpolycarbonsäureester angereicherten Sumpfprodukts SP und eines an wenigstens einer Verunreinigung angereicherten Gasstroms AG1,
wobei der Gasstrom G1 ein unter den Verfahrensbedingungen nicht kondensierbares Gas oder Gasgemisch sowie maximal 30 Gew.-% Wasserdampf enthält, und wobei man den Gasstrom AG1 einer zumindest teilweisen Kondensation der darin enthaltenen Verunreinigungen unterzieht, unter Erhalt eines an Verunreinigungen abgereicherten Gasstroms AG2, den man, gegebenenfalls zusammen mit dem restlichen Teil des Gasstroms AG1, wieder in den Sumpf des Stoffaustauschapparats einleitet.

Die aus dem Stand der Technik bekannten Verfahren zur Herstellung von Cyclohexanpolycarbonsäureestern und die so erhaltenen Ester sind noch verbesserungsbedürftig. So weisen die mit Hilfe dieser Verfahren hergestellten Cyclohexanpolycarbonsäureester immer noch einen unerwünscht hohen Anteil an Nebenprodukten auf. Es wurde gefunden, dass speziell die bei der Veresterung der Benzolpolycarbonsäureester gebildeten Mittelsieder mit einem Siedepunkt oberhalb des Siedepunkts von Wasser und dem zur Veresterung eingesetzten Alkohol und unterhalb des Siedepunkts der Benzolpolycarbonsäureester und der Cyclohexanpolycarbonsäureester bisher nur unzureichend entfernt werden und als Nebenkomponenten im Produkt verbleiben. Dies gilt speziell für die aus den zur Veresterung eingesetzten Alkoholen durch nucleophile Substitution als Nebenreaktion gebildeten Ether, wie z. B. Diisononylether. Die Bildung von Ethern als Nebenprodukt bei der Veresterung kann nur begrenzt limitiert werden. Je nach Qualität der verwendeten Rohstoffe und der Prozessbedingungen kann der Gehalt an Ethern im fertigen Produkt bei bis zu mehreren Tausend Gew.-ppm liegen. Zudem können Ether auch bei der Hydrierung der Benzolpolycarbonsäureester zu den Cyclohexanpolycarbonsäureestern gebildet werden. Ein hoher Anteil an Nebenprodukten kann dazu führen, dass die durch Verfahren gemäß dem Stand der Technik hergestellten Cyclohexanpolycarbonsäureester nachteilige anwendungstechnische Eigenschaften bei der Verwendung als Weichmacher aufweisen, wie z. B. eine hohe Flüchtigkeit und/oder eine schlechte Verträglichkeit mit Kunststoffen, beispielweise PVC. Dadurch sind die aus dem Stand der Technik bekannten Cyclohexanpolycarbonsäureester weniger gut für sensible Anwendungen geeignet, bei denen die aus den weichgemachten Kunststoffen hergestellten Produkte in Kontakt mit Menschen kommen, z. B. bei Kinderspielzeug, Lebensmittelverpackungen oder medizinischen Artikeln.

So besteht weiterer Bedarf an einem Verfahren, das die Herstellung von Cyclohexanpolycarbonsäureestern mit einem geringen Anteil an Nebenprodukten, speziell an Mittelsiedern, und insbesondere an Ethern, ermöglicht. Insbesondere liegt der vorliegenden Erfindung die Aufgabe zugrunde, ein Verfahren zur Herstellung von Cyclohexan-1 ,2-dicarbonsäurediisononylester mit einem geringen Gehalt an Diisononylether bereit zu stellen. Das Verfahren soll wirtschaftlich und effizient sein und besonders aufwändige Aufreinigungsschritte und/oder den Verlust an Ausgangsalkohol vermeiden.

Überraschenderweise wurde gefunden, dass es möglich ist, bei der Aufarbeitung der Rohester aus der Veresterung einer Benzolpolycarbonsäure mit wenigstens einem Alkohol, durch Strippen des Rohesters mit einem wasserdampfhaltigen Gasstrom, die als Nebenprodukt gebildeten Ether mit dem Brüden zumindest teilweise aus dem Esterprodukt zu entfernen. Des Weiteren wurde gefunden, dass es möglich ist, durch die Wahl geeigneter Katalysatoren die Bildung von Ethern bei der Hydrierung der Benzolpolycarbonsäureester zu den Cyclohexanpolycarbonsäureestern zu vermindern oder zu vermeiden.

### ZUSAMMENFASSUNG DER ERFINDUNG

Die Erfindung ist im beigefügten Anspruchssatz beschrieben. Ein erster Gegenstand der Erfindung ist ein Verfahren zur Aufarbeitung eines Rohesters aus der Veresterung einer Benzolpolycarbonsäure mit wenigstens einem C₄-C₁₂-Monoalkanol, wobei der Rohester zusätzlich
- wenigstens einen Di-(C₄-C₁₂-alkyl)ether aus der Veretherung des wenigstens einen C₄-C₁₂-Monoalkanols,
- gegebenenfalls das wenigstens eine C₄-C₁₂-Monoalkanol, und
- gegebenenfalls Wasser

enthält, bei dem man den Rohester einer thermischen Aufreinigung in wenigstens einem Stoffaustauschapparat durch Einleiten eines wasserdampfhaltigen Gasstroms im Bereich des Sumpfs des Stoffaustauschapparats unterzieht, unter Erhalt eines an dem wenigstens einen Benzolpolycarbonsäureester angereicherten und dem wenigstens einen Di-(C₄-C₁₂-alkyl)ether abgereicherten Sumpfprodukts und eines an dem wenigstens einen Di-(C₄-C₁₂-alkyl)ether angereicherten Brüdens,
wobei man den Brüden zumindest teilweise kondensiert, das Kondensat trennt in eine wässrige Phase und eine organische Phase, enthaltend Di-(C₄-C₁₂-alkyl)ether und C₄-C₁₂-Monoalkanol, einen Teil der organischen Phase als Rücklauf in die thermische Aufreinigung des Rohesters zurückführt und einen anderen Teil der organischen Phase ausschleust.

Ein zweiter Gegenstand der Erfindung ist ein Verfahren zur Aufarbeitung eines Rohesters aus der Veresterung einer Benzolpolycarbonsäure mit wenigstens einem C₄-C₁₂-Monoalkanol, wobei der Rohester zusätzlich
- wenigstens einen Di-(C₄-C₁₂-alkyl)ether aus der Veretherung des wenigstens einen C₄-C₁₂-Monoalkanols,
- gegebenenfalls das wenigstens eine C₄-C₁₂-Monoalkanol, und
- gegebenenfalls Wasser

enthält, bei dem man den Rohester einer thermischen Aufreinigung in wenigstens einem Stoffaustauschapparat durch Einleiten eines wasserdampfhaltigen Gasstroms im Bereich des Sumpfs des Stoffaustauschapparats unterzieht, unter Erhalt eines an dem wenigstens einen Benzolpolycarbonsäureester angereicherten und dem wenigstens einen Di-(C₄-C₁₂-alkyl)ether abgereicherten Sumpfprodukts und eines an dem wenigstens einen Di-(C₄-C₁₂-alkyl)ether angereicherten Brüdens,
wobei man den Brüden zumindest teilweise kondensiert, das Kondensat trennt in eine wässrige Phase und eine organische Phase, enthaltend Di-(C₄-C₁₂-alkyl)ether und C₄-C₁₂-Monoalkanol, zumindest einen Teil der organischen Phase einer Auftrennung unterzieht in eine an Di-(C₄-C₁₂-alkyl)ether angereicherte Fraktion und eine an C₄-C₁₂-Monoalkanol angereicherte Fraktion und die an Di-(C₄-C₁₂-alkyl)ether angereicherte Fraktion teilweise oder vollständig ausschleust.

Die Erfindung erlaubt die Bereitstellung einer Zusammensetzung, enthaltend wenigstens einen Benzolpolycarbonsäureester der allgemeinen Formel (II) 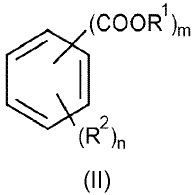 worin
- m: für 2, 3 oder 4 steht,
- n: für 0, 1, 2 oder 3 steht,
- R¹: unabhängig voneinander für geradkettiges oder verzweigtes C₄-C₁₂-Alkyl steht, und
- R²: unabhängig voneinander für geradkettiges oder verzweigtes C₁-C₄-Alkyl steht,
und 1 bis 500 Gew.-ppm, bezogen auf das Gesamtgewicht der Zusammensetzung, wenigstens eines Ethers der allgemeinen Formel R¹-O-R¹.

Die Erfindung erlaubt die Bereitstellung einer Zusammensetzung, enthaltend Diisononylphthalat und 10 bis 500 Gew.-ppm, bezogen auf das Gesamtgewicht der Zusammensetzung, Diisononylether.

Die Erfindung erlaubt die Bereitstellung einer Zusammensetzung, enthaltend wenigstens einen Cyclohexanpolycarbonsäureester der allgemeinen Formel (I) 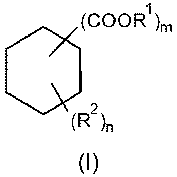 worin
- m: für 2, 3 oder 4 steht,
- n: für 0, 1, 2 oder 3 steht,
- R¹: unabhängig voneinander für geradkettiges oder verzweigtes C₄-C₁₂-Alkyl steht, und
- R²: unabhängig voneinander für geradkettiges oder verzweigtes C₁-C₄-Alkyl steht,
und 1 bis 1000 Gew.-ppm, bezogen auf das Gesamtgewicht der Zusammensetzung, wenigstens eines Ethers der allgemeinen Formel R¹-O-R¹.

Die Erfindung erlaubt die Bereitstellung einer Zusammensetzung, enthaltend Cyclohexan-1,2-dicarbonsäurediisononylester und 10 bis 1000 Gew.-ppm, bezogen auf das Gesamtgewicht der Zusammensetzung, Diisononylether.

Ferner beschrieben ist die Verwendung einer Zusammensetzung, wie zuvor und im Folgenden definiert, als Weichmacher, bevorzugt als Weichmacher für Polyvinylchlorid.

Ferner beschrieben ist die Verwendung einer Zusammensetzung, wie zuvor und im Folgenden definiert, in Produkten, mit denen Menschen in Kontakt kommen, bevorzugt in Kinderspielzeug, Lebensmittelverpackungen oder in medizinischen Artikeln.

### BESCHREIBUNG DER ERFINDUNG

Der Ester der Benzolpolycarbonsäure mit wenigstens einem C₄-C₁₂-Monoalkanol wird im Folgenden auch kurz als "Benzolpolycarbonsäureester" bezeichnet. Entsprechend wird der Ester der Cyclohexanpolycarbonsäure mit wenigstens einem C₄-C₁₂-Monoalkanol auch kurz als Cyclohexanpolycarbonsäureester bezeichnet.

Der in dem erfindungsgemäßen Verfahren zur Aufarbeitung eingesetzte Rohester ist eine Zusammensetzung, welche Komponenten mit unterschiedlichen Siedepunkten enthält. Dabei enthält der Rohester als Hochsieder wenigstens einen Benzolpolycarbonsäureester mit wenigstens einem C₄-C₁₂-Monoalkanol. Niedrigsiedende Komponenten sind die C₄-C₁₂-Monoalkanole, Wasser und C₄-C₁₂-Alkene. Mittelsiedende Komponenten mit einem Siedepunkt zwischen den Hochsiedern und den Niedrigsiedern sind die Di-(C₄-C₁₂-alkyl)ether.

Bevorzugt enthält der zur Aufarbeitung eingesetzte Rohester
- 91 bis 99,8 Gew.-% wenigstens eines Esters einer Benzolpolycarbonsäure mit wenigstens einem C₄-C₁₂-Monoalkanol,
- 0,05 bis 1 Gew.-% wenigstens eines Di-(C₄-C₁₂-alkyl)ethers,
- 0,1 bis 5 Gew.-% wenigstens eines C₄-C₁₂-Monoalkanols, und
- 0,05 bis 3 Gew.-% Wasser.

Für den Einsatz in dem erfindungsgemäßen Verfahren zur Aufarbeitung eines Rohesters geeignete und bevorzugte Benzolpolycarbonsäureester sind die zuvor und im Folgenden näher definierten Verbindungen der allgemeinen Formel (II), auf die in vollem Umfang Bezug genommen wird.

### Aufarbeitung eines Rohesters der Benzolpolycarbonsäure

In dem erfindungsgemäßen Verfahren wird ein Benzolpolycarbonsäureester-haltiges Rohprodukt einer thermischen Aufreinigung in wenigstens einem Stoffaustauschapparat durch Einleiten eines wasserdampfhaltigen Gasstroms im Bereich des Sumpfs des Stoffaustauschapparats unterzogen. Eine solche thermische Aufreinigung wird nachfolgend auch als "Strippung" bezeichnet. Allgemein wird unter dem Begriff "Strippung" ein dem Fachmann bekanntes Verfahren verstanden, bei dem ein Rohprodukt, das verdampfbare Verunreinigungen enthält, in einem geeigneten Stoffaustauschapparat mit einem die Verunreinigung zumindest teilweise aufnehmenden gasförmigen Strippmedium, auch "Strippgas" genannt, in Kontakt gebracht wird. Speziell erfolgt die thermische Aufreinigung in dem wenigstens einem Stoffaustauschapparat unter Vakuum.

Die Aufarbeitung eines Rohesters nach dem erfindungsgemäßen Verfahren erfolgt vorzugsweise kontinuierlich. Das heißt, dass in den Stoffaustauschapparat kontinuierlich Rohester eingespeist, ein wasserdampfhaltiger Gasstrom eingeleitet und ein Brüdenstrom ausgeschleust wird. Davon unabhängig kann die Entfernung wenigstens eines Teils des Di-(C₄-C₁₂-alkyl)ethers aus dem Brüdenstrom und/oder die Rückführung wenigstens eines Teils des kondensierten Brüdens in den Stoffaustausch diskontinuierlich oder kontinuierlich erfolgen. Letzteres wird im Folgenden eingehender beschrieben.

Erfindungsgemäß wird in den Stoffaustauschapparat als Strippgas ein wasserdampfhaltiger Gasstrom eingeleitet. Vorzugsweise enthält dieser Gasstrom wenigstens 50 Vol.-%, besonders bevorzugt wenigstens 70 Vol.-%, insbesondere wenigstens 90 Vol.-% Wasserdampf. Gewünschtenfalls kann der in den Stoffaustauschapparat eingeleitete Gasstrom zusätzlich wenigstens ein unter den Verfahrensbedingungen nicht kondensierbares Gas enthalten. Vorzugsweise ist das nicht kondensierbare Gas ausgewählt unter Stickstoff, Kohlendioxid, Sauerstoff, Wasserstoff, Helium, Neon, Argon und Mischungen davon. Insbesondere besteht der in den Stoffaustauschapparat eingeleitete Gasstrom nur aus Wasserdampf.

Im Rahmen der vorliegenden Erfindung wird unter dem Begriff "thermische Aufreinigung" ein dem Fachmann bekannter Prozess verstanden, bei dem ein Stoffgemisch durch Zuführung eines Gases und gegebenenfalls zusätzlich durch Zuführung von thermischer Energie aufgetrennt wird. Grundsätzlich bewirkt die Zuführung von thermischer Energie die Auftrennung eines Stoffgemisches aufgrund der unterschiedlichen Siedepunkte der im Stoffgemisch enthaltenen Verbindungen. Hierbei handelt es sich dann insbesondere um eine destillative Auftrennung. Durch die Zuführung eines Gases zum Stoffgemisch erfolgt die Auftrennung im Wesentlichen durch die unterschiedliche Flüchtigkeit der einzelnen in dem Stoffgemisch enthaltenen Verbindungen. Dabei werden leichtflüchtige Verbindungen, d. h. Verbindungen, die leichter in die Gasphase übertreten können, eher mitgeschleppt als schwerflüchtige Verbindungen, d. h. Verbindungen, die nur schwer in die Gasphase übertreten können. Bevorzugt wird bei der thermischen Aufreinigung des Rohesters nach dem erfindungsgemäßen Verfahren das aufzutrennende Gemisch sowohl durch das Einleiten eines Strippgases als auch durch Zuführung thermischer Energie aufgetrennt.

Die Wärmezufuhr erfolgt überwiegend über das eingespeiste Benzolpolycarbonsäureester-haltige Rohprodukt. Darüber hinaus kann zusätzlich eine Wärmezufuhr über den wasserdampfhaltigen Gasstrom (d. h. das Strippgas) und/oder Erhitzen des Kolonnensumpfs mittels Sumpfheizung erfolgen.

Im Rahmen der vorliegenden Erfindung wird unter dem Begriff "Stoffaustauschapparat" eine Vorrichtung verstanden, in der Gase und Flüssigkeiten miteinander in Kontakt gebracht werden und dabei ein Stofftransport zwischen dem Gas bzw. der Gasphase und der Flüssigkeit bzw. der Flüssigphase ermöglicht wird (Stofftransport aus dem Gas bzw. der Gasphase in die Flüssigkeit bzw. Flüssigphase und/oder aus der Flüssigkeit bzw. Flüssigphase in das Gas oder in die Gasphase).

Im dem vorliegenden Verfahren handelt es sich bei dem Stoffaustauschapparat in der Regel um eine Vorrichtung, welche den Übertritt von in der Flüssigkeit bzw. der Flüssigphase befindlichen leichtflüchtigen Bestandteilen in das Gas bzw. in die Gasphase ermöglicht.

Als Stoffaustauschapparat eignen sich in der Regel alle dem Fachmann geläufigen Vorrichtungen zur Auftrennung von Reaktionsgemischen, die flüssige Komponenten enthalten. Geeignete Vorrichtungen umfassen Kolonnen, die mit Einbauten und/oder mit Packungen ausgerüstet sein können, Blasensäulen, Drehbandkolonnen-Verdampfer, Dünnschichtverdampfer, Fallfilmverdampfer, Zwangsumlaufverdampfer, Sambay-Verdampfer, Sprühapparate, etc. und Kombinationen davon. In einer speziellen Ausgestaltung wird als Stoffaustauschapparat eine einzelne Kolonne eingesetzt.

Bevorzugt wird zur thermischen Aufreinigung des Rohesters wenigstens eine Kolonne verwendet, insbesondere eine Stripp-Destillationskolonne oder eine Anordnung von Kolonnen, die wenigstens eine Stripp-Destillationskolonne umfasst. In einer speziellen Ausgestaltung erfolgt die Aufreinigung in einer einzelnen Stripp-Destillationskolonne.

Besonders bevorzugt wird zur thermischen Aufreinigung des Rohesters wenigstens eine Kolonne verwendet, die mit Einbauten, wie beispielsweise mit Böden, wie Glockenböden oder Siebböden, oder mit Platten, wie Siebplatten, und/oder mit Packungen, wie beispielsweise mit Füllkörperpackungen (z. B. Pallringen, IMTP Füllkörper oder anderen Hochleistungsfüllkörpern) oder strukturierten Packungen (z. B. Mellapak Plus^{™}), ausgerüstet ist. Geeignete Ausgestaltungen von zur thermischen Aufreinigung geeigneten Destillationsvorrichtungen findet sich beispielsweise in Green, Don W.; Perry, Robert H. (2008). Perry's Chemical Engineer's Handbook (8th Edition) McGraw-Hill., Section 14.

Besonders bevorzugt wird zur thermischen Aufreinigung des Rohesters wenigstens eine gepackte Kolonne verwendet. Diese weist speziell druckverlustarme Einbauten und/oder Packungen, wie insbesondere Hochleistungsstrukturpackungen, auf.

In einer speziellen Ausgestaltung des erfindungsgemäßen Verfahrens wird zur thermischen Aufreinigung des Rohesters wenigstens eine Kolonne verwendet, wobei der Rohester im Seitenbereich der Kolonne eingespeist wird. Unter dem "Seitenbereich der Kolonne" wird im Rahmen der vorliegenden Erfindung ein Bereich unterhalb des Kopfbereichs und oberhalb des Sumpfs verstanden. In der Regel befinden sich unterhalb der Einspeisung des Rohesters trennungswirksame Einbauten. Bevorzugt befinden sich unterhalb und oberhalb der Einspeisung des Rohesters trennungswirksame Einbauten. Der Bereich oberhalb der Einspeisung wird auch als "Verstärkerteil" und der Bereich unterhalb der Einspeisung als "Abtriebsteil" bezeichnet. Im Rahmen der Erfindung kann an Stelle eines Abtriebsteils auch eine analoge Vorrichtung, z. B. ein außenliegender Zwangsumlaufverdampfer, vorgesehen werden. Unter dem "Kopfbereich der Kolonne" wird im Rahmen der vorliegenden Erfindung der obere Bereich der Kolonne verstanden, der sich oberhalb der Einspeisung des Rohesters und, soweit vorhanden, oberhalb der trennungswirksamen Kolonneneinbauten befindet. Entsprechend bezeichnet der "Sumpfbereich der Kolonne" den bodenseitigen Bereich der Kolonne, der sich unterhalb der trennungswirksamen Kolonneneinbauten befindet und der das flüssige Sumpfprodukt enthält.

In einer speziellen Ausführungsform wird zur thermischen Aufreinigung des Rohesters wenigstens eine Kolonne eingesetzt, die
- einen seitlichen Zulauf für den Rohester,
- einen oberhalb der Zulaufstelle für den Rohester gelegenen Verstärkungsteil,
- einen oberhalb des Verstärkungsteils gelegenen Rücklauf für wenigstens einen Teil des kondensierten Brüdens,
- eine Zuleitung für den wasserdampfhaltigen Gasstrom im Bereich des Sumpfs der Kolonne,
aufweist.

Vorzugsweise weist der oberhalb der Zulaufstelle für den Rohester gelegene Verstärkungsteil 0 bis 10 theoretische Trennstufen, besonders bevorzugt 0 bis 5 theoretische Trennstufen, insbesondere 0 bis 2 theoretische Trennstufen, auf.

Die zur thermischen Aufreinigung des Rohesters eingesetzte wenigstens eine Kolonne kann weitere Einbauten enthalten. Dazu zählen z. B. Flüssigkeitsverteiler. Diese befinden sich vorzugsweise unterhalb des Rücklaufs für wenigstens einen Teil des kondensierten Brüdens und/oder unterhalb der Zulaufstelle für den Rohester. Dazu zählen weiterhin Flüssigkeitssammler. Diese befinden sich vorzugsweise unterhalb oder innerhalb des Verstärkungsteils. Flüssigkeitssammler dienen der Neuverteilung und/oder zumindest teilweisen Ausschleusung der gesammelten Flüssigkeiten.

Der bei der erfindungsgemäßen Aufarbeitung des Rohesters erhaltene Brüden wird am Kopf des Stoffaustauschapparat abgezogen. Wird zur thermischen Aufreinigung des Rohesters eine Kolonne eingesetzt, so wird der Brüden am Kopf der Kolonne abgezogen.

Zur teilweisen oder vollständigen Kondensation des Brüdens können alle geeigneten Kondensatoren verwendet werden. Diese können mit beliebigen Kühlmedien gekühlt werden. Kondensatoren mit Luftkühlung und/oder Wasserkühlung sind bevorzugt, wobei die Luftkühlung besonders bevorzugt ist.

Der kondensierte Brüden wird einer Phasentrennung in eine wässrige Phase und eine organische Phase unterzogen. Üblicherweise wird das Kondensat hierzu in einen Phasenscheider (Dekanter) geleitet, wo es durch mechanisches Absetzen in zwei Phasen zerfällt, die getrennt abgezogen werden können. Die wässrige Phase wird abgetrennt und kann, gegebenenfalls nach Aufarbeitung, verworfen oder zur Herstellung von Dampf zur Strippung des Rohesters verwendet werden. Der kondensierte Brüden wird teilweise ausgeschleust oder teilweise oder vollständig einer weiteren Verwertung, wie im Folgenden beschrieben, unterzogen werden.

Sofern der Brüden leichter als das C₄-C₁₂-Monoalkanol siedende Komponenten enthält, speziell C₄-C₁₂-Alkene, die sich nicht mit vertretbarem Aufwand in einer Stufe mit C₄-C₁₂-Monoalkanol und Di-(C₄-C₁₂-alkyl)ether kondensieren lassen oder deren Anreicherung im kondensierten Brüden nicht gewünscht ist, können diese als Gasphase abgetrennt und nachkondensiert oder ausgeschleust werden.

Es wurde überraschenderweise gefunden, dass es mit dem erfindungsgemäßen Verfahren gelingt, den Rohester unter Erhalt eines an Di-(C₄-C₁₂-alkyl)ether abgereicherten und an Benzolpolycarbonsäureester angereicherten Sumpfprodukts und eines an Di-(C₄-C₁₂-alkyl)ether angereicherten Brüdens aufzuarbeiten. Der Gehalt des Brüdens an Di-(C₄-C₁₂-alkyl)ether lässt sich vorzugsweise über einen oder mehrere der folgenden Parameter steuern:
- Der Temperatur des Rohesters beim Eintritt in den Stoffaustauschapparat,
- dem Druck bei der thermischen Aufreinigung des Rohesters,
- der Temperatur des in den Stoffaustauschapparat eingeleiteten wasserdampfhaltigen Gasstroms, und
- der Menge des in den Stoffaustauschapparat eingeleiteten wasserdampfhaltigen Gasstroms.

Dabei gelingt es gleichzeitig zu vermeiden, dass das Wertprodukt, d. h. der Benzolpolycarbonsäureester, in signifikanter Menge in den Brüden gelangt.

Die Temperatur bei der thermischen Aufreinigung des Rohesters liegt in der Regel im Bereich von 20 bis 280 °C, bevorzugt im Bereich von 40 bis 250 °C, insbesondere im Bereich von 100 bis 220 °C. Diese Temperatur entspricht der Temperatur beim Eintritt des Rohesters in den Stoffaustauschapparat, speziell in die Kolonne. Bei Verwendung einer Kolonne mit seitlicher Zuführung des Rohesters entspricht die Temperatur der Temperatur beim Eintritt des Rohesters über die seitliche Zuführung.

Die thermische Aufreinigung des Rohesters kann bei Umgebungsdruck oder bei vermindertem Druck erfolgen. Bevorzugt erfolgt die thermische Aufreinigung bei vermindertem Druck.

Besonders bevorzugt erfolgt die thermische Aufreinigung des Rohesters bei einem Druck im Bereich von 1 bis 500 mbar (absolut), insbesondere im Bereich von 10 bis 300 mbar (absolut).

Bevorzugt liegt die Temperatur des in den Stoffaustauschapparat eingeleiteten wasserdampfhaltigen Gasstroms in einem Bereich von 100 bis 200 °C, besonders bevorzugt 110 bis 170 °C.

Der Druck des wasserdampfhaltigen Gasstroms beträgt vor Eintritt in den Stoffaustauschapparat maximal 10 bar, bevorzugt maximal 6 bar, insbesondere maximal 4 bar. Unmittelbar vor oder mit Eintritt in den Stoffaustauschapparat wird der wasserdampfhaltige Gasstrom auf den Druck des Stoffaustauschapparats entspannt. Dabei kommt es zu einer Abkühlung des wasserdampfhaltigen Gasstroms.

Bevorzugt beträgt die Menge des in den Stoffaustauschapparat eingeleiteten wasserdampfhaltigen Gasstroms 12 bis 62 NL Gas pro kg Rohester (entspricht ca. 1 bis 5 Gew.-%), besonders bevorzugt 25 bis 50 NL Gas pro kg Rohester (entspricht ca. 2 bis 4 Gew.-%). (NL = Normliter).

Der im Brüden enthaltene Di-(C₄-C₁₂-alkyl)ether wird zumindest teilweise ausgeschleust. Das heißt, zumindest ein Teil des im Brüden enthaltenen Di-(C4-C12-alkyl)ethers wird weder in die Veresterung der Benzolpolycarbonsäure mit wenigstens einem C₄-C₁₂-Monoalkanol noch in einen der Aufarbeitungsschritte zurückgeführt. Somit gelingt es, auch bei kontinuierlicher Aufarbeitung des Rohesters, einen Benzolpolycarbonsäureester mit gegenüber bekannten Verfahren deutlich verringertem Gehalt an Di-(C₄-C₁₂-alkyl)ether bereitzustellen.

Die Abtrennung zumindest eines Teils des im Brüden enthaltenen Di-(C₄-C₁₂-alkyl)-ethers erfolgt bevorzugt nach einer der im Folgenden beschriebenen Varianten 1 oder 2.

### Variante 1:

In einer Ausführungsform des erfindungsgemäßen Verfahrens wird der aus dem Stoffaustauschapparat ausgetragene Brüden zumindest teilweise kondensiert, das Kondensat in eine wässrige Phase und eine organische Phase getrennt, wobei die organische Phase Di-(C₄-C₁₂-alkyl)ether und C₄-C₁₂-Monoalkanol enthält, ein Teil der organischen Phase als Rücklauf in die thermische Aufreinigung des Rohesters zurückführt und ein anderer Teil der organischen Phase ausschleust.

Die Zurückführung eines Teils der organischen Phase als Rücklauf in die thermische Aufreinigung des Rohesters kann kontinuierlich oder diskontinuierlich erfolgen. Bevorzugt wird ein Teil der organischen Phase kontinuierlich als Rücklauf in die thermische Aufreinigung des Rohesters zurückgeführt.

Der Teil der organischen Phase, der als Rücklauf in die thermische Aufreinigung des Rohesters zurückgeführt wird, wird im Kopfbereich des Stoffaustauschapparats oberhalb der Zulaufstelle für den Rohester und, falls vorhanden, des Verstärkungsteils zurückgeführt.

Bevorzugt wird für die Variante 1 als Stoffaustauschapparat eine Kolonne eingesetzt, die einen seitlichen Zulauf für den Rohester, einen oberhalb der Zulaufstelle für den Rohester gelegenen Verstärkungsteil und einen oberhalb des Verstärkungsteils gelegenen Rücklauf aufweist, wobei über den Rücklauf ein Teil der aus dem kondensierten Brüden abgetrennten organischen Phase zurückgeführt wird.

Es wurde überraschenderweise gefunden, dass durch die teilweise Rückführung der organischen Phase im Kopfbereich der Kolonne die Trennwirkung der Kolonne so verbessert wird, dass der Gehalt an Benzolpolycarbonsäureester im Brüden deutlich verringert werden kann. Gleichzeitig wird der Gehalt an Di-(C₄-C₁₂-alkyl)ether im Brüden nicht oder nicht wesentlich verringert. Somit gelingt es bei dieser Variante, ein an Di-(C₄-C₁₂-alkyl)ether abgereichertes Sumpfprodukt bei deutlich geringerem Verlust an Benzolpolycarbonsäureester herzustellen.

Bevorzugt beträgt die Menge an Rücklauf in die thermische Aufreinigung 0,5 bis 50 kg/h/1000 kg Rohester, besonders bevorzugt 2,5 bis 25 kg/h/1000 kg Rohester, insbesondere 5 bis 15 kg/h/1000 kg Rohester.

Grundsätzlich ist es möglich, dass als Rücklauf die organische Phase teilweise oder vollständig durch andere flüssige Komponenten ersetzt wird. Dazu können z. B. Wasser, Rohester, aufgereinigter Benzolpolycarbonsäureester und Mischungen davon verwendet werden.

In einer geeigneten Ausführung erfolgt die Analytik der Inhaltsstoffe des Rohesters, des Brüden, der organischen Phase, etc. über Gaschromatographie (GC). Dann ermittelt man die Anteile der in einer Probe enthaltenen Verbindungen über deren Flächenintegrale. D.h. die Werte über den Gehalt der einzelnen Verbindungen in einer Probe werden originär als GC-Flächen-% erhalten. Es ist möglich, über Normierungsfaktoren die Mengenangaben von GC-Flächenprozent in Gew.-% umzurechnen. Eine Messreihe mit Reinverbindungen und Gemischen der Reinverbindungen in unterschiedlichen Mengenverhältnissen hat z. B. ergeben, dass die Normierungsfaktoren für C₄-C₁₂-Monoalkanol und Benzolpolycarbonsäure bei 1 liegen.

Der kondensierte Brüden wird, wie zuvor beschrieben, einer Phasentrennung in eine wässrige Phase und eine organische Phase unterzogen. Die bei der Trennung des Kondensats erhaltene wässrige Phase kann abgetrennt und verworfen oder zur Herstellung von Dampf zur Strippung des Rohesters verwendet werden.

Bevorzugt weist die aus dem kondensierten Brüden abgetrennte organische Phase 80 bis 99 Gew.-%, bevorzugt 82 bis 98,5 Gew.-%, wenigstens eines C₄-C₁₂-Monoalkanols, bezogen auf das Gesamtgewicht der organischen Phase auf.

Bevorzugt weist die aus dem kondensierten Brüden abgetrennte organische Phase höchstens 9 Gew.-%, besonders bevorzugt höchstens 8 Gew.-%, insbesondere höchstens 6 Gew.-%, Benzolpolycarbonsäureester, bezogen auf das Gesamtgewicht der organischen Phase auf.

Bevorzugt weist die aus dem kondensierten Brüden abgetrennte organische Phase wenigstens 2 Gew.-%, bevorzugt wenigstens 3 Gew.-%, Di-(C₄-C₁₂-alkyl)ether, bezogen auf das Gesamtgewicht der organischen Phase auf.

Bevorzugt weist die aus dem kondensierten Brüden abgetrennte organische Phase höchstens 8 Gew.-%, bevorzugt höchstens 6 Gew.-%, Wasser, bezogen auf das Gesamtgewicht der organischen Phase auf.

Die aus dem kondensierten Brüden abgetrennte organische Phase kann weitere Komponenten enthalten. Bevorzugt ist jede dieser Komponenten nur in einer untergeordneten Menge, bevorzugt in einer Menge von maximal 2 Gew.-% je Komponente, besonders bevorzugt in einer Menge von maximal 1 Gew.-% je Komponente, insbesondere in einer Menge von maximal 0,5 Gew.-% je Komponente, bezogen auf das Gesamtgewicht der organischen Phase, enthalten. Speziell kann die organische Phase wenigstens eine weitere leichtsiedende Komponente enthalten. Dazu zählen z. B. C₄-C₁₂-Alkene. So können bei der Veresterung der Benzolpolycarbonsäure mit einem C₉-Monoalkanol-Isomerengemisch die isomeren Nonene enthalten sein. Weitere Leichtsieder sind die zur Veresterung eingesetzte Benzolpolycarbonsäure oder deren Anhydride oder Nebenprodukte daraus. Dazu zählen z. B. Phthalsäureanhydrid, Phthalid, Benzoesäure oder Benzoate. Weiterhin kann die organische Phase wenigstens eine weitere mittelsiedende Komponente und/oder wenigstens eine weitere hochsiedende Komponente enthalten.

Besonders bevorzugt enthält die aus dem kondensierten Brüden abgetrennte organische Phase:
- 0 bis 9 Gew.-%, bevorzugt 0 bis 6 Gew.-%, Benzolpolycarbonsäureester,
- 2 bis 8 Gew.-% bevorzugt 3 bis 6 Gew.-%, wenigstens eines Di-(C₄-C₁₂alkyl)ethers,
- 83 bis 98 Gew.-%, bevorzugt 85 bis 95 Gew.-%, wenigstens eines C₄-C₁₂-Monoalkanols,
- 0 bis 8 Gew.-%, bevorzugt 0,1 bis 6 Gew.-%, Wasser,
bezogen auf das Gesamtgewicht der organischen Phase. Die aus dem kondensierten Brüden abgetrennte organische Phase kann wenigstens eine weitere der zuvor genannten leicht-, mittel- oder hochsiedenden Komponenten enthalten, wobei deren Gesamtmenge bevorzugt 0 bis 5 Gew.-%, besonders bevorzugt 0 bis 1 Gew.-%, bezogen auf das Gesamtgewicht der organischen Phase, beträgt.

Gemäß der Variante 1 des erfindungsgemäßen Verfahrens wird ein Teil der organischen Phase ausgeschleust und ein Teil als Rücklauf in die thermische Aufreinigung des Rohesters zurückgeführt. In einer bevorzugten Ausführungsform wird ein weiterer Teil der organischen Phase in die Veresterung der Benzolpolycarbonsäure mit dem wenigstens einen C₄-C₁₂-Monoalkanol zurückgeführt. So kann der Verlust an wertvollem Eduktalkohol minimiert und dennoch gleichzeitig der Ethergehalt des Benzolpolycarbonsäureesters gering gehalten werden.

Die Ausschleusung eines Teils der organischen Phase kann diskontinuierlich oder kontinuierlich erfolgt. Bevorzugt wird ein Teil der organischen Phase diskontinuierlich ausgeschleust.

Definitionsgemäß bezeichnet eine "Regelung" einen Vorgang, bei dem fortlaufend eine Größe, die Regelgröße (Istwert), erfasst, mit einer anderen Größe, der Führungsgröße (Sollwert), verglichen und im Sinne einer Angleichung an die Führungsgröße beeinflusst wird. Die Regelabweichung als Differenz zwischen Istwert und Sollwert wird dem Regler zugeführt, der daraus eine Stellgröße bildet. Die Stellgröße ist die Ausgangsgröße (die Stellung) des verwendeten Stellglieds, mit dessen Hilfe ein gezielter Eingriff in die Steuerstrecke erfolgt. Das Stellglied kann Bestandteil des Reglers sein, in vielen Fällen stellt es jedoch eine separate Vorrichtung dar. Durch das Stellen oder Verstellen des Stellglieds wird der Prozess gesteuert, z. B. durch Änderung eines Masse- oder Energieflusses. Beispiele für Stellglieder sind Ventile, Schalter, etc.

Regelgröße in dem erfindungsgemäßen Verfahren ist der Gehalt des Sumpfprodukts an Di-(C₄-C₁₂-alkyl)ether. Die Messung kann mittels GC erfolgen. In einer geeigneten Vorgehensweise werden regelmäßig Proben entnommen und analysiert. Eine Online Probenentnahme und Messung ist ebenfalls denkbar.

In einer bevorzugten Ausführungsform der 1. Variante des erfindungsgemäßen Verfahrens regelt man den Gehalt des Sumpfprodukts an Di-(C₄-C₁₂-alkyl)ether, indem man Stelleingriffe an wenigstens einer der folgenden Stellgrößen vornimmt:
- Dem Massenstrom des Rücklaufs der organischen Phase,
- dem Massenstrom der in die Veresterung zurückgeführten organischen Phase,
- dem Massenstrom der ausgeschleusten organischen Phase.

Vorzugsweise geht man dabei so vor, dass man
- einen Zielwert für den Gehalt des Sumpfprodukts an Di-(C₄-C₁₂-alkyl)ether und einen oberen und unteren Grenzwerts für die Abweichung des Istwerts vom Zielwert festlegt,
- den Istwert des Gehalts des Sumpfprodukts an Di-(C₄-C₁₂-alkyl)ether bestimmt,
- nach Erreichen des oberen Grenzwerts für die Abweichung des Istwerts vom Zielwert Stelleingriffe vornimmt, bis der Gehalt des Sumpfprodukts an Di-(C₄-C₁₂alkyl)ether auf den unteren Grenzwert für die Abweichung des Istwerts vom Zielwert abgesunken ist.

Der Zielwert für den Gehalt des Sumpfprodukts an Di-(C₄-C₁₂-alkyl)ether liegt vorzugsweise bei höchstens 1000 Gew.-ppm, besonders bevorzugt höchstens 800 Gew.-ppm, insbesondere höchstens 600 Gew.-ppm, speziell höchstens 500 Gew.-ppm.

Es wurde speziell gefunden, dass sich der Gehalt des Sumpfprodukts an Di-(C₄-C₁₂alkyl)ether vorteilhaft regeln lässt, indem man die Ausschleusung eines Teils der organischen Phase diskontinuierlich durchführt. Dazu kann man beispielsweise den Gehalt des Sumpfprodukts an Di-(C₄-C₁₂-alkyl)ether in regelmäßigen Abständen messen und bei Überschreitung eines festgelegten Maximalwerts solange einen Teil der organischen Phase ausschleusen, bis der Gehalt des Sumpfprodukts an Di-(C₄-C₁₂-alkyl)-ether wieder auf einen festgelegten Minimalwert gefallen ist. Die Rückführung eines weiteren Teils der organischen Phase in die Veresterung kann dann unabhängig oder abhängig von der diskontinuierlichen Ausschleusung eines Teils der organischen Phase erfolgen. So ist es z. B. möglich, wechselweise einen Teil der organischen Phase auszuschleusen und, wenn keine Ausschleusung erfolgt, einen Teil der organischen Phase in die Veresterung zurückzuführen.

In einer weiteren Variante des erfindungsgemäßen Verfahrens wird zumindest ein Teil des Di-(C₄-C₁₂-alkyl)ether über einen Seitenabzug der zur Aufarbeitung des Rohesters eingesetzten Kolonne ausgeschleust.

Auch nach dieser Variante wird als Stoffaustauschapparat eine Kolonne eingesetzt, die einen seitlichen Zulauf für den Rohester, einen oberhalb der Zulaufstelle für den Rohester gelegenen Verstärkungsteil und einen oberhalb des Verstärkungsteils gelegenen Rücklauf aufweist, wobei über den Rücklauf ein Teil der aus dem kondensierten Brüden abgetrennten organischen Phase zurückgeführt wird.

Zusätzlich weist die Kolonne innerhalb des Verstärkerteils oder unter dem Verstärkerteil aber oberhalb der Zulaufstelle für den Rohester einen Flüssigkeitssammler auf, der die vom Kopf der Kolonne herabströmende Flüssigkeit sammelt und eine teilweise oder vollständige Ausschleusung ermöglicht.

Alternativ oder zusätzlich zur Ausschleusung eines Di-(C₄-C₁₂-alkyl)ether-haltigen Stroms aus dem Flüssigkeitssammler kann, wie zuvor beschrieben, ein Teil der aus dem kondensierten Brüden abgetrennten organischen Phase ausgeschleust werden.

### Variante 2:

In einer weiteren bevorzugten Ausführungsform des erfindungsgemäßen Verfahrens wird der aus dem Stoffaustauschapparat ausgetragene Brüden zumindest teilweise kondensiert, das Kondensat in eine wässrige Phase und eine organische Phase getrennt, wobei die organische Phase Di-(C₄-C₁₂-alkyl)ether und C₄-C₁₂-Monoalkanol enthält, zumindest ein Teil der organischen Phase einer Auftrennung unterzogen in eine an Di-(C₄-C₁₂-alkyl)ether angereicherte Fraktion und eine an C₄-C₁₂-Monoalkanol angereicherte Fraktion und die an Di-(C₄-C₁₂-alkyl)ether angereicherte Fraktion teilweise oder vollständig ausschleust.

Durch die weitere Auftrennung zumindest eines Teils der organischen Phase aus der Trennung des Brüdenkondensats kann ein Verlust an Eduktalkohol aus der Veresterung vermindert und gleichzeitig der Gehalt an Ether im Sumpfprodukt weiter verringert werden. Es wurde gefunden, dass es möglich ist, aus der organischen Phase den C₄-C₁₂-Monoalkanol in hoher Reinheit abzutrennen. Bezüglich der Zusammensetzung der organischen Phase wird auf die zur Variante 1 gemachten Angaben in vollem Umfang Bezug genommen.

Die in der organischen Phase enthaltenen Schwersieder, speziell aus der Aufreinigung des Rohesters mit dem Brüden abgetrennte Anteile an Benzolpolycarbonsäureester, verbleiben dabei im Sumpfprodukt. Auch die in der organischen Phase enthaltenen Mittelsieder, speziell die bei der Aufreinigung des Rohesters mit dem Brüden abgetrennten Di-(C₄-C₁₂-alkyl)ether, verbleiben im Sumpfprodukt und können mit diesem ausgeschleust werden.

Die destillative Auftrennung der organischen Phase erfolgt nach üblichen, dem Fachmann bekannten Verfahren. Bevorzugt wird eine Destillationskolonne mit mehreren Trennstufen, einem Kopfkondensator und Rücklauf eingesetzt, der es ermöglicht, das Kopfprodukt zumindest teilweise im Bereich des Kolonnenkopfs zurückzuführen. Die Destillationskolonne kann die üblichen Einbauten, wie strukturierte Packungen, Schüttfüllkörper, Trennböden und Kolonneneinbauten (z. B. Verteiler und Sammler), aufweisen. Anstelle eines Abtriebsteils kann auch eine analoge Vorrichtung, z. B. ein außenliegender Zwangsumlaufverdampfer, vorgesehen werden.

In einer geeigneten Ausführungsform wird die organische Phase in den Kolonnensumpf oder am bodenseitigen Ende der Kolonne unterhalb der Einbauten zugeführt. Die Kolonne weist dann keinen Abtriebsteil auf. Sie verfügt vorzugsweise über mindestens eine theoretische Stufe, bevorzugt mindestens 4 theoretische Stufen, insbesondere mindestens 6 theoretische Stufen. Speziell weist die Kolonne 4 bis 25 theoretische Trennstufen, bevorzugt 6 bis 25 theoretische Trennstufen, insbesondere 8 bis 20 theoretische Trennstufen, auf.

In einer weiteren geeigneten Ausführungsform wird die organische Phase der Kolonne über einen Seiteneinlauf zugeführt. Die Destillationskolonne verfügt dann über einen Verstärkungsteil und einen Abtriebsteil. Vorzugsweise weist die Kolonne dann mindestens eine theoretische Stufe im Verstärkerteil, bevorzugt mindestens 4 theoretische Stufen im Verstärkerteil, insbesondere mindestens 6 theoretische Stufen im Verstärkerteil auf.

Die Destillation erfolgt bei Umgebungsdruck oder bevorzugt unter vermindertem Druck. Bevorzugt beträgt der Druck bei der Destillation 0,01 bar bis 1 bar, besonders bevorzugt 0,02 bar bis 0,5 bar, insbesondere 0,05 bar bis 0,2 bar.

Die Temperatur im Kolonnensumpf liegt vorzugsweise in einem Bereich von 50 bis 280 °C, besonders bevorzugt von 80 bis 200 °C.

Das Rücklaufverhältnis (Verhältnis der Rücklaufmenge zum Destillatabzug) liegt bevorzugt in einem Bereich von 0 bis 10, besonders bevorzugt von 0,05 bis 5, insbesondere von 0,1 bis 2.

Die destillative Auftrennung wenigstens eines Teils der organischen Phase kann kontinuierlich, semikontinuierlich oder diskontinuierlich erfolgen.

Bei einer geeigneten diskontinuierlichen Ausführungsform wird die organische Phase aus der Trennung des Brüdenkondensats in einem Behälter gesammelt und aus diesem einer destillativen Auftrennung in eine an Di-(C₄-C₁₂-alkyl)ether angereicherte Fraktion und eine an C₄-C₁₂-Monoalkanol angereicherte Fraktion zugeführt. Die destillative Auftrennung kann kontinuierlich oder semikontinuierlich erfolgen. In einer geeigneten Ausgestaltung für eine semikontinuierliche Auftrennung der organischen Phase wird die aus dem Phasentrennbehälter ausgeleitete organische Phase zunächst in einen Sammelbehälter geleitet und aus diesem in die destillative Auftrennung. Ist die Destillationskolonne befähigt, größere Mengen organische Phase pro Zeiteinheit aufzutrennen, als aus der Phasentrennung ausgeschleust wird, so kann man die Destillation für eine gewisse Zeit kontinuierlich durchführen, bis der Sammelbehälter leer ist. Dann wird die Destillation unterbrochen, bis wieder genug organische Phase für das nächste Destillationsintervall vorhanden ist.

Die an C₄-C₁₂-Monoalkanol angereicherte Fraktion kann, wie im Folgenden beschrieben, verwertet werden. Die an Di-(C₄-C₁₂-alkyl)ether angereicherte Fraktion wird ausschleust.

Bei einer bevorzugten kontinuierlichen Ausführungsform wird die organische Phase aus der Trennung des Brüdenkondensats direkt einer kontinuierlichen destillativen Auftrennung in eine an Di-(C₄-C₁₂-alkyl)ether angereicherte Fraktion und eine an C₄-C₁₂-Monoalkanol angereicherte Fraktion zugeführt. Die an C₄-C₁₂-Monoalkanol angereicherte Fraktion kann, wie im Folgenden beschrieben, verwertet werden. Die an Di-(C₄-C₁₂-alkyl)ether angereicherte Fraktion wird ausschleust.

Bevorzugt weist auch bei der Variante 2 des erfindungsgemäßen Verfahrens der zur Aufarbeitung des Rohesters eingesetzte Stoffaustauschapparat einen flüssigen Rücklauf im Kopfbereich auf. Speziell wird für die Variante 2 als Stoffaustauschapparat eine Kolonne eingesetzt, die einen seitlichen Zulauf für den Rohester, einen oberhalb der Zulaufstelle für den Rohester gelegenen Verstärkungsteil und einen oberhalb des Verstärkungsteils gelegenen Rücklauf aufweist, wobei über den Rücklauf ein Teil der aus dem kondensierten Brüden abgetrennten organischen Phase und/oder wenigstens ein Teil der bei der Auftrennung der organischen Phase erhaltenen an C₄-C₁₂-Monoalkanol angereicherten Fraktion zurückgeführt wird.

In einer bevorzugten Ausführungsform wird nur einen Teil der bei der Trennung des kondensierten Brüdens erhaltenen organischen Phase einer Auftrennung unterzogen in eine an Di-(C₄-C₁₂-alkyl)ether angereicherte Fraktion und eine an C₄-C₁₂-Monoalkanol angereicherte Fraktion, und ein anderer Teil der organischen Phase wird als Rücklauf in die thermische Aufreinigung des Rohesters zurückgeführt.

In einer bevorzugten Ausführungsform wird die an C₄-C₁₂-Monoalkanol angereicherte Fraktion teilweise oder vollständig als Rücklauf in die thermische Aufreinigung des Rohesters zurückführt. Somit kann ein Sumpfprodukt mit sehr geringem Gehalt an Di-(C₄-C₁₂-alkyl)ether erhalten werden.

In einer bevorzugten Ausführungsform wird die an C₄-C₁₂-Monoalkanol angereicherte Fraktion teilweise oder vollständig in die Veresterung der Benzolpolycarbonsäure mit dem wenigstens einen C₄-C₁₂-Monoalkanol zurückführt. Somit kann ein Verlust an wertvollem Eduktalkohol vermieden werden.

Die Zurückführung der aus dem kondensierten Brüden abgetrennten organischen Phase und/oder der bei der Auftrennung der organischen Phase erhaltenen an C₄-C₁₂-Monoalkanol angereicherten Fraktion als Rücklauf in die thermische Aufreinigung des Rohesters kann kontinuierlich oder diskontinuierlich, bevorzugt kontinuierlich, erfolgen.

### Benzolpolycarbonsäureester und Cyclohexanpolycarbonsäureester

Das zuvor beschriebene Verfahren ermöglicht die Herstellung von Benzolpolycarbonsäureestern mit einem geringen Anteil an Dialkylethern. Sie eignen sich zur Herstellung von Cyclohexandicarbonsäureestern, die ebenfalls einen geringen Anteil an Nebenprodukten und speziell Dialkylethern aufweisen.

Der in dem erfindungsgemäßen Verfahren eingesetzte Benzolpolycarbonsäureester ist vorzugsweise ausgewählt unter Verbindungen der allgemeinen Formel (II) 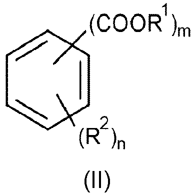 worin
- m: für 2, 3 oder 4 steht,
- n: für 0, 1, 2 oder 3 steht,
- R¹: unabhängig voneinander für geradkettiges oder verzweigtes C₄-C₁₂-Alkyl steht, und
- R²: unabhängig voneinander für geradkettiges oder verzweigtes C₁-C₄-Alkyl steht.

Die erfindungsgemäß hergestellten Cyclohexanpolycarbonsäureester sind vorzugsweise ausgewählt unter Verbindungen der allgemeinen Formel (I) 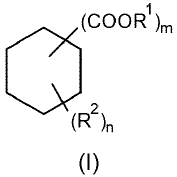 worin
- m: für 2, 3 oder 4 steht,
- n: für 0, 1, 2 oder 3 steht,
- R¹: unabhängig voneinander für geradkettiges oder verzweigtes C₄-C₁₂-Alkyl steht, und
- R²: unabhängig voneinander für geradkettiges oder verzweigtes C₁-C₄-Alkyl steht.

Die im Folgenden angegebenen geeigneten und bevorzugten Ausführungsformen für m, n, R¹ und R² gelten gleichermaßen für die Verbindungen der Formeln (I) und (II). Die Verbindungen (I) werden aus den Verbindungen (II) durch Hydrierung des Benzolrings erhalten, so dass in den Verbindungen (I) jedes Ringkohlenstoffatom ein Wasserstoffatom mehr trägt als das entsprechende Ringkohlenstoffatom in den Verbindungen (II).

Wenn m 2 oder 3 ist, können die Reste R¹ gleich oder verschieden sein. Die C₁-C₄-Alkylgruppen können geradkettig oder verzweigt sein. Wenn R¹ für eine Alkylgruppe steht, handelt es sich bevorzugt um Methyl-, Ethyl-, n-Propyl-, i-Propyl, n-Butyl, i-Butyl, sek.-Butyl oder tert.-Butyl. Bevorzugt ist m 0, d. h. es liegen keine C₁-C₄-Alkyl-Substituenten vor, sondern ausschließlich Wasserstoffatome, so dass ein aromatischer Benzolring (allgemeine Formel II) bzw. ein gesättigter Cyclohexylring (allgemeine Formel (I)) vorliegt.

Die n Reste R² können gleich oder verschieden sein. Die C₄-C₁₂-Alkylgruppen können geradkettig oder verzweigt sein. R² ist bevorzugt ein C₆-C₁₂-Alkyl, ganz besonders bevorzugt C₈-C₁₀-Alkyl. Beispiele für derartige Alkylgruppen sind n-Butyl, i-Butyl, sek.-Butyl oder tert.-Butyl, n-Pentyl, n-Hexyl, n-Octyl, 2-Ethyl-hexyl, n-Nonyl, iso-Nonyl, n-Decyl, iso-Decyl, n-Undecyl, iso-Undecyl, n-Dodecyl, iso-Dodecyl, n-Tridecyl, iso-Tridecyl, Stearyl, und n-Eicosyl.

Bei den Alkylgruppen kann es sich jeweils um einzelne Isomere der genannten Alkylgruppen oder um Gemische verschiedener Alkylgruppen handeln. Die verschiedenen Alkylgruppen können verschiedene Isomere mit derselben Zahl von Kohlenstoffatomen sein und/oder Alkylgruppen, die eine verschiedene Anzahl von Kohlenstoffatomen aufweisen.

Bei den erfindungsgemäß erhaltenen Cyclohexanpolycarbonsäureestern der allgemeinen Formel (I) handelt es sich insbesondere um Mono-, Di-, Tri- oder Tetraester der Cyclohexanpolycarbonsäuren. Bevorzugt sind alle Carbonsäuregruppen verestert. Die eingesetzten Ester sind Alkylester, wobei bevorzugte Alkylgruppen R² bereits vorstehend genannt sind.

Bevorzugt werden erfindungsgemäß Cyclohexanpolycarbonsäureester ausgewählt aus der Gruppe bestehend aus kernhydrierten Mono- und Dialkylestern der Phthalsäure, Isophthalsäure und Terephthalsäure, kernhydrierten Mono-, Di- und Trialkylestern der Trimellitsäure, der Trimesinsäure und der Hemimellitsäure oder Mono-, Di-, Tri- und Tetraalkylestern der Pyrromellitsäure erhalten, wobei die Alkylgruppen R¹ die oben genannten Bedeutungen haben.

Die erfindungsgemäß bevorzugt eingesetzten Benzolpolycarbonsäureester sind insbesondere ausgewählt aus der Gruppe bestehend aus Phthalsäure, Isophthalsäure, Terephthalsäure, Trimellitsäure, Trimesinsäure, Hemimellitsäure, Pyromeliltsäure und Mischungen davon. Ganz besonders bevorzugt wird Phthalsäure eingesetzt. Die vorstehend genannten Säuren sind kommerziell erhältlich.

Weiter bevorzugt werden erfindungsgemäß Benzolpolycarbonsäureester der allgemeinen Formel (II) eingesetzt. Diese werden beispielsweise erhalten, indem wenigstens eine Benzolpolycarbonsäure ausgewählt aus der Gruppe bestehend aus Phthalsäure, Isophthalsäure, Terephthalsäure, Trimellitsäure, Trimesinsäure, Hemimellitsäure, Pyromeliltsäure und Mischungen davon, mit entsprechenden Alkoholen R¹-OH umgesetzt werden.

Als Alkohole werden bevorzugt die den Resten R¹ der Cyclohexanpolycarbonsäureester der Formel I entsprechenden Alkohole eingesetzt.

Bevorzugt werden somit lineare oder verzweigte Alkohole mit C₄-C₁₂-Alkylresten eingesetzt. Bei den zur Veresterung mit den Benzolpolycarbonsäuren eingesetzten Alkoholen kann es sich jeweils um die den vorstehend genannten Resten R entsprechenden einzelnen Isomere der Alkohole oder um Gemische verschiedener Alkohole mit isomeren Alkylresten mit derselben Zahl von Kohlenstoffatomen handeln und/oder um Gemische verschiedener Alkohole mit unterschiedlicher Zahl der Kohlenstoffatome.

Die zur Umsetzung mit den Benzolpolycarbonsäuren geeigneten Alkohole oder Alkoholgemische können nach allen dem Fachmann bekannten Verfahren hergestellt werden. Geeignete Verfahren zur Herstellung von Alkoholen oder Verfahrensschritte, die bei der Herstellung von Alkoholen angewendet werden, sind zum Beispiel:
Hydroformylierung mit anschließender Hydrierung der gebildeten Aldehyde, zum Beispiel wie in WO 92/13818 offenbart;
Hydrierung von Aldolprodukten, zum Beispiel wie in DE-A 102 51 311 offenbart;
Hydratisierung von Alkenen, zum Beispiel wie in US 5,136,108 offenbart;
Hydrierung von Carbonsäuren und Carbonsäureestern, insbesondere Fettsäuren und Fettsäureestern, zum Beispiel wie in US 5,463,143 offenbart;
Hydrierung von ungesättigten Alkoholen oder von Carbonylverbindungen, zum Beispiel wie in EP-A 0 394 842 offenbart;
Hydrierung von Epoxiden, zum Beispiel wie in GB-A 879 803 offenbart;
Verfahren, umfassend einen Telomerisationsschritt, zum Beispiel wie in US 3,091,628 offenbart;
Verfahren, umfassend einen Isomerisierungsschritt, zum Beispiel wie in DE-A 42 28 887 offenbart;
Hydrolyse von Sulfaten, zum Beispiel wie in GB-A 1,165,309 offenbart;
Umsetzung von Dienen mit Aminen, zum Beispiel wie in DE-A 44 31 528 offenbart;
enzymatische Herstellung von Alkoholen, zum Beispiel wie in WO 93/24644 offenbart;
selektive Hydrierung von Dienen, zum Beispiel wie in US 3,203,998 offenbart;
Herstellung von Alkoholen aus Nitrilen, zum Beispiel wie in EP-A 0 271 092 offenbart;
Herstellung von Alkoholen durch Umsetzung von Alkinen, zum Beispiel wie in RU 205 9597-C1 offenbart; und
Hydrogenolyse von substituierten Tetrahydropyranen, zum Beispiel wie in GB 1,320,188 offenbart.

Dem Fachmann sind weitere Verfahren zur Herstellung von Alkoholen bekannt, die ebenfalls zur Veresterung von Benzolpolycarbonsäuren mit geeigneten Alkoholen oder Alkoholgemischen eingesetzt werden können.

Bevorzugt eingesetzte Alkohole sind - wie vorstehend erwähnt - Alkohole, die C₄-C₁₂-Alkylreste aufweisen. Insbesondere die längerkettigen C₅-C₁₂-Alkohole bzw. Alkoholgemische, die diese Alkohole enthalten, werden besonders bevorzugt durch katalytische Hydroformylierung (auch als Oxoreaktion bezeichnet) von Olefinen und anschließende Hydrierung der gebildeten Aldehyde hergestellt.

Geeignete Hydroformylierungsverfahren sind dem Fachmann bekannt und sind in den vorstehend genannten Dokumenten offenbart. Die in den genannten Dokumenten offenbarten Alkohole und Alkoholgemische können mit den vorstehend genannten Benzolpolycarbonsäuren zu den gewünschten Benzolpolycarbonsäurealkylestern bzw. -estergemischen umgesetzt werden.

C₅-Alkohole bzw. Gemische, die C₅-Alkohole, besonders bevorzugt n-Pentanol enthalten, können zum Beispiel durch Hydroformylierung von Butadien in Anwesenheit einer wässrigen Lösung einer Rhodiumverbindung und eines Phosphins als Katalysator hergestellt werden. Ein solches Verfahren ist zum Beispiel in EP-A 0 643 031 offenbart.

Geeignete C₇-Alkoholmischungen, die zur Veresterung mit den Benzolpolycarbonsäuren eingesetzt werden können, sind zum Beispiel in JP-A 2000/319 444 offenbart. Die Herstellung der C₇-Alkoholmischung erfolgt durch Hydroformylierung mit anschließender Hydrierung der gebildeten Aldehyde.

Mischungen enthaltend C₈-Alkohole und deren Herstellungsverfahren sind zum Beispiel in GB-A 721 540 offenbart, worin ein Verfahren zur Herstellung von Isooctylalkoholen ausgehend von Heptenen mittels Hydroformylierung und anschließender Hydrierung beschrieben wird. Ein beispielhaft genanntes, weiteres Dokument, das die Herstellung von C₇-Alkoholen bzw. diese Alkohole enthaltenden Mischungen offenbart, ist DE-A 195 30 414.

C₉-Alkohole bzw. Mischungen enthaltend C₉-Alkohole werden bevorzugt durch Dimerisierung von Butenen, Hydroformylierung der erhaltenen Octene und anschließende Hydrierung des erhaltenen C₉-Aldehyds hergestellt.

Geeignete Verfahren und C₉-Alkohle enthaltende Mischungen sind zum Beispiel in WO 92/13818 offenbart.

C₁₀-Alkohole und Mischungen enthaltend diese Alkohole sind zum Beispiel in WO 2003/66642 offenbart.

C₁₂-Alkohole bzw. Mischungen enthaltend C₁₂-Alkohole, insbesondere Trimethylnonanol, und ein Verfahren zu dessen Herstellung sind zum Beispiel in WO 98/03462 offenbart.

Besonders bevorzugt werden erfindungsgemäß Dialkylester der vorstehend genannten Cyclohexandicarbonsäuren, insbesondere 1,2-, 1,3- oder 1 ,4-Dialkylester und ganz besonders bevorzugt 1,2-Dialkylester erhalten. Dabei können Dialkylester erhalten werden bzw. die entsprechenden Benzoldicarbonsäuredialkylester eingesetzt werden, worin beide Estergruppen der Dialkylester dieselben Alkylreste tragen, sowie Estergruppen, worin die beiden Estergruppen der Dialkylester unterschiedliche Alkylgruppen tragen. Beispiele für gemischte und nicht gemischte Dialkylester sind bereits vorstehend genannt. Weiterhin ist es möglich, dass die Alkylgruppen zwar die gleiche Kohlenstoffatomanzahl aufweisen, jedoch geradkettig sind oder unterschiedliche Verzweigungen aufweisen und somit Isomerengemische bilden. Solche Isomerengemische können auch eingesetzt werden, wenn die Kohlenstoffanzahl der Alkylgruppen der Dialkylester unterschiedlich ist. Der Anteil der verschiedenen Isomeren der Alkylgruppen ergibt sich im Allgemeinen aus der Zusammensetzung der Alkohole, die zur Veresterung der Benzoldicarbonsäuren eingesetzt werden, die nach Veresterung erfindungsgemäß zu den Cyclohexandicarbonsäureestern hydriert werden. Geeignete Alkoholmischungen sind vorstehend bereits genannt. Im Sinne der vorliegenden Anmeldung sind somit unter geradkettigen oder verzweigten Alkylresten mit einer bestimmten Anzahl von Kohlenstoffatomen neben den jeweils einzelnen Isomeren Isomerengemische zu verstehen, deren Zusammensetzung sich - wie vorstehend erwähnt - aus der Zusammensetzung der zur Veresterung der Benzoldicarbonsäuren eingesetzten Alkohole ergibt.

Unter geradkettigen Alkylresten sind im Sinne der vorliegenden Anmeldung ausschließlich geradkettige Alkylreste zu verstehen, jedoch auch Mischungen von Alkylresten, die überwiegend geradkettig sind.

Handelt es sich bei den Alkylresten R¹ der Cyclohexanpolycarbonsäureester um C₄-Alkylreste, so werden diese durch Umsetzung der Benzolpolycarbonsäuren der Formel (II) mit R¹ gleich Wasserstoff mit n-Butanol, iso-Butanol, sek.-Butanol oder tert.-Butanol erhalten. Dabei können zur Herstellung von Benzolpolycarbonsäureestern, worin R¹ ein C₄ ist, jeweils Gemische der genannten Butanole eingesetzt werden oder einzelne Isomere. Bevorzugt werden einzelne Isomere des Butanols eingesetzt. Die Herstellung der vorstehend genannten C₄-Alkohole ist dem Fachmann bekannt.

Handelt es sich bei den Alkylresten R der Cyclohexanpolycarbonsäureester um C₅- bis C₁₂-Alkylreste, werden bevorzugt C₅- bis C₁₂-Alkohole eingesetzt, die Verzweigungsgrade (ISO-Index) von im Allgemeinen 0,10 bis 4, bevorzugt 0,5 bis 3, besonders bevorzugt 0,8 bis 2 und insbesondere bei 1 bis 1,5, aufweisen, d. h. im Allgemeinen handelt es sich bei den jeweiligen Alkoholen um Gemische verschiedener Isomere.

Ganz besonders bevorzugt werden C₉-Alkoholgemische mit einem ISO-Index vom 1 bis 2,5, insbesondere Nonanolgemische mit einem ISO-Index von 1,25 bzw. 1,6 eingesetzt. Der ISO-Index ist eine dimensionslose Größe, die mittels Gaschromatographie bestimmt wurde.

| | |
|---|---|
| Methode: | Kapillar GC |
| Apparatur: | Kapillar Gaschromatograph mit Autosampler, Split/Splitless Injektionssystem und Flammenionisationsdetektor (FID) |
| Chemikalien: | MSTFA (N-Methyl-N-trimethylsilyltrifluoracetamid) entsprechende Vergleichssubstanzen zur Bestimmung der Retentionszeiten |
| Probenvorbereitung: | 3 Tropfen der Probe werden in 1 ml MSTFA gelöst und für 60 Minuten bei 80 °C gehalten |
| GC Bedingungen: | Kapillarsäule Ultra-1, Länge 50 m, Innendurchmesser 0,25 mm, Filmdicke 0,1 Mikrometer, Trägergas Helium |
| Säulenvordruck | 200 psi constant |
| Split: | 80 ml/min |
| Septumspülung: | 3 ml/min |
| Ofentemperatur: | 120 °C, 25 min, isotherm |
| Injektortemperatur: | 250 °C |
| Detektortemperatur: | 250 °C (FID) |
| Injektionsvolumen: | 0,5 Mikroliter |
| Berechnung: | Die Vorgehensweise bei der Berechnung des Iso-Index wird in der folgenden Tabelle ersichtlich. |

Tabelle mit beispielhafter Berechnung des Iso-Index:

| Komponente | Name | Verzweigung | Anteil in Vol-% | Index |
|---|---|---|---|---|
| 1 | 2-Ethyl-2-methylhexanol-1 | 2 | 1,00 | 0,0200 |
| 2 | 2-Ethyl-4-methylhexanol-1 | 2 | 1,00 | 0,0200 |
| 3 | 2-Ethyl-4-methylhexanol-1 | 2 | 1,00 | 0,0200 |
| 4 | 2-Propyl-3-methylpentanol-1 | 2 | 1,00 | 0,0200 |
| 5 | 2-Propyl-hexanol-1 | 1 | 1,00 | 0,0100 |
| 6 | 2,5-Dimethylheptanol-1 | 2 | 1,00 | 0,0200 |
| 7 | 2,3-Dimethylheptanol-1 | 2 | 1,00 | 0,0200 |
| 8 | 2,3,4-Trimethylhexanol-1 | 3 | 1,00 | 0,0300 |
| 9 | 2-Ethylheptanol-1 | 1 | 1,00 | 0,0100 |
| 10 | 3-Ethyl-4-methylhexanol-1 | 2 | 82,00 | 1,6400 |
| 11 | 3-Ethylheptanol-1 | 1 | 1,00 | 0,0100 |
| 12 | 2-Methyloctanol-1 | 1 | 1,00 | 0,0100 |
| 13 | 4,5-Dimethylheptanol-1 | 2 | 1,00 | 0,0200 |
| 14 | 4,5-Dimethylheptanol-1 | 2 | 1,00 | 0,0200 |
| 15 | 4-Methyloctanol-1 | 1 | 1,00 | 0,0100 |
| 15a | 7-Methyloctanol-1 | 1 | 1,00 | 0,0000 |
| 16 | 6-Methyloctanol-1 | 1 | 1,00 | 0,0100 |
| 17 | Nonanol-1 | 0 | 1,00 | 0,0000 |
| | Unbekannte Komponente | 2 | 1,00 | 0,0200 |
| | Summe | | 99,00 | 1,9000 |
| | | | Iso-Index: | 1,9200 |

Die C₅- bis C₁₂-Alkohole werden gemäß den vorstehend genannten Verfahren hergestellt. Zur Herstellung von Benzolpolycarbonsäureestern, worin R¹ ein C₉-Alkyrest ist, wird besonders bevorzugt ein Nonanol-Gemisch eingesetzt, worin 0 bis 20 Gew.-%, bevorzugt 0,5 bis 18 Gew.-%, besonders bevorzugt 6 bis 16 Gew.-% des Nonanol-Gemisches keine Verzweigung aufweisen, 5 bis 90 Gew.-%, bevorzugt 10 bis 80 Gew.-%, besonders bevorzugt 45 bis 75 Gew.-%, eine Verzweigung, 5 bis 70 Gew.-%, bevorzugt 10 bis 60 Gew.-%, besonders bevorzugt 15 bis 35 Gew.-% zwei Verzweigungen, 0 bis 10 Gew.-%, bevorzugt 0 bis 8 Gew.-%, besonders bevorzugt 0 bis 4 Gew.-% drei Verzweigungen aufweisen und 0 bis 40 Gew.-%, bevorzugt 0,1 bis 30 Gew.-%, besonders bevorzugt 0,5 bis 6,5 Gew.-%. sonstige Komponenten sind. Unter sonstigen Komponenten sind im Allgemeinen Nonanole mit mehr als drei Verzweigungen, Decanole oder Octanole zu verstehen, wobei die Summe der genannten Komponenten 100 Gew.-% ergibt.

Die vorliegende Erfindung betrifft daher bevorzugt das erfindungsgemäße Verfahren, wobei es sich bei dem wenigstens einen Derivat der Benzolpolycarbonsäure um Mono-, Di-, Tri-, Tetraester und Anyhdride der Benzolpolycarbonsäure handelt. Bevorzugt sind alle Carbonsäuregruppen verestert. Die eingesetzten Ester sind Alkylester, wobei bevorzugte Alkylgruppen R¹ bereits vorstehend genannt sind.

Weiter bevorzugt betrifft die vorliegende Erfindung das erfindungsgemäße Verfahren, wobei es sich bei dem wenigstens einen Derivat einer Benzolpolycarbonsäure um Mono-, Di-, Tri-, Tetraester der Benzolpolycarbonsäure, wobei diese durch Umsetzung mit einem Nonanol-Gemisch, worin 0 bis 20 Gew.-%, bevorzugt 0,5 bis 18 Gew.-%, besonders bevorzugt 6 bis 16 Gew.-% des Nonanol-Gemisches keine Verzweigung aufweisen, 5 bis 90 Gew.-%, bevorzugt 10 bis 80 Gew.-%, besonders bevorzugt 45 bis 75 Gew.-%, eine Verzweigung, 5 bis 70 Gew.-%, bevorzugt 10 bis 60 Gew.-%, besonders bevorzugt 15 bis 35 Gew.-% zwei Verzweigungen, 0 bis 10 Gew.-%, bevorzugt 0 bis 8 Gew.-%, besonders bevorzugt 0 bis 4 Gew.-% drei Verzweigungen aufweisen und 0 bis 40 Gew.-%, bevorzugt 0,1 bis 30 Gew.-%, besonders bevorzugt 0,5 bis 6,5 Gew.-%. sonstige Komponenten sind, wobei die Summe der genannten Komponenten 100 Gew.-% ergibt, umgesetzt worden ist, handelt.

Eine besonders bevorzugte Ausführungsform eines Nonanol-Gemisches, das zur Herstellung von bevorzugt verwendeten Benzolpolycarbonsäureestern eingesetzt wird, weist die folgende Zusammensetzung auf:
1,73 bis 3,73 Gew.-%, bevorzugt 1,93 bis 3,53 Gew.-%, besonders bevorzugt 2,23 bis 3,23 Gew.-% 3-Ethyl-6-methyl-hexanol;
0,38 bis 1,38 Gew.-%, bevorzugt 0,48 bis 1,28 Gew.-%, besonders bevorzugt 0,58 bis 1,18 Gew.-% 2,6 Dimethylheptanol;
2,78 bis 4,78 Gew.-%, bevorzugt 2,98 bis 4,58 Gew.-%, besonders bevorzugt 3,28 bis 4,28 Gew.-% 3,5-Dimethylheptanol;
6,30 bis 16,30 Gew.-%, bevorzugt 7,30 bis 15,30 Gew.-%, besonders bevorzugt 8,30 bis 14,30 Gew.-% 3,6-Dimethylheptanol;
5,74 bis 11,74 Gew.-%, bevorzugt 6,24 bis 11,24 Gew.-%, besonders bevorzugt 6,74 bis 10,74 Gew.-% 4,6-Dimethylheptanol;
1,64 bis 3,64 Gew.-%, bevorzugt 1,84 bis 3,44 Gew.-%, besonders bevorzugt 2,14 bis 3,14 Gew.-% 3,4,5-Trimethylhexanol;
1,47 bis 5,47 Gew.-%, bevorzugt 1,97 bis 4,97 Gew.-%, besonders bevorzugt 2,47 bis 4,47 Gew.-% 3,4,5-Trimethylhexanol, 3-Methyl-4-ethylhexanol und 3-Ethyl-4-methylhexanol;
4,00 bis 10,00 Gew.-%, bevorzugt 4,50 bis 9,50 Gew.-%, besonders bevorzugt 5,00 bis 9,00 Gew.-% 3,4-Dimethylheptanol;
0,99 bis 2,99 Gew.-%, bevorzugt 1,19 bis 2,79 Gew.-%, besonders bevorzugt 1,49 bis 2,49 Gew.-% 4-Ethyl-5-methylhexanol und 3-Ethylheptanol;
2,45 bis 8,45 Gew.-%, bevorzugt 2,95 bis 7,95 Gew.-%, besonders bevorzugt 3,45 bis 7,45 Gew.-% 4,5-Dimethylheptanol und 3-Methyloctanol;
1,21 bis 5,21 Gew.-%, bevorzugt 1,71 bis 4,71 Gew.-%, besonders bevorzugt 2,21 bis 4,21 Gew.-% 4,5-Dimethylheptanol;
1,55 bis 5,55 Gew.-%, bevorzugt 2,05 bis 5,05 Gew.-%, besonders bevorzugt 2,55 bis 4,55 Gew.-% 5,6-Dimethylheptanol;
1,63 bis 3,63 Gew.-%, bevorzugt 1,83 bis 3,43 Gew.-%, besonders bevorzugt 2,13 bis 3,13 Gew.-% 4-Methyloctanol;
0,98 bis 2,98 Gew.-%, bevorzugt 1,18 bis 2,78 Gew.-%, besonders bevorzugt 1,48 bis 2,48 Gew.-% 5-Methyloctaonol;
0,70 bis 2,70 Gew.-%, bevorzugt 0,90 bis 2,50 Gew.-%, besonders bevorzugt 1,20 bis 2,20 Gew.-% 3,6,6-Trimethylhexanol;
1,96 bis 3,96 Gew.-%, bevorzugt 2,16 bis 3,76 Gew.-%, besonders bevorzugt 2,46 bis 3,46 Gew.-% 7-Methyloctanol;
1,24 bis 3,24 Gew.-%, bevorzugt 1,44 bis 3,04 Gew.-%, besonders bevorzugt 1,74 bis 2,74 Gew.-% 6-Methyloctanol; 0,1 bis 3 Gew.-%, bevorzugt 0,2 bis 2 Gew.-%, besonders bevorzugt 0,3 bis 1 Gew.-% n-Nonanol;
25 bis 35 Gew.-%, bevorzugt 28 bis 33 Gew.-%, besonders bevorzugt 29 bis 32 Gew.-% sonstige Alkohole mit 9 und 10 Kohlenstoffatomen;
wobei die Gesamtsumme der genannten Komponenten 100 Gew.-% ergibt.

Ein solches Isononanol-Gemisch liegt verestert mit Phthalsäure im Diisononylphthalat der CAS Nr. 68515-48-0 vor, aus dem der Cyclohexan-1,2-dicarbonsäurediisononylester mit entsprechender Isononyl-Komponente durch das erfindungsgemäße Verfahren durch Hydrierung des aromatischen Kerns erzeugt werden kann. Solche Isononanol-Gemische können über den Weg der Zeolith-katalysierten Oligomerisierung von C₂-, C₃- und C₄-Olefingemischen, dem sogenannten Polygas-Prozess, Gewinnung einer C₈-Fraktion aus dem Oligomerisat und deren anschließende Hydroformylierung und Hydrierung erhalten werden.

Eine weitere besonders bevorzugte Ausführungsform eines Nonanol-Gemisches, das zur Herstellung von bevorzugt verwendeten Benzolpolycarbonsaureestern eingesetzt wird, weist die folgende Zusammensetzung auf:
6,0 bis 16,0 Gew.-%, bevorzugt 7,0 bis 15,0 Gew.-%, besonders bevorzugt 8,0 bis 14,0 Gew.-% n-Nonanol;
12,8 bis 28,8 Gew.-%, bevorzugt 14,8 bis 26,8 Gew.-%, besonders bevorzugt 15,8 bis 25,8 Gew.-% 6-Methyloctanol;
12,5 bis 28,8 Gew.-%, bevorzugt 14,5 bis 26,5 Gew.-%, besonders bevorzugt 15,5 bis 25,5 Gew.-% 4-Methyloctanol;
3,3 bis 7,3 Gew.-%, bevorzugt 3,8 bis 6,8 Gew.-%, besonders bevorzugt 4,3 bis 6,3 Gew.-% 2-Methyloctanol;
5,7 bis 11,7 Gew.-%, bevorzugt 6,3 bis 11,3 Gew.-%, besonders bevorzugt 6,7 bis 10,7 Gew.-% 3-Ethylheptanol;
1,9 bis 3,9 Gew.-%, bevorzugt 2,1 bis 3,7 Gew.-%, besonders bevorzugt 2,4 bis 3,4 Gew.-% 2-Ethylheptanol;
1,7 bis 3,7 Gew.-%, bevorzugt 1,9 bis 3,5 Gew.-%, besonders bevorzugt 2,2 bis 3,2 Gew.-% 2-Propylhexanol;
3,2 bis 9,2 Gew.-%, bevorzugt 3,7 bis 8,7 Gew.-%, besonders bevorzugt 4,2 bis 8,2 Gew.-% 3,5-Dimethylheptanol; 6,0 bis 16,0 Gew.-%, bevorzugt 7,0 bis 15,0 Gew.-%, besonders bevorzugt 8,0 bis 14,0 Gew.-% 2,5-Dimethylheptanol;
1,8 bis 3,8 Gew.-%, bevorzugt 2,0 bis 3,6 Gew.-%, besonders bevorzugt 2,3 bis 3,3 Gew.-% 2,3-Dimethylheptanol;
0,6 bis 2,6 Gew.-%, bevorzugt 0,8 bis 2,4 Gew.-%, besonders bevorzugt 1,1 bis 2,1 Gew.-% 3-Ethyl-4-methylhexanol;
2,0 bis 4,0 Gew.-%, bevorzugt 2,2 bis 3,8 Gew.-%, besonders bevorzugt 2,5 bis 3,5 Gew.-% 2-Ethyl-4-methylhexanol;
0,5 bis 6,5 Gew.-%, bevorzugt 1,5 bis 6 Gew.-%, besonders bevorzugt 1,5 bis 5,5 Gew.-% sonstige Alkohole mit 9 Kohlenstoffatomen;
wobei die Gesamtsumme der genannten Komponenten 100 Gew.-% ergibt.

Ein solches Isononanol-Gemisch liegt verestert mit Phthalsäure im Diisononylphthalat der CAS Nr. 28553-12-0 vor, aus dem der Cyclohexan-1,2-dicarbonsäurediisononylester mit entsprechender Isononyl-Komponente durch die erfindungsgemäße Hydrierung des aromatischen Kerns erzeugt werden kann. Solche Isononanol-Gemische können über den Weg der Dimerisierung von vorwiegend n-Butenen zu Octen-Gemischen mittels Nickel-haltigen Katalysatoren, beispielsweise nach dem Verfahren der WO 95/14647, anschließende Hydroformylierung des erhaltenen Octen-Gemisches, vorzugsweise Kobalt-katalysierte Hydroformylierung, und Hydrierung erhalten werden.

Ganz besonders bevorzugte Produkte des erfindungsgemäßen Verfahrens sind ausgewählt aus der Gruppe, bestehend aus Cyclohexan-1,2-dicarbonsäuredi-n-octylester, Cyclohexan-1,2-dicarbonsäurediisooctylester, Cyclohexan-1,2-dicarbonsäuredi-(2-ethylhexyl)-ester, Cyclohexan-1,2-dicarbonsäuredi-n-nonylester, Cyclohexan-1,2-dicarbonsäurediisononylester, Cyclohexan-1,2-dicarbonsäuredi-(2-propylheptyl)-ester, Cyclohexan-1,2-dicarbonsäuredi-n-decyl-ester, Cyclohexan-1,2-dicarbonsäurediisodecylester und Mischungen davon.

Weitere erfindungsgemäß bevorzugt erhaltene Produkte sind die in der WO 99/32427 offenbarten, im Folgenden nochmals aufgelisteten Cyclohexan-1,2-dicarbonsäureester:
Cyclohexan-1,2-dicarbonsäuredi(isononyl)ester, erhältlich durch die erfindungsgemäße Hydrierung eines Di(isononyl)phthalats mit der CAS Nr. 68515-48-0;
Cyclohexan-1,2-dicarbonsäuredi(isononyl)ester, erhältlich durch die erfindungsgemäße Hydrierung eines Di(isononyl)phthalats mit der CAS Nr. 28553-12-0, basierend auf n-Buten;
Cyclohexan-1,2-dicarbonsäuredi(isononyl)ester, erhältlich durch die erfindungsgemäße Hydrierung eines Di(isononyl)phthalats mit der CAS Nr. 28553-12-0 basierend auf Isobuten;
ein 1,2-Di-C₉-Ester der Cyclohexandicarbonsäure, erhältlich durch die erfindungsgemäße Hydrierung eines Di(nonyl)phthalats mit der CAS Nr. 68515-46-8;
ein Cyclohexan-1,2-dicarbonsäuredi(isodecyl)ester, erhältlich durch die erfindungsgemäße Hydrierung eines Di(isodecyl)phthalats mit der CAS Nr. 68515-49-1;
ein 1,2-Di(isodecyl)cyclohexandicarbonsäureester, erhältlich durch die erfindungsgemäße Hydrierung eines Di(isodecyl)phthalats, das hauptsächlich aus Di-(2-propylheptyl)phthalat besteht;
Des Weiteren sind auch die kommerziell erhältlichen Benzolcarbonsäureester mit den Handelsnamen Jayflex DINP (CAS Nr. 68515-25 48-0), Jayflex DIDP (CAS Nr. 68515-49-1), Palatinol 9-P, Vestinol 9 (CAS Nr. 28553-12-0), Palatinol N (CAS Nr. 28553-12-0), Jayflex DIOP (CAS Nr. 27554-26-3), Palatinol AH (CAS Nr. 117-81-7) und Palatinol Z (CAS Nr. 26761-40-0) geeignete Edukte für das erfindungsgemäße Verfahren.

### Verfahren zur Herstellung eines Cyclohexanpolycarbonsäureesters

Die Benzolpolycarbonsäureester aus der zuvor beschriebenen Aufarbeitung eines Rohesters eignen sich aufgrund Ihres geringen Gehalts an Nebenprodukten, speziell an Di-(C₄-C₁₂-alkyl)ethern, in vorteilhafterweise zur Herstellung von Cyclohexanpolycarbonsäureestern.

Ein weiterer Gegenstand der Erfindung ist ein Verfahren zur Herstellung eines Cyclohexanpolycarbonsäureesters, bei dem man
i) einen Rohester aus der Veresterung einer Benzolpolycarbonsäure mit wenigstens einem C₄-C₁₂-Monoalkanol bereitstellt,
ii) den in Schritt i) bereitgestellten Rohester einer Aufarbeitung unterzieht, wie zuvor definiert, wobei ein gegenüber dem Rohester an Di-(C₄-C₁₂-alkyl)ethern abgereicherter Benzolpolycarbonsäureester erhalten wird,
iii) den in Schritt ii) erhaltenen, an Di-(C₄-C₁₂-alkyl)ethern abgereicherten, Benzolpolycarbonsäureester einer Hydrierung mit einem wasserstoffhaltigen Gas in Gegenwart eines Hydrierkatalysators unterzieht.

Bezüglich der Bereitstellung des Rohesters aus der Veresterung einer Benzolpolycarbonsäure mit wenigstens einem C₄-C₁₂-Monoalkanol in Schritt i) wird auf die vorherigen Ausführungen in vollem Umfang Bezug genommen. Des Weiteren wird bezüglich der Aufarbeitung des Rohesters in Schritt ii) auf die vorherigen Ausführungen in vollem Umfang Bezug genommen.

Es wurde gefunden, dass man Cyclohexanpolycarbonsäureester mit einem verringerten Anteil an Nebenprodukten, insbesondere Di-(C₄-C₁₂-alkyl)ethern, erhält, wenn die Hydrierung der entsprechenden Benzolpolycarbonsäureester nach dem im Folgenden beschriebenen Herstellungsverfahren erfolgt. Insbesondere gelingt so die Herstellung mit einem geringen Anteil an Diisononylethern.

Das erfindungsgemäße Inkontaktbringen der wenigstens einen Benzolpolycarbonsäureester mit einem Wasserstoff enthaltenden Gas resultiert in einer Hydrierung dieser Verbindungen, um die gewünschte wenigstens eine Cyclohexanpolycarbonsäureester zu erhalten. Erfindungsgemäß bevorzugt wird nur das aromatische System hydriert, d. h. reduziert, um das entsprechende gesättigte cycloaliphatische System zu erhalten, d. h. ggf. weitere in dem wenigstens einen Substrat vorliegende reduzierbare Gruppen werden erfindungsgemäß bevorzugt nicht reduziert.

Für das erfindungsgemäße Verfahren geeignete Katalysatoren enthalten mindestens ein Aktivmetall, welches als Vollkontakt-Katalysator oder aufgebracht auf einem Träger eingesetzt werden kann.

Als Aktivmetall können prinzipiell alle Metalle der VIII. Nebengruppe des Periodensystems eingesetzt werden. Vorzugsweise werden als Aktivmetalle Platin, Rhodium, Palladium, Cobalt, Nickel oder Ruthenium oder ein Gemisch aus zwei oder mehr davon eingesetzt, wobei insbesondere Ruthenium oder Nickel als Aktivmetall verwendet werden. Unter den ebenfalls verwendbaren Metallen der I., II. oder VII. Nebengruppe des Periodensystems, die ebenfalls allesamt prinzipiell verwendbar sind, werden vorzugsweise Kupfer, Zink und/oder Rhenium eingesetzt.

Entsprechende Katalysatoren sind zum Beispiel in Ullmann's Encyclopedia of Industrial Chemistry Vol. 18, Kapitel Hydrogenation and Dehydrogenation, Seiten 483-541, beschrieben.

Geeignete Aktivmetalle können auf einem inerten Träger aufgebracht sein, geeignete Trägermaterialien sind z. B. Aktivkohlen, Metalloxide und Zeolithe. Bevorzugt sind Katalysatoren, die als Trägermaterial Aluminiumoxid, Siliziumdioxid, Kohle enthalten oder Mischoxide, die Aluminiumoxid und/oder Siliziumdioxid enthalten. Geeignete Materialien sind z. B. in U/lmann's Encyclopedia of Industrial Chemistry Vol. 17, Kapitel Heterogeneous Catalysis and Solid Catalysts, 2. Development and Types of Solid Catalysts, Seiten 483-541, beschrieben.

Der Gehalt des Aktivmetalls beträgt im Fall von Platinmetallen (Ruthenium, Rhodium, Palladium, Platin) im allgemeinen 0,01 bis 30 Gew.-%, vorzugsweise 0,01 bis 5 Gew.-% und insbesondere 0,1 bis 5 Gew.-%, jeweils bezogen auf das Gesamtgewicht des verwendeten Katalysators, wobei die bei den im Folgenden beschriebenen, vorzugsweise eingesetzten Katalysatoren 1 bis 3 vorzugsweise verwendeten Gehalte nochmals bei der Diskussion dieser Katalysatoren einzeln angegeben sind. Im Falle von Nickel oder Cobalt als Aktivmetall beträgt der Aktivmetallgehalt im Allgemeinen zwischen 5 und 100 Gew-%, bezogen auf das Gesamtgewicht des Katalysators.

Im Folgenden werden die erfindungsgemäß bevorzugt verwendeten Katalysatoren detailliert beschrieben. Dabei erfolgt die Beschreibung beispielhaft unter Bezugnahme auf die Verwendung von Ruthenium als Aktivmetall. Die unten stehenden Angaben sind auch auf die anderen geeigneten Aktivmetalle, wie zuvor definiert, übertragbar.

### Katalysator 1

Die erfindungsgemäß verwendeten Katalysatoren 1 können technisch hergestellt werden durch Auftragen mindestens eines Metalls der VIII. Nebengruppe des Periodensystems und gegebenenfalls mindestens eines Metalls der I. oder VII. Nebengruppe des Periodensystems auf einen geeigneten Träger.

Die Auftragung kann durch Tränken des Trägers in wässrigen Metallsalzlösungen, wie z. B. wässrigen Rutheniumsalzlösungen, durch Aufsprühen entsprechender Metallsalzlösungen auf den Träger oder durch andere geeignete Verfahren erreicht werden. Als Metallsalze der I., VII. oder VIII. Nebengruppe des Periodensystems eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle, wobei die Nitrate und Nitrosylnitrate bevorzugt sind.

Bei Katalysatoren, die neben dem Metall der VIII. Nebengruppe des Periodensystems noch weitere Metalle als Aktivmetall auf dem Träger aufgetragen enthalten, können die Metallsalze bzw. Metallsalzlösungen gleichzeitig oder nacheinander aufgebracht werden.

Die mit der Metallsalzlösung beschichteten bzw. getränkten Träger werden anschließend, vorzugsweise bei Temperaturen von 100 bis 150 °C, getrocknet und wahlweise bei Temperaturen von 200 bis 600 °C, vorzugsweise von 350 bis 450 °C calziniert. Bei getrennter Auftränkung wird der Katalysator nach jedem Tränkschritt getrocknet und wahlweise calziniert, wie oben beschrieben. Die Reihenfolge, in der die Aktivkomponenten aufgetränkt werden, ist dabei frei wählbar.

Anschließend werden die beschichteten und getrockneten sowie wahlweise calcinierten Träger durch Behandlung in einem Gasstrom, der freien Wasserstoff enthält, bei Temperaturen von 30 bis 600 °C, vorzugsweise von 150 bis 450 °C aktiviert. Vorzugsweise besteht der Gasstrom aus 50 bis 100 Vol.-% H₂ und 0 bis 50 Vol.-% N₂.

Die Metallsalzlösung oder -lösungen werden in einer solchen Menge auf den oder die Träger aufgebracht, dass der Gesamtgehalt an Aktivmetall, jeweils bezogen auf das Gesamtgewicht des Katalysators, 0,01 bis 30 Gew.-%, vorzugsweise 0,01 bis 5 Gew.-%, weiter bevorzugt 0,01 bis 1 Gew.-%, und insbesondere 0,05 bis 1 Gew.-% beträgt.

Die Metalloberfläche auf dem Katalysator 1 beträgt dabei insgesamt vorzugsweise 0,01 bis 10 m²/g, weiter bevorzugt 0,05 bis 5 m²/g und insbesondere 0,05 bis 3 m²/g des Katalysators. Die Metalloberfläche wird mittels der von J. Lemaitre et al. in Characterization of Heterogeneous Catalysts, Hrsg. Francis Delanney, Marcel Dekker, New York 1984, S. 310 - 324, beschriebenen Chemisorptionsverfahren bestimmt.

Im erfindungsgemäß verwendeten Katalysator 1 beträgt das Verhältnis der Oberflächen des/der Aktivmetalls/-metalle und des Katalysatorträgers vorzugsweise weniger als 0,05, wobei der untere Grenzwert bei 0,0005 liegt.

Die zur Herstellung der erfindungsgemäß verwendeten Katalysatoren verwendbaren Trägermaterialien sind solche, die makroporös sind und einen mittleren Porendurchmesser von mindestens 50 nm, vorzugsweise mindestens 100 nm, insbesondere mindestens 500 nm aufweisen und deren Oberfläche nach BET bei höchstens 30 m²/g, vorzugsweise höchstens 15 m²/g, weiter bevorzugt höchstens 10 m²/g, insbesondere höchstens 5 m²/g und weiter bevorzugt höchstens 3 m²/g liegt. Der mittlere Porendurchmesser des Trägers beträgt vorzugsweise 100 nm bis 200 µm, weiter bevorzugt 500 nm bis 50 µm. Die Oberfläche des Trägers beträgt vorzugsweise 0,2 bis 15 m²/g, weiter bevorzugt 0,5 bis 10 m²/g, insbesondere 0,5 bis 5 m²/g und weiter bevorzugt 0,5 bis 3 m²/g.

Die Oberfläche des Trägers wird bestimmt nach dem BET-Verfahren durch N₂-Adsorption, insbesondere nach DIN 66131. Die Bestimmung des mittleren Porendurchmessers und der Porengrößenverteilung erfolgt durch Hg-Porosimetrie, insbesondere nach DIN 66133.

Vorzugsweise kann die Porengrößenverteilung des Trägers annähernd bimodal sein, wobei die Porendurchmesserverteilung mit Maxima bei etwa 600 nm und etwa 20 µm bei der bimodalen Verteilung eine spezielle Ausführungsform der Erfindung darstellt.

Weiter bevorzugt ist ein Träger mit einer Oberfläche von 1,70 bis 180 m²/g, beispielsweise 1,75 m²/g, der diese bimodale Verteilung des Porendurchmessers aufweist. Das Porenvolumen dieses bevorzugten Trägers beträgt vorzugsweise etwa 0,50 bis 0,60 ml/g, beispielsweise 0,53 ml/g.

Als makroporöses Trägermaterial verwendbar sind beispielsweise Makroporen aufweisende Aktivkohle, Siliziumcarbid, Aluminiumoxid, Siliziumdioxid, Titandioxid, Zirkoniumdioxid, Magnesiumoxid, Zinkoxid oder Gemische aus zwei oder mehr davon, wobei Aluminiumoxid und Zirkoniumdioxid vorzugsweise verwendet werden.

Weitere Details bezüglich Katalysator 1 bzw. zu seiner Herstellung sind der DE-A 25 196 24 484 zu entnehmen, deren diesbezüglicher Inhalt durch Bezugnahme vollständig in die vorliegende Anmeldung einbezogen wird.

### Katalysator 2

Die erfindungsgemäß verwendeten Katalysatoren 2 enthalten ein oder mehrere Metalle der VIII. Nebengruppe des Periodensystems als Aktivkomponente(n) auf einem Träger, wie hierin definiert. Bevorzugt werden Ruthenium, Palladium und/oder Rhodium als Aktivkomponente(n) verwendet.

Die erfindungsgemäß verwendeten Katalysatoren 2 können technisch hergestellt werden durch Auftragen mindestens eines Aktivmetalls der VIII. Nebengruppe des Periodensystems, vorzugsweise Ruthenium oder Palladium und gegebenenfalls mindestens eines Metalls der I. oder VII. Nebengruppe des Periodensystems auf einen geeigneten Träger. Die Auftragung kann durch Tränken des Trägers in wässrigen Metallsalzlösungen, wie z. B. Ruthenium- oder Palladiumsalzlösungen, durch Aufsprühen entsprechender Metallsalzlösungen auf den Träger oder durch andere geeignete Verfahren erreicht werden. Als Metallsalze zur Herstellung der Metallsalzlösungen eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle, wobei die Nitrate und Nitrosylnitrate bevorzugt sind.

Bei Katalysatoren, die mehrere Aktivmetalle auf den Träger aufgetragen enthalten, können die Metallsalze bzw. Metallsalzlösungen gleichzeitig oder nacheinander aufgebracht werden.

Die mit der Metallsalzlösung beschichteten bzw. getränkten Träger werden anschließend getrocknet, wobei Temperaturen von 100 bis 150 °C bevorzugt sind. Wahlweise können diese Träger bei Temperaturen von 200 bis 600 °C, vorzugsweise von 350 bis 450 °C calziniert werden. Anschließend werden die beschichteten Träger durch Behandlung in einem Gasstrom, der freien Wasserstoff enthält, bei Temperaturen von 30 bis 600 °C, vorzugsweise von 100 bis 450 °C und insbesondere von 100 bis 300 °C aktiviert. Der Gasstrom besteht vorzugsweise aus 50 bis 100 Vol.-% H₂ und 0 bis 50 Vol.-% N₂.

Werden auf die Träger mehrere Aktivmetalle aufgetragen und erfolgt das Auftragen nacheinander, so kann der Träger nach jedem Auftragen bzw. Tränken bei Temperaturen von 100 bis 150 °C getrocknet werden und wahlweise bei Temperaturen von 200 bis 600 °C calziniert werden. Dabei kann die Reihenfolge, in der die Metallsalzlösung aufgetragen oder aufgetränkt wird, beliebig gewählt werden.

Die Metallsalzlösung wird in einer solchen Menge auf den/die Träger aufgebracht, dass der Gehalt an Aktivmetall 0,01 bis 30 Gew.-%, vorzugsweise 0,01 bis 10 Gew.-%, weiter bevorzugt 0,01 bis 5 Gew.-%, und insbesondere 0,3 bis 1 Gew.-%, bezogen auf das Gesamtgewicht des Katalysators, beträgt.

Die Metalloberfläche auf dem Katalysator beträgt insgesamt vorzugsweise 0,01 bis 10 m²/g, besonders bevorzugt 0,05 bis 5 m²/g und weiter bevorzugt 0,05 bis 3 m²/g des Katalysators. Die Metalloberfläche wurde durch das Chemisorptionsverfahren gemessen, wie es in J. Lemaitre et al., Characterization of Heterogeneous Catalysts, Hrsg. Francis Delanney, Marcel Dekker, New York (1984), S. 310 - 324, beschrieben ist.

Im erfindungsgemäß verwendeten Katalysator 2 beträgt das Verhältnis der Oberflächen des mindestens einen Aktivmetalls und des Katalysatorträgers weniger als 0,3, vorzugsweise weniger als 0,1 und insbesondere 0,05 oder weniger, wobei der untere Grenzwert bei 0,0005 liegt.

Die zur Herstellung der erfindungsgemäß verwendeten Katalysatoren 2 verwendbaren Trägermaterialien besitzen Makroporen und Mesoporen.

Dabei weisen die erfindungsgemäß bevorzugt verwendbaren Träger eine Porenverteilung auf, der gemäß 5 bis 50 %, vorzugsweise 10 bis 45 %, weiter bevorzugt 10 bis 30 % und insbesondere 15 bis 25 % des Porenvolumens von Makroporen mit Porendurchmessern im Bereich von 50 nm bis 10.000 nm und 50 bis 95 %, vorzugsweise 55 bis 90 %, weiter bevorzugt 70 bis 90 % und insbesondere 75 bis 85 % des Porenvolumens von Mesoporen mit einem Porendurchmesser von 2 bis 50 nm gebildet werden, wobei sich jeweils die Summe der Anteile der Porenvolumina zu 100 % addiert.

Das Gesamtporenvolumen der erfindungsgemäß verwendeten Träger beträgt 0,05 bis 1,5 cm³/g, vorzugsweise 0,1 bis 1,2 cm³/g und insbesondere 0,3 bis 1,0 cm³/g. Der mittlere Porendurchmesser der erfindungsgemäß verwendeten Träger beträgt 5 bis 20 nm, vorzugsweise 8 bis 15 nm und insbesondere 9 bis 12 nm.

Vorzugsweise beträgt die Oberfläche des Trägers 50 bis 500 m²/g, weiter bevorzugt 200 bis 350 m²/g und insbesondere 250 bis 300 m²/g des Trägers.

Die Oberfläche des Trägers wird nach dem BET-Verfahren durch N₂-Adsorption, insbesondere nach DIN 66131, bestimmt. Die Bestimmung des mittleren Porendurchmessers und der Größenverteilung erfolgt durch Hg-Porosimetrie, insbesondere nach DIN 66133.

Obwohl prinzipiell alle bei der Katalysatorherstellung bekannten Trägermaterialien, d. h. die die oben definierte Porengrößenverteilung aufweisen, eingesetzt werden können, werden vorzugsweise Makroporen aufweisende Aktivkohle, Siliziumcarbid, Aluminiumoxid, Siliziumdioxid, Titandioxid, Zirkoniumdioxid, Magnesiumoxid, Zinkoxid oder deren Gemische, weiter bevorzugt Aluminiumoxid und Zirkoniumdioxid, eingesetzt.

Eine spezielle Ausführungsform sind Katalysatoren, die als Aktivmetall Ruthenium alleine oder in Kombination mit wenigstens einem weiteren Metall der I., VII. oder VIII. Nebengruppe des Periodensystems der Elemente, in einer Menge des Aktivmetalls von 0,01 bis 30 Gew.-%, bezogen auf das Gesamtgewicht des Katalysators, aufgebracht auf einem Träger, umfassen oder Katalysatoren, die als Aktivmetall Palladium alleine oder in Kombination mit wenigstens einem weiteren Metall der I., VII. oder VIII. Nebengruppe des Periodensystems der Elemente, in einer Menge des Aktivmetalls von 0,01 bis 30 Gew.-%, bezogen auf das Gesamtgewicht des Katalysators, aufgebracht auf einem Träger, umfassen, wobei jeweils 5 bis 50 % des Porenvolumens des Trägers von Makroporen mit einem Porendurchmesser im Bereich von 50 nm bis 10.000 nm und 50 bis 95 % des Porenvolumens des Trägers von Mesoporen mit einem Porendurchmesser im Bereich von 2 bis weniger als 50 nm gebildet werden, wobei sich die Summe der Porenvolumina zu 100 % addiert. Insbesondere wird als Aktivmetall Ruthenium alleine oder in Kombination mit wenigstens einem weiteren Metall der I., VII. oder VIII. Nebengruppe des Periodensystems der Elemente eingesetzt.

Weitere Details bezüglich Katalysators 2 bzw. zu seiner Herstellung sind der EP-A-0814098 und der DE-A 196 24 485, als einem der Prioritätsdokumente der EP-A-0814098, zu entnehmen. Der Inhalt dieser Dokumente wird durch Bezugnahme vollständig in die vorliegende Anmeldung einbezogen.

Der Einsatz der Katalysatoren 2 als Hydrierkatalysatoren ist eine besonders bevorzugte Ausführungsform des erfindungsgemäßen Verfahrens.

### Katalysator 3

Die erfindungsgemäß verwendeten Katalysatoren 3 können technisch hergestellt werden durch Auftragen eines Aktivmetalls der VIII. Nebengruppe des Periodensystems und gegebenenfalls mindestens eines Metalls der I. oder VII. Nebengruppe des Periodensystems auf einen geeigneten Träger. Die Auftragung kann durch Tränken des Trägers in wässrigen Metallsalzlösungen, wie z. B. Rutheniumsalzlösungen, durch Aufsprühen entsprechender Metallsalzlösungen auf den Träger oder durch andere geeignete Verfahren erreicht werden. Als Rutheniumsalze zur Herstellung der Rutheniumsalzlösungen wie auch als Metallsalze der I., VII. oder VIII. Nebengruppe eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle, bevorzugt sind dabei die Nitrate und Nitrosylnitrate.

Bei Katalysatoren, die mehrere Metalle auf den Träger aufgetragen enthalten, können die Metallsalze bzw. Metallsalzlösungen gleichzeitig oder nacheinander aufgebracht werden.

Die mit der Rutheniumsalz- bzw. Metallsalzlösung beschichteten bzw. getränkten Träger werden sodann getrocknet, vorzugsweise bei Temperaturen von 100 bis 150 °C, und wahlweise bei Temperaturen von 200 bis 600 °C calziniert.

Darauffolgend werden die beschichteten Träger aktiviert durch Behandlung der beschichteten Träger in einem Gasstrom, der freien Wasserstoff enthält, bei Temperaturen von 30 bis 600 °C, vorzugsweise von 150 bis 450 °C. Der Gasstrom besteht vorzugsweise aus 50 bis 100 Vol.-% H₂ und 0 bis 50 Vol.-% N₂.

Werden auf die Träger neben dem Aktivmetall der VIII. Nebengruppe des Periodensystems Metalle der I. oder VII. Nebengruppe aufgetragen und erfolgt das Auftragen nacheinander, so kann der Träger nach jedem Auftragen bzw. Tränken bei Temperaturen von 100 bis 150 °C getrocknet werden und wahlweise bei Temperaturen von 200 bis 600 °C calziniert werden. Dabei kann die Reihenfolge, in der die Metallsalzlösungen aufgetragen oder aufgetränkt werden, beliebig gewählt werden.

Die Metallsalzlösung wird in einer solchen Menge auf den oder die Träger aufgebracht, dass 0,01 bis 30 Gew.-%, bezogen auf das Gesamtgewicht des Katalysators, an Aktivmetall auf den Träger aufgebracht vorliegen. Vorzugsweise beträgt diese Menge 0,2 bis 15 Gew.-%, besonders bevorzugt etwa 0,5 Gew.-%.

Die Metalloberfläche auf dem Katalysator 3 beträgt insgesamt vorzugsweise 0,01 bis 10 m²/g, besonders bevorzugt 0,05 bis 5 m²/g, insbesondere 0,05 bis 3 m² pro g des Katalysators.

Die zur Herstellung der erfindungsgemäß verwendeten Katalysatoren 3 verwendbaren Trägermaterialien sind vorzugsweise solche, die makroporös sind und einen mittleren Porendurchmesser von mindestens 0,1 µm, vorzugsweise mindestens 0,5 µm, und eine Oberfläche von höchstens 15 m²/g aufweisen, vorzugsweise höchstens 10 m²/g, besonders bevorzugt höchstens 5 m²/g, insbesondere höchstens 3 m²/g.

Bevorzugt liegt der mittlere Porendurchmesser des Trägers in einem Bereich von 0,1 bis 200 µm, insbesondere von 0,5 bis 50 µm. Bevorzugt beträgt die Oberfläche des Trägers 0,2 bis 15 m²/g, besonders bevorzugt 0,5 bis 10 m²/g, insbesondere 0,5 bis 5 m²/g, speziell 0,5 bis 3 m²/g des Trägers.

Die Oberfläche des Trägers wird bestimmt nach dem BET-Verfahren durch N₂-Adsorption, insbesondere nach DIN 66131. Die Bestimmung des mittleren Porendurchmessers und der Porengrößenverteilung erfolgte durch Hg-Porosimetrie, insbesondere nach DIN 66133. Vorzugsweise kann die Porengrößenverteilung des Trägers annähernd bimodal sein, wobei die Porendurchmesserverteilung mit Maxima bei etwa 0,6 µm und etwa 20 µm bei der bimodalen Verteilung eine spezielle Ausführungsform der Erfindung darstellt.

Besonders bevorzugt ist ein Träger mit einer Oberfläche von etwa 1,75 m²/g, der diese bimodale Verteilung des Porendurchmessers aufweist. Das Porenvolumen dieses bevorzugten Trägers beträgt vorzugsweise etwa 0,53 ml/g.

Als makroporöses Trägermaterial verwendbar sind beispielsweise Makroporen aufweisende Aktivkohle, Siliziumcarbid, Aluminiumoxid, Siliziumdioxid, Titandioxid, Zirkoniumdioxid, Magnesiumoxid, Zinkoxid oder deren Gemische. Bevorzugt sind Aluminiumoxid und Zirkoniumdioxid.

Weitere Details bezüglich Katalysator 3 bzw. zu seiner Herstellung sind der DE-A 196 04 791.9 zu entnehmen, deren diesbezüglicher Inhalt durch Bezugnahme vollständig in die vorliegende Anmeldung einbezogen wird.

### Katalysator 4:

Erfindungsgemäß besonders geeignet sind Schalenkatalysatoren (Katalysatoren 4) mit einem Aktivmetall auf einem Träger. Entsprechende Schalenkatalysatoren sind in der WO 2011/082991 genannt. Der Inhalt der WO 2011/082991 wird durch Bezugnahme vollständig in die vorliegende Anmeldung einbezogen.

Besonders bevorzugt ist ein entsprechender Schalenkatalysator, enthaltend ein Aktivmetall ausgewählt aus der Gruppe bestehend aus Ruthenium, Rhodium, Palladium, Platin und Mischungen davon, aufgebracht auf ein Trägermaterial enthaltend Siliziumdioxid, wobei das Porenvolumen des Trägermaterials 0,6 bis 1,0 ml/g, bestimmt durch Hg-Porosimetrie, beträgt, die BET-Oberfläche 280 bis 500 m²/g beträgt, und mindestens 90 % der vorhandenen Poren einen Durchmesser von 6 bis 12 nm aufweisen.

Die vorliegende Erfindung betrifft daher bevorzugt das erfindungsgemäße Verfahren, wobei als Katalysator ein Schalenkatalysator, enthaltend ein Aktivmetall, ausgewählt aus der Gruppe bestehend aus Ruthenium, Rhodium, Palladium, Platin und Mischungen davon, aufgebracht auf ein Trägermaterial enthaltend Siliziumdioxid, wobei das Porenvolumen des Trägermaterials 0,6 bis 1,0 ml/g, bestimmt durch Hg-Porosimetrie, beträgt, die BET-Oberfläche 280 bis 500 m²/g beträgt, und mindestens 90 % der vorhandenen Poren einen Durchmesser von 6 bis 12 nm aufweisen, eingesetzt wird.

Der bevorzugt eingesetzte Schalenkatalysator enthält ein Aktivmetall ausgewählt aus der Gruppe bestehend aus Ruthenium, Rhodium, Palladium, Platin und Mischungen davon, bevorzugt Ruthenium, ganz besonders bevorzugt Ruthenium als einziges Aktivmetall.

In dem erfindungsgemäß bevorzugt eingesetzten Schalenkatalysator beträgt die Menge des Aktivmetalls im Allgemeinen weniger als 1 Gew.-%, bevorzugt 0,1 bis 0,5 Gew.-%, besonders bevorzugt 0,25 bis 0,35 Gew.-%, bezogen auf das Gesamtgewicht des Katalysators.

Schalenkatalysatoren sind dem Fachmann an sich bekannt. Im Rahmen der vorliegenden Erfindung bedeutet der Begriff "Schalenkatalysator", dass das vorhandene mindestens eine Aktivmetall, bevorzugt Ruthenium, zum überwiegenden Teil in einer äu-ßeren Schale des Trägermaterials vorliegt.

In dem erfindungsgemäß bevorzugt eingesetzten Schalenkatalysatoren liegen bevorzugt 40 bis 70 Gew.-%, besonders bevorzugt 50 bis 60 Gew.-% des Aktivmetalls, bezogen auf die Gesamtmenge des Aktivmetalls, in der Schale des Katalysators bis zu einer Eindringtiefe von 200 µm vor. In einer besonders bevorzugten Ausführungsform liegen 60 bis 90 Gew.-%, ganz besonders bevorzugt 70 bis 80 Gew.-% des Aktivmetalls, bezogen auf die Gesamtmenge des Aktivmetalls, in der Schale des Katalysators bis zu einer Eindringtiefe von 500 µm vor. Die vorstehend genannten Daten werden mittels SEM (scanning electron microscopy) EPMA (electron probe microanalysis) - EDXS (energy dispersive X-ray spectroscopy) ermittelt und stellen gemittelte Werte dar. Weitere Informationen bezüglich den vorstehend genannten Messverfahren und Techniken sind zum Beispiel in Spectroscopy in Cata/ysisvon J.W. Niemantsverdriet, VCH, 1995 oder Handbook of Microscopy von S. Amelinckx et al. offenbart. Zur Ermittlung der Eindringtiefe der Aktivmetallteilchen werden mehrere Katalysatorteilchen (z. B. 3, 4 oder 6) angeschliffen. Mittels Linescans werden dann die Profile der Aktivmetall/Si Konzentrationsverhältnisse erfasst. Auf jeder Messlinie werden mehrere, zum Beispiel 15 bis 20, Messpunkte in gleichen Abständen gemessen, die Messfleckgröße beträgt circa 10 µm * 10 µm. Nach Integration der Aktivmetallmenge über die Tiefe kann die Häufigkeit des Aktivmetalls in einer Zone bestimmt werden.

Ganz besonders bevorzugt beträgt die Menge des Aktivmetalls, bezogen auf das Konzentrationsverhältnis von Aktivmetall zu Si, an der Oberfläche des Schalenkatalysators, 2 bis 25 %, bevorzugt 4 bis 10 %, besonders bevorzugt 4 bis 6 %, ermittelt mittels SEM EPMA - EDXS. Die Oberflächenanalyse erfolgt mittels Bereichsanalysen von Bereichen von 800 µm * 2000 µm und mit einer Informationstiefe von circa 2 µm. Die Elementzusammensetzung wird in Gew.-% (normiert auf 100 Gew.-%) bestimmt. Das mittlere Konzentrationsverhältnis (Aktivmetall/Si) wird über 10 Messbereiche gemittelt.

Unter der Oberfläche des Schalenkatalysators ist im Sinne der vorliegenden Erfindung die äußere Schale des Katalysators bis zu einer Eindringtiefe von circa 2 µm zu verstehen. Diese Eindringtiefe entspricht der Informationstiefe bei der vorstehend erwähnten Oberflächenanalyse.

Ganz besonders bevorzugt ist ein Schalenkatalysator, worin die Menge des Aktivmetalls, bezogen auf das Gewichtsverhältnis von Aktivmetall zu Si (Gew./Gew. in %), an der Oberfläche des Schalenkatalysators 4 bis 6 % beträgt, in einer Eindringtiefe von 50 µm 1,5 bis 3 % und im Bereich von 50 bis 150 µm Eindringtiefe 0,5 bis 2 %, ermittelt mittels SEM EPMA (EDXS), beträgt. Die genannten Werte stellen gemittelte Werte dar.

Des Weiteren nimmt die Größe der Aktivmetallteilchen bevorzugt mit zunehmender Eindringtiefe ab, ermittelt mittels (FEG)-TEM-Analyse.

Das Aktivmetall liegt in dem erfindungsgemäßen Schalenkatalysator bevorzugt teilweise oder vollständig kristallin vor. In bevorzugten Fällen kann in der Schale des erfindungsgemäßen Schalenkatalysators mittels SAD (Selected Area Diffraction) feinstkristallines Aktivmetall nachgewiesen werden.

Der erfindungsgemäß bevorzugte Schalenkatalysator kann zusätzlich Erdalkalimetallionen (M²⁺), also M = Be, Mg, Ca, Sr und/oder Ba, insbesondere Mg und/oder Ca, ganz besonders Mg enthalten. Der Gehalt an Erdalkalimetallion/en (M²⁺) im Katalysator beträgt bevorzugt 0,01 bis 1 Gew.-%, insbesondere 0,05 bis 0,5 Gew.-%, ganz besonders 0,1 bis 0,25 Gew.-%, jeweils bezogen auf das Gewicht des Siliziumdioxid-Trägermaterials.

Ein wesentlicher Bestandteil der erfindungsgemäß bevorzugten Katalysatoren ist das Trägermaterial enthaltend Siliziumdioxid, bevorzugt amorphes Siliziumdioxid. Unter dem Begriff "amorph" versteht man in diesem Zusammenhang, dass der Anteil kristalliner Siliziumdioxid-Phasen weniger als 10 Gew.-% des Trägermaterials ausmacht. Die zur Herstellung der Katalysatoren verwendeten Trägermaterialien können allerdings Überstrukturen aufweisen, die durch regelmäßige Anordnung von Poren im Trägermaterial gebildet werden.

Als Trägermaterialien kommen grundsätzlich amorphe Siliziumdioxid-Typen in Betracht, die mindestens zu 90 Gew.-% aus Siliziumdioxid bestehen, wobei die verbleibenden 10 Gew.-%, vorzugsweise nicht mehr als 5 Gew.-%, des Trägermaterials auch ein anderes oxidisches Material sein können, z. B. MgO, CaO, TiOz, ZrO₂ und/oder Al₂O₃.

In einer bevorzugten Ausführungsform der Erfindung ist das Trägermaterial halogenfrei, insbesondere chlorfrei, d. h. der Gehalt an Halogen im Trägermaterial beträgt im Allgemeinen weniger als 500 Gew.-ppm, z. B. im Bereich von 0 bis 400 Gew.-ppm. Somit ist ein Schalenkatalysator bevorzugt, der weniger als 0,05 Gew.-% Halogenid (ionenchromatographisch bestimmt), bezogen auf das Gesamtgewicht des Katalysators, enthält. Besonders bevorzugt liegt der Halogenidgehalt des Trägermaterials unterhalb der analytischen Nachweisgrenze. Bevorzugt sind Trägermaterialien enthaltend Siliziumdioxid, die eine spezifische Oberfläche im Bereich von 280 bis 500 m²/g, besonders bevorzugt 280 bis 400 m²/g, ganz besonders bevorzugt 300 bis 350 m²/g, (BET-Oberfläche nach DIN 66131) aufweisen. Sie können sowohl natürlichen Ursprungs als auch künstlich hergestellt worden sein. Beispiele für geeignete amorphe Trägermaterialien auf Basis von Siliziumdioxid sind Kieselgele, Kieselgur, pyrogene Kieselsäuren und Fällungskieselsäuren. In einer bevorzugten Ausführungsform der Erfindung weisen die Katalysatoren Kieselgele als Trägermaterialien auf.

Das Porenvolumen des Trägermaterials beträgt erfindungsgemäß 0,6 bis 1,0 ml/g, bevorzugt 0,65 bis 0,9 ml/g, beispielsweise 0,7 bis 0,8 ml/g, bestimmt durch Hg-Porosimetrie (DIN 66133). In dem erfindungsgemäß bevorzugten Schalenkatalysator weisen mindestens 90 % der vorhandenen Poren einen Porendurchmesser von 6 bis 12 nm, bevorzugt 7 bis 11 nm, besonders bevorzugt 8 bis 10 nm, auf. Der Porendurchmesser kann nach dem Fachmann bekannten Verfahren bestimmt werden, beispielsweise durch Hg-Porosimetrie oder N₂-Physisorption. In einer bevorzugten Ausführungsform weisen mindestens 95 %, besonders bevorzugt mindestens 98 %, der vorhandenen Poren einen Porendurchmesser von 6 bis 12 nm, bevorzugt 7 bis 11 nm, besonders bevorzugt 8 bis 10 nm, auf.

In dem erfindungsgemäß bevorzugten Schalenkatalysator liegen in einer bevorzugten Ausführungsform keine Poren vor, die kleiner als 5 nm sind. Des Weiteren liegen in dem erfindungsgemäß bevorzugten Schalenkatalysator keine Poren vor, die größer als 25 nm, insbesondere größer als 15 nm, sind. In diesem Zusammenhang bedeutet "keine Poren", dass mit üblichen Messverfahren, beispielsweise Hg-Porosimetrie oder N₂-Physisorption keine Poren mit diesen Durchmessern gefunden werden. In dem erfindungsgemäß bevorzugten Schalenkatalysator liegen im Rahmen der Messgenauigkeit der verwendeten Analytik keine Makroporen, sondern ausschließlich Mesoporen vor.

Bei dem erfindungsgemäß bevorzugten Schalenkatalysators werden besonders bevorzugt Formkörper aus dem Trägermaterial, die z. B. durch Extrudieren, Strangpressen oder Tablettieren erhältlich sind und die z. B. die Form von Kugeln, Tabletten, Zylindern, Strängen, Ringen bzw. Hohlzylindern, Sternen und dergleichen, besonders bevorzugt Kugeln, aufweisen können, eingesetzt. Die Abmessungen dieser Formkörper bewegen sich üblicherweise im Bereich von 0,5 mm bis 25 mm. Bevorzugt werden Katalysatorkugeln mit Kugeldurchmessern von 1,0 bis 6,0 mm, besonders bevorzugt 2,5 bis 5,5 mm, eingesetzt. In dem erfindungsgemäß bevorzugten Schalenkatalysator beträgt die Dispersität des Aktivmetalls bevorzugt 30 bis 60 %, besonders bevorzugt 30 bis 50 %. Verfahren zur Messung der Dispersität des Aktivmetalls sind dem Fachmann an sich bekannt, beispielsweise durch Puls-Chemisorption, wobei die Bestimmung der Edelmetalldispersion (spezifische Metalloberfläche, Kristallitgrösse) mit CO Pulsmethode durchgeführt wird (DIN 66136(1-3)).

In dem erfindungsgemäß bevorzugten Schalenkatalysator beträgt die Oberfläche des Aktivmetalls bevorzugt 0,2 bis 0,8 m²/g, besonders bevorzugt 0,3 bis 0,7 m²/g. Verfahren zur Messung der Oberfläche des Aktivmetalls sind dem Fachmann an sich bekannt.

Die Herstellung der erfindungsgemäß bevorzugten Schalenkatalysatoren erfolgt beispielsweise dadurch, dass man zunächst das Trägermaterial mit einer Lösung enthaltend eine Vorläuferverbindung des Aktivmetalls ein- oder mehrfach tränkt, den erhaltenen Feststoff trocknet und anschließend reduziert. Die einzelnen Verfahrensschritte sind dem Fachmann an sich bekannt und in der WO 2011/082991 beschrieben.

Der Einsatz der Katalysatoren 4 als Hydrierkatalysatoren ist eine weitere besonders bevorzugte Ausführungsform des erfindungsgemäßen Verfahrens.

### Katalysator 5:

Erfindungsgemäß geeignet sind ebenfalls Katalysatoren 5 mit einem Aktivmetall auf einem mesoporösen Träger gemäß WO 2004/046078. Katalysator 5 enthält ein oder mehrere katalytisch aktiven Metall(e), wie Platin, Palladium, Ruthenium oder Mischungen davon, abgeschieden auf einem Katalysatorträgermaterial, umfassend ein oder mehrere geordnete mesoporöse Materialien. Der Inhalt der WO 2004/046078 wird durch Bezugnahme vollständig in die vorliegende Anmeldung einbezogen.

### Katalysator 6:

Erfindungsgemäß geeignet sind ebenfalls Katalysatoren 6 mit einem Aktivmetall auf einem mesoporösen Träger gemäß US 7,893,295. Der darin genannte Katalysator enthält ein oder mehrere katalytisch aktive Metall(e), wie Platin, Palladium Ruthenium oder Mischungen davon, abgeschieden auf einem Katalysatorträgermaterial, umfassend ein oder mehrere geordnete mesoporöse Materialien. Der Inhalt der US 7,893,295 wird durch Bezugnahme vollständig in die vorliegende Anmeldung einbezogen.

### Katalysator 7:

Erfindungsgemäß geeignet sind ebenfalls mesoporöse Metall/Träger-Katalysatoren 7 gemäß DE 10225565. Dieser Katalysator enthält mindestens ein Metall der Gruppe 8 auf oder in einem Trägermaterial mit einem mittleren Porendurchmesser von 25 bis 50 nm und einer spezifischen Oberfläche von mehr als 30 m²/g. Der Inhalt der DE 10225565 wird durch Bezugnahme vollständig in die vorliegende Anmeldung einbezogen.

### Katalysator 8:

Erfindungsgemäß geeignet sind ebenfalls Katalysatoren 8 auf Basis Nickel/Zink gemäß DE 10146847. Der Inhalt der DE 10146847 wird durch Bezugnahme vollständig in die vorliegende Anmeldung einbezogen.

### Katalysator 9:

Erfindungsgemäß geeignet sind ebenfalls mikroporöse Metall-Katalysatoren 9 auf Titandioxid-Trägern gemäß WO 04/009526. Der Inhalt der WO 04/009526 wird durch Bezugnahme vollständig in die vorliegende Anmeldung einbezogen.

Die Temperatur bei der Hydrierung liegt vorzugsweise in einem Bereich von 50 bis 250 °C, besonders bevorzugt von 70 bis 200 °C, insbesondere von 90 bis 150 °C.

Der absolute Druck bei der Hydrierung liegt vorzugsweise in einem Bereich von 50 bis 330 bar, besonders bevorzugt in einem Bereich von 200 bis 300 bar, insbesondere in einem Bereich von 220 bis 270 bar.

Das erfindungsgemäße Verfahren kann entweder kontinuierlich oder diskontinuierlich durchgeführt werden, wobei die kontinuierliche Verfahrensdurchführung bevorzugt ist.

Vorzugsweise erfolgt die Hydrierung der Benzolpolycarbonsäureester an einem Festbettkatalysator in Rieselfahrweise oder in Sumpffahrweise. Bei der Rieselfahrweise lässt man das flüssige Reaktionsmedium in dem Reaktor über das in diesem angeordnete Katalysatorbett rieseln, wobei sich auf dem Katalysator ein dünner Flüssigkeitsfilm ausbildet. Beim Arbeiten in Sumpffahrweise wird ein wasserstoffhaltiges Gas in den mit dem flüssigen Reaktionsmedium gefluteten Reaktor eingeleitet, wobei der Wasserstoff das Katalysatorbett in aufsteigenden Gasperlen passiert.

Das Wasserstoff enthaltende Gas kann dabei sowohl im Gleichstrom mit der Lösung des zu hydrierenden Edukts als auch im Gegenstrom über den Katalysator geleitet werden. Die Hydrierung kann auch diskontinuierlich nach der Batchfahrweise durchgeführt werden.

Bei der kontinuierlichen Verfahrensführung beträgt die Katalysatorbelastung vorzugsweise 0,05 bis 120 kg (Benzolpolycarbonsäure) / (L(Katalysator) x h), besonders bevorzugt 0,5 bis 12 kg (Benzolpolycarbonsäure) / (L(Katalysator) x h).

Das erfindungsgemäße Verfahren kann in Ab- oder Anwesenheit eines Lösungs- oder Verdünnungsmittels durchgeführt werden, d. h. es ist nicht erforderlich, das Verfahren in Lösung durchzuführen.

Vorzugsweise wird jedoch ein Lösungs- oder Verdünnungsmittel eingesetzt. Als Lösungs- oder Verdünnungsmittel kann jedes geeignete Lösungsmittel- oder Verdünnungsmittel eingesetzt werden. Die Auswahl ist dabei nicht kritisch, solange das eingesetzte Lösungs- oder Verdünnungsmittel in der Lage ist, mit der wenigstens einen Benzolpolycarbonsäureester eine homogene Lösung zu bilden.

Beispielsweise können die Lösungs- oder Verdünnungsmittel auch Wasser enthalten. Beispielsweise ist ein Lösungs- oder Verdünnungsmittel ausgewählt aus der Gruppe, bestehend aus geradkettigen oder cyclischen Ethern, wie beispielsweise Tetrahydrofuran oder Dioxan, aliphatischen Alkoholen, in denen der Alkylrest vorzugsweise 1 bis 10 Kohlenstoffatome, insbesondere 3 bis 6 Kohlenstoffatome aufweist, beispielsweise i-Propanol, n-Butanol, i-Butanol, n-Hexanol und Mischungen davon.

Die Menge des eingesetzten Lösungs- oder Verdünnungsmittels ist nicht in besonderer Weise beschränkt und kann je nach Bedarf frei gewählt werden, wobei jedoch solche Mengen bevorzugt sind, die zu einer 10 bis 70 gew.-%igen Lösung des wenigstens einen zur Hydrierung vorgesehenen Benzolpolycarbonsäureesters führen.

Besonders bevorzugt wird im Rahmen des erfindungsgemäßen Verfahrens das bei der Hydrierung gebildete Produkt, also der entsprechende Cyclohexanpolycarbonsäureester als Lösungsmittel eingesetzt, gegebenenfalls neben anderen Lösungs- oder Verdünnungsmitteln. In jedem Fall kann ein Teil des im Verfahren gebildeten Produkts der noch zu hydrierenden Benzolpolycarbonsäureester beigemischt werden. Bezogen auf das Gewicht der zur Hydrierung vorgesehenen Verbindung wird vorzugsweise die 1-bis 30-fache, besonders bevorzugt die 5- bis 20-fache, insbesondere die 5- bis 10-fache Menge, des Umsetzungsproduktes als Lösungs- oder Verdünnungsmittel zugemischt.

Das erfindungsgemäße Verfahren wird im Allgemeinen bei einer Leerohrgeschwindigkeit von 1 bis 500 m/h durchgeführt, bevorzugt wird das erfindungsgemäße Verfahren bei einer Leerohrgeschwindigkeit von 5 bis 300 m/h durchgeführt, besonders bevorzugt wird das erfindungsgemäße Verfahren bei einer Leerohrgeschwindigkeit von 10 bis 180 m/h durchgeführt.

Die folgenden Figuren und Beispiele dienen der Verdeutlichung der Erfindung, ohne sie in irgendeiner Weise zu beschränken.

### FIGURENBESCHREIBUNG

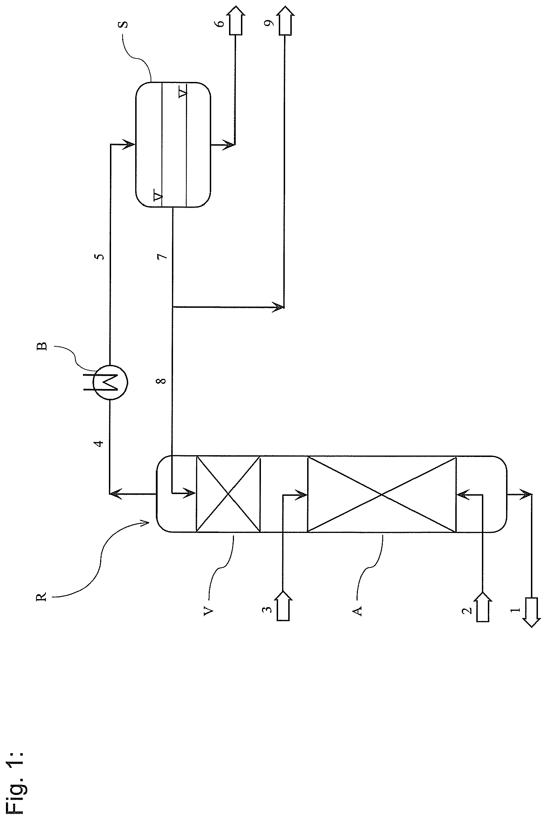

Im Folgenden wird die Erfindung anhand der Figuren 1 bis 3 näher erläutert, ohne die Erfindung jedoch auf die darin dargestellten Ausführungsformen zu beschränken.

In den Figuren werden die folgenden Bezugszeichen verwendet:
- A: Abtriebsteil
- B: Brüdenkondensator
- D: Destillationskolonne
- K: Kondensator
- R: Rektifikationskolonne
- S: Phasentrennbehälter
- V: Verstärkungsteil
- 1: Sumpfprodukt (Ester)
- 2: Wasserdampfhaltiger Gasstrom (Dampf)
- 3: Zulauf (Rohester mit Alkohol, Ether, Wasser)
- 4: Brüden
- 5: Brüdenkondensat
- 6: wässrige Phase
- 7: organische Phase (Ether, Alkohol)
- 8: Rücklauf organische Phase (Ether, Alkohol)
- 8': Rücklauf Alkohol-Kondensat
- 9: Ausschleusstrom organische Phase (Ether, Alkohol)
- 9': Ausschleusstrom Ether
- 10: Teilstrom organische Phase
- 11: alkoholhaltige Gasphase
- 12: Alkohol-Kondensat
- 13: Kondensat-Rücklauf
- 14: Abzug Alkohol-Kondensat

- Figur 1: zeigt eine bevorzugte Ausführungsform des erfindungsgemäßen Verfahrens mit einem Brüden-Phasentrennbehälter.
- Figur 2: zeigt eine weitere bevorzugte Ausführungsform des erfindungsgemäßen Verfahrens mit einer zusätzlichen Destillationskolonne zur Auftrennung des Brüdenkondensats.
- Figur 3: zeigt eine ebenfalls bevorzugte Ausführungsform des erfindungsgemäßen Verfahrens mit einer zusätzlichen Destillationskolonne zur Auftrennung des Brüdenkondensats in einer alternativen Verschaltung zu der in Figur 2 dargestellten Ausführungsform.
- Figur 4: zeigt den Gehalt an Diisononylether im Sumpfprodukt in Abhängigkeit von der Eintrittstemperatur des Rohesters.
- Figur 5: zeigt eine Ausgestaltung gemäß Variante 1 des erfindungsgemäßen Verfahrens. Kurve 2 zeigt, dass eine konstante Menge der aus dem kondensierten Brüden durch Phasentrennung erhaltenen organischen Phase (200 kg/Std) als Rückführstrom auf den Kopf der Strippkolonne gefahren wird. Kurve 3 zeigt, in welchen Intervallen die übrige Menge der organischen Phase wird in die Estersynthese zurückgeführt oder ausgeschleust wird. Kurve 1 zeigt den Gehalt an Diisononylether im Sumpfprodukt.

In Figur 1 ist eine Ausführungsform des erfindungsgemäßen Verfahrens dargestellt. In einer Rektifikationskolonne R wird zwischen Abtriebsteil A und Verstärkungsteil V ein Rohester als Zulauf 3 zugeführt. Der Rohester besteht im Wesentlichen aus einem oder mehreren Benzolpolycarbonsäureester(n) , C₄-C₁₂-Monoalkanol(en), Di-(C₄-C₁₂-alkyl)ether(n) und Wasser. Im Abtriebsteil A wird ein wasserdampfhaltiger Gasstrom 2 (im Folgenden auch als Dampf bezeichnet) im Gegenstrom von oberhalb des Kolonnensumpfes zugeführt. Im Kolonnensumpf wird Sumpfprodukt 1 abgezogen. Das Sumpfprodukt 1 besteht im Wesentlichen aus Benzolpolycarbonsäureester mit einem gegenüber dem Feed abgereicherten Anteil an Di-(C₄-C₁₂-alkyl)ether, C₄-C₁₂-Monoalkanol und Wasser. Der Dampf 2 durchströmt den Abtriebsteil A und anschließend den Verstärkungsteil V, wobei Alkohol, Ether und Wasser aus dem Zulauf 3 in die Dampfphase 2 übergehen. Am Kopf der Kolonne wird Brüden 4 abgezogen. Der Brüden 4 ist ein Gemisch, das Wasserdampf, Di-(C₄-C₁₂-alkyl)ether und C₄-C₁₂-Monoalkanol enthält, wobei auch geringe Mengen an Benzolpolycarbonsäureester und weitere Komponenten enthalten sein können. Der Brüden 4 wird in einem Brüdenkondensator B mindestens teilweise kondensiert und als Brüdenkondensat 5 einem Phasentrennbehälter S zugeführt. Im Phasentrennbehälter S wird im unteren Bereich eine wässrige Phase 6 abgezogen, die im Wesentlichen Wasser enthält. Darüber wird eine organische Phase 7 abgezogen, die im Wesentlichen Di-(C₄-C₁₂-alkyl)ether und C₄-C₁₂-Monoalkanol enthält. Die aus dem Phasentrennbehälter S abgezogene organische Phase 7 wird in einen Ausschleusstrom 9 und einen Kondensat-Rücklauf 8 aufgeteilt. Der Kondensat-Rücklauf 8 wird auf den Kopf der Rektifikationskolonne R oberhalb des Verstärkungsteil V zugeführt, so dass sie zu dem Dampf 2 im Gegenstrom geführt wird.

Die aus dem Phasentrennbehälter S abgezogene wässrige Phase 6 kann in geeigneter Weise entsorgt oder gegebenenfalls in den Aufarbeitungsprozess zurückgeführt werden. Der Ausschleusstrom 9 kann in geeigneter Weise entsorgt oder einer geeigneten Verwendung zugeführt werden.

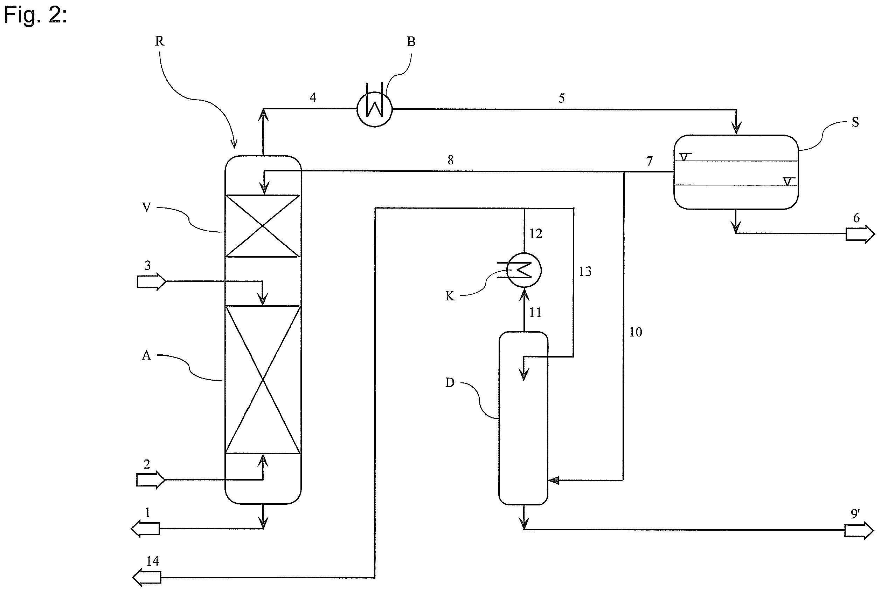

In Figur 2 ist eine weitere Ausführungsform des erfindungsgemäßen Verfahrens dargestellt. In der Rektifikationskolonne R wird zwischen Abtriebsteil A und Verstärkungsteil V der Rohester als Zulauf 3 zugeführt. Im Abtriebsteil A wird Dampf 2 im Gegenstrom von oberhalb des Kolonnensumpfes zugeführt. Im Kolonnensumpf wird das Sumpfprodukt 1 abgezogen. Der Dampf 2 durchströmt den Abtriebsteil A und anschließend den Verstärkungsteil V, wobei Alkohol, Ether und Wasser aus dem Zulauf 3 in die Dampfphase übergehen. Am Kopf der Kolonne wird der Brüden 4 abgezogen. Der Brüden 4 wird in einem Brüdenkondensator B mindestens teilweise kondensiert und als Brüdenkondensat 5 einem Phasentrennbehälter S zugeführt. Im Phasentrennbehälter S wird im unteren Bereich die wässrige Phase 6 abgezogen. Die aus dem Phasentrennbehälter S abgezogene wässrige Phase 6 kann in geeigneter Weise entsorgt oder gegebenenfalls in den Aufarbeitungsprozess zurückgeführt werden.

Im Phasentrennbehälter S wird über der wässrigen Phase 6 die organische Phase 7 abgezogen. Die organische Phase 7 wird in einen Rücklauf 8 und einen Teilstrom 10 aufgeteilt. Der Rücklauf 8 wird auf den Kopf der Rektifikationskolonne R oberhalb des Verstärkungsteil V zugeführt, so dass er im Gegenstrom zu dem Dampf 2 geführt wird.

Der Teilstrom 10 wird einer Destillationskolonne D oberhalb des Sumpfes zugeführt. Im Sumpf der Destillationskolonne D wird ein Ausschleusstrom 9' abgezogen, der im Wesentlichen Di-(C₄-C₁₂-alkyl)ether, C₄-C₁₂-Monoalkanol und/oder Benzolpolycarbonsäureester enthält. Der Ausschleusstrom 9' kann in geeigneter Weise entsorgt oder einer geeigneten Verwendung zugeführt werden.

Am Kopf der Destillationskolonne D wird eine alkoholhaltige Gasphase abgezogen und in einem Kondensator K mindestens teilweise kondensiert. Die alkoholhaltige Gasphase kann neben C₄-C₁₂-Monoalkanol geringe Mengen an Wasser und Di-(C₄-C₁₂-alkyl)-ether enthalten. Ein Teil des erhaltenen Alkohol-Kondensats 12 wird als Kondensat-Rücklauf 13 auf den Kopf der Destillationskolonne D zurückgeführt. Das restliche Alkohol-Kondensat wird als Kondensat-Abzug 14 ausgeschleust und kann teilweise oder vollständig in die Veresterung der Benzolpolycarbonsäure mit dem wenigstens einen C₄-C₁₂-Monoalkanol zurückgeführt werden.

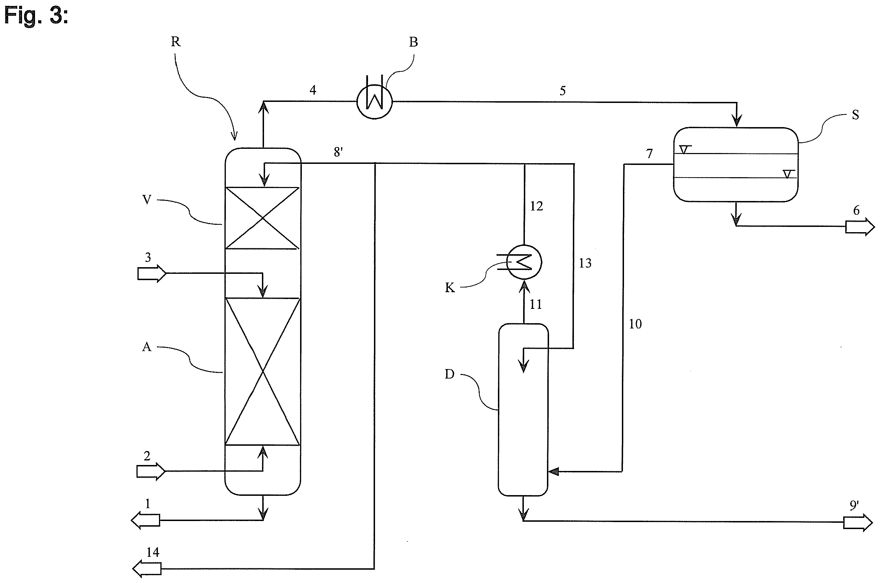

Figur 3 zeigt eine weitere Ausführungsform des erfindungsgemäßen Verfahrens. Dabei handelt es um eine alternative Verschaltung zu der in Figur 2 dargestellten Variante mit destillativer Auftrennung der organischen Phase. In der Rektifikationskolonne R wird zwischen Abtriebsteil A und Verstärkungsteil V der Rohester als Zulauf 3 zugeführt. Im Abtriebsteil A wird Dampf 2 im Gegenstrom von oberhalb des Kolonnensumpfes zugeführt. Im Kolonnensumpf wird das Sumpfprodukt 1 abgezogen. Der Dampf 2 durchströmt den Abtriebsteil A und anschließend den Verstärkungsteil V, wobei Alkohol, Ether und Wasser aus dem Zulauf 3 in die Dampfphase übergehen. Am Kopf der Kolonne wird der Brüden 4 abgezogen. Der Brüden 4 wird in einem Brüdenkondensator B mindestens teilweise kondensiert und als Brüdenkondensat 5 einem Phasentrennbehälter S zugeführt. Im Phasentrennbehälter S wird im unteren Bereich die wässrige Phase 6 abgezogen. Die aus dem Phasentrennbehälter S abgezogene wässrige Phase 6 kann in geeigneter Weise entsorgt oder gegebenenfalls in den Aufarbeitungsprozess zurückgeführt werden.

Im Phasentrennbehälter S wird über der wässrigen Phase 6 die organische Phase 7 abgezogen. Die organische Phase 7 wird einer Destillationskolonne D oberhalb des Sumpfes zugeführt. Im Sumpf der Destillationskolonne D wird ein Ausschleusstrom 9' abgezogen, der im Wesentlichen Di-(C₄-C₁₂-alkyl)ether und gegebenenfalls Beimengungen von Wasser, C₄-C₁₂-Monoalkanol und/oder Benzolpolycarbonsäureester enthält. Der Ausschleusstrom 9' kann in geeigneter Weise entsorgt oder einer geeigneten Verwendung zugeführt werden.

Am Kopf der Destillationskolonne D wird eine alkoholhaltige Gasphase 11 abgezogen und in einem Kondensator K mindestens teilweise kondensiert. Die alkoholhaltige Gasphase kann neben C₄-C₁₂-Monoalkanol geringe Mengen an Wasser und Di-(C₄-C₁₂-alkyl)ether enthalten. Ein Teil des erhaltenen Alkohol-Kondensats 12 wird als Kondensat-Rücklauf 13 auf den Kopf der Destillationskolonne D zurückgeführt. Ein weiterer Teil des verbliebenen Alkohol-Kondensats 12 wird als Rücklauf 8' auf den Kopf der Rektifikationskolonne R oberhalb des Verstärkungsteil V zugeführt, so dass er im Gegenstrom zu dem Dampf 2 geführt wird. Der restliche Teil des Alkohol-Kondensats 12 wird als Kondensat-Abzug 14 ausgeschleust und kann teilweise oder vollständig in die Veresterung der Benzolpolycarbonsäure mit dem wenigstens einen C₄-C₁₂-Monoalkanol zurückgeführt werden.

### BEISPIELE

### Beispiel 1

### Einfluss der Eintrittstemperatur des Rohesters in die Strippkolonne

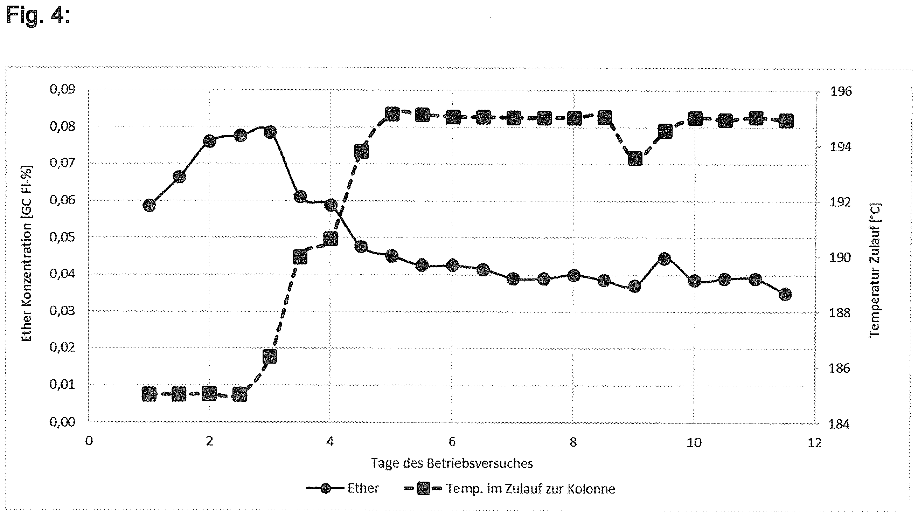

In eine Rektifikationskolonne (Strippkolonne) gemäß Figur 1 wird zwischen Abtriebsteil und Verstärkungsteil ein rohes Diisononylphthalat als Zulauf mit einer Zulaufrate von 13000 kg/h zugeführt. Der Rohester enthält weiterhin Diisononanol, Diisononylether und Wasser. Die Strippkolonne wird bei einem Druck von 50 mbar absolut betrieben. Oberhalb des Kolonnensumpfes wird im Gegenstrom zum Rohester Dampf mit 4 bar eingespeist und der Rohester mit 3 Gew.-% Dampf, bezogen auf Rohester, gestrippt. Dabei wird die Eintrittstemperatur des Rohesters in die Kolonne zunächst konstant bei 185 °C gelassen und dann in mehreren Schritten auf 195 °C erhöht. Am Kopf der Kolonne wird der Brüden abgezogen, kondensiert und ausgeschleust. Eine Rückführung von kondensiertem Brüden auf den Kopf der Kolonne erfolgt nicht. Es wird der Gehalt des Sumpfprodukts an Diisononylether bestimmt. Figur 4 zeigt den Gehalt an Diisononylether im Sumpfprodukt in Abhängigkeit von der Eintrittstemperatur des Rohesters. Es zeigt sich, dass der Gehalt des Sumpfprodukts an Diisononylether durch Erhöhung der Eintrittstemperatur des Rohesters in die Kolonne von ca. 600 - 800 Gew.-ppm deutlich auf ca. 400 Gew.-ppm verringert werden kann.

### Beispiel 2:

### Hydrierung von Diisononylphthalat (DINP) zu Cyclohexan-1,2-dicarbonsäure-diisononylester (DINCH)

Als Rohstoff für die erfindungsgemäße Hydrierung 1 wurde Diisononylphthalat aus einer Charge des Beispiels 1 eingesetzt, bei dem der Gehalt an Diisononylether infolge der erhöhten Stripptemperatur bei 500 Gew.-ppm lag. Bei der Hydrierung V1 handelt es sich um einen Vergleichsversuch, wobei als Rohstoff Diisononylphthalat aus einer Charge des Beispiels 1 eingesetzt wurde, wobei die Ether Konzentration aufgrund der tieferen Stripptemperatur bei 590 Gew.-ppm lag. Als Hydrierkatalysator wurde bei allen Versuchen ein Ruthenium-Katalysator auf einem makro/mesoporösen AluminiumoxidTräger eingesetzt, der nach dem Katalysator-Herstellbeispiel von Seite 7, Zeilen 36 - 47 der DE 19624485 A1 hergestellt wurde. Der Rutheniumgehalt des Katalysators lag bei 0,5 %.

Die Hydrierungen wurden in einer Kaskade von 3 Reaktoren (Innendurchmesser 1 m, Länge 20 m) durchgeführt. Dabei wurde der erste Reaktor als Hauptreaktor mit Umlauf betrieben, d. h. der Austrag des ersten Reaktors wurde teilweise zum Eingang des ersten Reaktors zurückgeführt. Die letzten 2 Reaktoren wurden als Nachreaktoren im geraden Durchgang betrieben. Die Reaktoren wurde mit jeweils 9000 kg Katalysator befüllt.

Die Hydrierung wurde mit reinem Wasserstoff durchgeführt. Der Zulauf wurde so gewählt, dass die Katalysatorbelastung im Hauptreaktor (kg(Diisonylphtalat)/(L(Katalysator)·h) den in der nachfolgenden Tabelle angegeben Wert erreicht. Die Rückführrate wurde so gewählt, dass die Leerrohr-Geschwindigkeit im Hauptreaktor bei den in der Tabelle 1 angegebenen Werten liegt. Der Wasserstoff wurde druckgeregelt bei dem in der Tabelle 1 angegebenen Druck zugeführt. Die Reaktionstemperaturen sind ebenfalls in der Tabelle 1 angegeben.

**Tabelle 1: Hydrierversuche**

| Nr | Hauptreaktor | | | | Nachreaktor 1 | | | Nachreaktor 2 | | | Druck Reaktoren [bar] |
|---|---|---|---|---|---|---|---|---|---|---|---|
| | A | B | C | D | A | B | C | A | B | C | |
| V1 | 9,8 | 160 | 123 | 10 | 3,0 | 52 | 138 | 1,3 | 23 | 117 | 250 |
| 1 | 9,8 | 160 | 123 | 10 | 3,0 | 52 | 138 | 1,3 | 23 | 117 | 250 |

| | | | | | | | | | | | |
|---|---|---|---|---|---|---|---|---|---|---|---|
| A) Katalysatorbelastung: kg (Diisononylphthalat)/(L(Katalysator) h) B) Leerrohr-Geschwindigkeit: m³(Diisononylphthalat)/(m²(Reaktor-Querschnitt) (h)) C) Temperatur [°C] D) Rückführrate: kg zurückgeführtes Hydrierprodukt / kg Diisononylphthalat | | | | | | | | | | | |

Die Analyse des eingesetzten Diisononylphthalats und des erhaltenen Cyclohexan-1,2-dicarbonsäurediisononylester erfolgte nach folgender GC-Methode:

| | |
|---|---|
| Säule: | DB-1 30m (100 % Dimethylpolysiloxan), ID 0,32 mm, FD 0,25 µm |
| Detektor: | Flammionisationsdetektor (FID) |
| Temperaturprogramm: | Anfangstemperatur 80 °C, 1 min Haltezeit, Aufheizen mit 5 °C/min auf 300 °C, Haltezeit 15 min |
| Injektionsvolumen: | 0,2 µL |
| Einlass-Temperatur: | 300 °C |
| Detektortemperatur: | 320 °C |

### Retentionszeiten:

Isononanol: 4 - 9,5 min
Cyclohexan-1,2-dicarbonsäurediisononylester (DINCH): 32,5 - 43 min

**Tabellen 2a und 2b: Ergebnisse der Hydrierversuche**

| | Diisononylphthalat (DINP) | Cyclohexan-1,2-dicarbonsäurediisononylester (DINCH) | | |
|---|---|---|---|---|
| Nr. | Diisononylether [Gew.-%]^{a)} | DINCH [Gew.-%] | Diisononylether [Gew.-%] | Summe Sonstige [Gew.-%] |
| V1 | 0,059 | 99,84 | 0,113 | 0,047 |
| 1 | 0,050 | 99,82 | 0,082 | 0,098 |

| Nr. | Cyclohexan-1,2-dicarbonsäurediisononylester (DINCH) | | | |
|---|---|---|---|---|
| | Wasser [Gew.-%] | Säurezahl [mg KOH/g] | Pt/Co-Farbzahl | Restaromat (DINP) [ppm] |
| V1 | 0,019 | 0,03 | 3 | 4 |
| 1 | 0,013 | 0,01 | 4 | 2 |

| | | | | |
|---|---|---|---|---|
| ^{a)} bestimmt als GC-Flächen-% | | | | |

Die Versuche zeigen, dass nach dem erfindungsgemäßen Verfahren aus einem Diisononylphthalat mit einem niedrigen Gehalt an Diisononylether durch Hydrierung unter Einsatz eines Ruthenium-Katalysators auf einem makro/mesoporösen AluminiumoxidTräger ein Cyclohexan-1,2-dicarbonsäurediisononylester (DINCH) mit einem Gehalt an Diisononylethern von höchstens 0,1 Gew.-% erhalten wird. Die Versuche zeigen ebenfalls, dass aus einem Diisononylphthalat mit einem erhöhten Gehalt an Diisononylethern mittels des erfindungsgemäßen Hydrierverfahrens ein Cyclohexan-1,2-dicarbon-säurediisononylester (DINCH) mit einem unerwünscht erhöhten Gehalt an Diisononylethern von mehr als 0,1 Gew.-% erhalten wird.

### Beispiel 3.

In die Strippkolonne gemäß Figur 1 wird kontinuierlich ein rohes Diisononylphthalat als Zulauf mit einer Zulaufrate von 20000 kg/h zugeführt. Die Eintrittstemperatur des Rohesters in die Kolonne beträgt 185 °C. Die Strippkolone wird unter 50 mbar absolut betrieben. Oberhalb des Kolonnensumpfes wird im Gegenstrom zum Rohester Dampf mit 4 bar eingespeist und der Rohester mit 3 Gew.-% Dampf, bezogen auf Rohester, gestrippt. Die kondensierten Brüden werden in einem Phasentrennbehälter getrennt in eine wässrige und eine organische Phase. Die wässrige Phase wird jeweils entsorgt. Bis zum Tag 8 wird auch die organische Phase aus dem Phasentrennbehälter vollständig als Ausschleusstrom abgezogen. Durch die kontinuierliche Ausschleusung des kondensierten Brüdens nimmt die Konzentration an Diisononylether im Sumpfprodukt innerhalb von 8 Tagen von 310 Gew.-ppm auf 250 Gew.-ppm ab. Dementsprechend nimmt die Konzentration an Diisononylether in der organischen Phase auch ab. Die Konzentration an Wertprodukt (DINP) in der organischen Phase und die Gesamtbrüdenmenge (Destillat) bleiben dagegen unverändert.

Durch das Zuschalten eines Rücklaufs von 200 kg/Std organische Phase an den Tagen 28 und 46 und Rückführung der restlichen organischen Phase in die Estersynthese geht die Konzentration von DINP in der organischen Phase deutlich zurück. Auch die Gesamtbrüdenmenge (Destillat) geht zurück, gleichzeitig kommt es zu einer leichten Erhöhung der Menge an Diisononylether in der organischen Phase sowie im gestrippten Sumpfprodukt. Dieses Verhalten ist reproduzierbar.

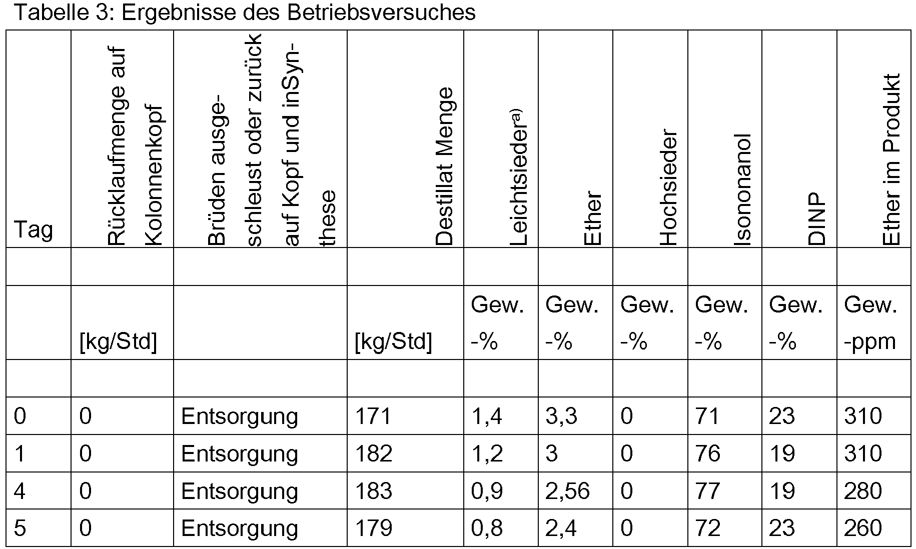

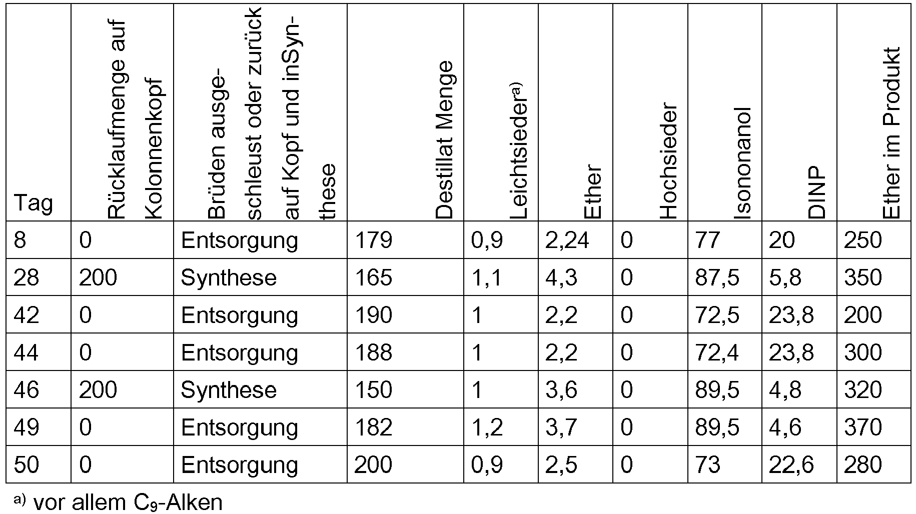

### Beispiel 4:

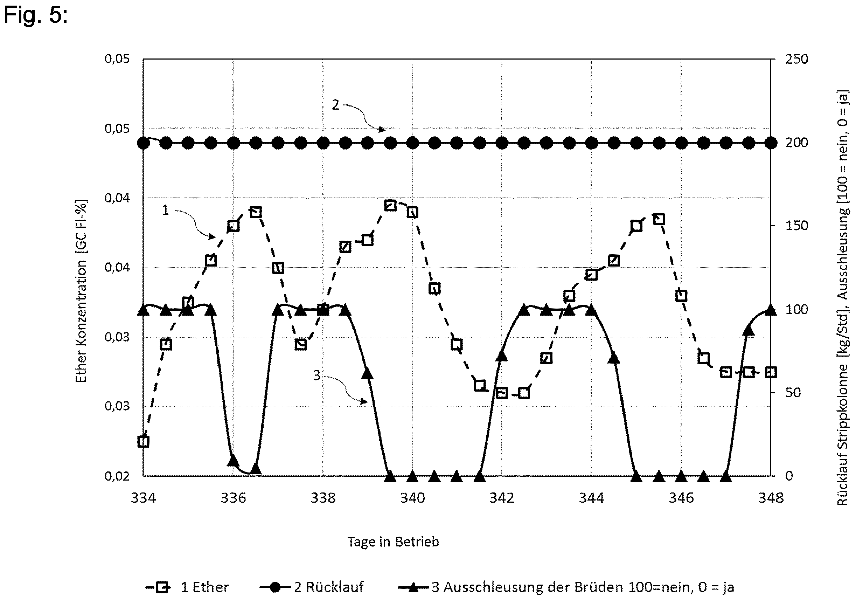

Wie in Beispiel 3 beschrieben wird in einer Strippkolonne gemäß Figur 1 ein rohes Diisononylphthalat behandelt. Ein Teil der aus dem kondensierten Brüden durch Phasentrennung erhaltenen organischen Phase wird als Rückführstrom auf den Kopf der Strippkolonne gefahren (200 kg/Std). Die übrige Menge der organischen Phase wird wechselweise in die Estersynthese zurückgeführt oder ausgeschleust. Durch dieses Vorgehen lässt sich die Konzentration von Diisononylether im Sumpfprodukt regeln und auf dem angestrebten Gehalt von 300 bis 400 Gew.-ppm halten. Somit gelingt es, geringe Ethergehalte im Produkt zu erzielen und gleichzeitig den Anteil an Wertprodukt (DINP) im ausgeschleusten Brüdenstrom deutlich zu verringern (siehe Figur 5).

### Beispiel 5

### Trennung von Ether und Alkohol im Labormaßstab

Es wurde ein mit Öl beheizter 2L Doppelmantelkessel mit Rührer und mit aufgesetzter Destillationskolonne (Durchmesser 30 mm, Länge 3,05 m gefüllt mit strukturierter Packung 1,75 m Sulzer DX und 1,3 m Montz A3-1000) eingesetzt. Die Kolonne verfügt über einen Kopfkondensator, über den ein definierter Rücklauf eingestellt werden kann und ermöglicht die Destillation unter Vakuum.

1,3 kg einer Mischung, die 5,36 Gew.-% Wasser, 1,15 Gew.-% Leichtsieder (Nonene, Phthalsäureanhydrid, Phthalid, Benzoesäure, Benzoesäurenonylester), 84,5 Gew.-% Isononanol, 3,5 Gew.-% Diisononylether, 1,04 Gew.-% weitere Mittelsieder und 4,4 Gew.-% DINP enthielt, wurden im Behältersumpf vorgelegt.

Die Destillation wurde unter 100 mbar Vakuum durchgeführt, die Wärmezufuhr in den Kessel und somit die Destillationstemperatur wurde über den Druckverlust der Kolonne geregelt. Das Rücklaufverhältnis wurde auf den Wert 2 und der Druckverlust über die Kolonne auf 2,5 mbar eingestellt.

Die Komponenten des Gemisches wurden entsprechend ihrer Siedetemperatur sukzessive abdestilliert. Über die Dauer des Experiments wurden Proben des Distillats von jeweils 20 bis 30 g entnommen. Insgesamt wurden 23 Proben genommen und mittels Gaschromatographie analysiert. Die Zusammensetzung der Proben ist in der Tabelle 4 wiedergegeben. Beim Erreichen einer Sumpftemperatur von 180 °C wurde die Destillation beendet. Der Rückstand im Kessel wurde mittels Gaschromatographie analysiert. Durch die Destillation ist es gelungen, das DINP/Ether-Gemisch von ca. 7,9 % auf ca. 84 % aufzukonzentrieren. Als Destillate konnten Isononanol/Wasser-Gemische mit einer Alkoholkonzentration von über 95 % isoliert werden. Ein solcher Alkohol kann zur Synthese von DINP verwendet werden.

**Tabelle 4:**

| Zusammensetzung der Destillatproben und des am Ende der Destillation zurückgebliebenen Sumpfs | | | | | | | |
|---|---|---|---|---|---|---|---|
| Destillatprobe | Wasser [Gew.-%] | Niedrigsieder [Gew.-%] | Isononanol [Gew.-%] | Mittelsieder 1^{a)} [Gew.-%] | Diisononylether [Gew.-%] | Mittelsieder 2^{b)} [Gew.-%] | DINP [Gew.-%] |
| 1 | 88,48 | 8,59 | 2,35 | 0,00 | 0,00 | 0,00 | 0,00 |
| 2 | 96,62 | 1,79 | 0,59 | 0,00 | 0,00 | 0,00 | 1,00 |
| 3 | 99,90 | 0,07 | 0,02 | 0,00 | 0,00 | 0,00 | 0,01 |
| 4 | 46,39 | 22,36 | 31,87 | 0,04 | 0,00 | 0,00 | 0,30 |
| 5 | 1,14 | 15,01 | 83,77 | 0,07 | 0,00 | 0,00 | 0,01 |
| 6 | 1,83 | 3,72 | 94,11 | 0,17 | 0,00 | 0,00 | 0,16 |
| 7 | 1,19 | 1,39 | 97,17 | 0,16 | 0,00 | 0,00 | 0,10 |
| 8 | 1,42 | 0,46 | 97,77 | 0,15 | 0,00 | 0,00 | 0,21 |
| 9 | 0,89 | 0,05 | 98,38 | 0,21 | 0,00 | 0,01 | 0,46 |
| 10 | 0,78 | 0,04 | 98,53 | 0,65 | 0,00 | 0,00 | 0,00 |
| 11 | 1,72 | 0,03 | 96,31 | 1,94 | 0,00 | 0,00 | 0,00 |
| 12 | 1,00 | 0,02 | 96,62 | 2,28 | 0,00 | 0,00 | 0,00 |
| 13 | 0,40 | 0,01 | 96,91 | 2,69 | 0,00 | 0,00 | 0,00 |
| 14 | 0,14 | 0,01 | 96,84 | 3,01 | 0,00 | 0,00 | 0,00 |
| 15 | 0,06 | 0,00 | 99,26 | 0,68 | 0,00 | 0,00 | 0,00 |
| 16 | 0,03 | 0,00 | 99,46 | 0,50 | 0,00 | 0,00 | 0,00 |
| 17 | 0,03 | 0,00 | 99,59 | 0,39 | 0,00 | 0,00 | 0,00 |
| 18 | 0,03 | 0,00 | 99,64 | 0,33 | 0,00 | 0,00 | 0,00 |
| 19 | 0,04 | 0,01 | 99,40 | 0,55 | 0,00 | 0,00 | 0,01 |
| 20 | 0,04 | 0,00 | 99,69 | 0,19 | 0,00 | 0,00 | 0,06 |
| 21 | 0,02 | 0,00 | 99,86 | 0,11 | 0,00 | 0,00 | 0,00 |
| 22 | 0,02 | 0,00 | 99,89 | 0,08 | 0,00 | 0,00 | 0,02 |
| 23 | 0,04 | 0,01 | 99,86 | 0,09 | 0,00 | 0,00 | 0,01 |
| Sumpf | 0,01 | 0 | 6,25 | 8,44 | 34,92 | 0,61 | 49,75 |

| | | | | | | | |
|---|---|---|---|---|---|---|---|
| ^{a)} niedriger siedend als Diisononylether ^{b)} höher siedend als Diisononylether | | | | | | | |

## Patentansprüche

1. Verfahren zur Aufarbeitung eines Rohesters aus der Veresterung einer Benzolpolycarbonsäure mit wenigstens einem C₄-C₁₂-Monoalkanol, wobei der Rohester zusätzlich
- wenigstens einen Di-(C₄-C₁₂-alkyl)ether aus der Veretherung des wenigstens einen C₄-C₁₂-Monoalkanols,
- gegebenenfalls das wenigstens eine C₄-C₁₂-Monoalkanol, und
- gegebenenfalls Wasser
enthält, bei dem man den Rohester einer thermischen Aufreinigung in wenigstens einem Stoffaustauschapparat durch Einleiten eines wasserdampfhaltigen Gasstroms im Bereich des Sumpfs des Stoffaustauschapparats unterzieht, unter Erhalt eines an dem wenigstens einen Benzolpolycarbonsäureester angereicherten und dem wenigstens einen Di-(C₄-C₁₂-alkyl)ether abgereicherten Sumpfprodukts und eines an dem wenigstens einen Di-(C₄-C₁₂-alkyl)ether angereicherten Brüdens,
wobei man den Brüden zumindest teilweise kondensiert, das Kondensat trennt in eine wässrige Phase und eine organische Phase, enthaltend Di-(C₄-C₁₂-alkyl)ether und C₄-C₁₂-Monoalkanol, einen Teil der organischen Phase als Rücklauf in die thermische Aufreinigung des Rohesters zurückführt und einen anderen Teil der organischen Phase ausschleust.

2. Verfahren nach Anspruch 1, wobei man einen dritten Teil der organischen Phase in die Veresterung der Benzolpolycarbonsäure mit dem wenigstens einen C₄-C₁₂-Monoalkanol zurückführt.

3. Verfahren nach einem der Ansprüche 1 oder 2, wobei die Ausschleusung eines Teils der organischen Phase diskontinuierlich oder kontinuierlich erfolgt.

4. Verfahren nach einem der vorhergehenden Ansprüche, wobei man den Gehalt des Sumpfprodukts an Di-(C₄-C₁₂-alkyl)ether regelt, indem man Stelleingriffe an wenigstens einer der folgenden Stellgrößen vornimmt:
- Dem Massenstrom des Rücklaufs der organischen Phase,
- dem Massenstrom der in die Veresterung zurückgeführten organischen Phase,
- dem Massenstrom der ausgeschleusten organischen Phase.

5. Verfahren nach Anspruch 4, bei dem man
- einen Zielwert für den Gehalt des Sumpfprodukts an Di-(C₄-C₁₂-alkyl)ether und einen oberen und unteren Grenzwerts für die Abweichung des Istwerts vom Zielwert festlegt,
- den Istwert des Gehalts des Sumpfprodukts an Di-(C₄-C₁₂-alkyl)ether bestimmt,
- nach Erreichen des oberen Grenzwerts für die Abweichung des Istwerts vom Zielwert Stelleingriffe vornimmt, bis der Gehalt des Sumpfprodukts an Di-(C₄-C₁₂-alkyl)ether auf den unteren Grenzwert für die Abweichung des Istwerts vom Zielwert abgesunken ist.

6. Verfahren nach Anspruch 5, wobei der Zielwert für den Gehalt des Sumpfprodukts an Di-(C₄-C₁₂-alkyl)ether bei höchstens 1000 Gew.-ppm, bevorzugt höchstens 800 Gew.-ppm, insbesondere höchstens 600 Gew.-ppm, speziell höchstens 500 Gew.-ppm, liegt.

7. Verfahren zur Aufarbeitung eines Rohesters aus der Veresterung einer Benzolpolycarbonsäure mit wenigstens einem C₄-C₁₂-Monoalkanol, wobei der Rohester zusätzlich
- wenigstens einen Di-(C₄-C₁₂-alkyl)ether aus der Veretherung des wenigstens einen C₄-C₁₂-Monoalkanols,
- gegebenenfalls das wenigstens eine C₄-C₁₂-Monoalkanol, und
- gegebenenfalls Wasser
enthält, bei dem man den Rohester einer thermischen Aufreinigung in wenigstens einem Stoffaustauschapparat durch Einleiten eines wasserdampfhaltigen Gasstroms im Bereich des Sumpfs des Stoffaustauschapparats unterzieht, unter Erhalt eines an dem wenigstens einen Benzolpolycarbonsäureester angereicherten und dem wenigstens einen Di-(C₄-C₁₂-alkyl)ether abgereicherten Sumpfprodukts und eines an dem wenigstens einen Di-(C₄-C₁₂-alkyl)ether angereicherten Brüdens,
wobei man den Brüden zumindest teilweise kondensiert, das Kondensat trennt in eine wässrige Phase und eine organische Phase, enthaltend Di-(C₄-C₁₂-alkyl)ether und C₄-C₁₂-Monoalkanol, zumindest einen Teil der organischen Phase einer Auftrennung unterzieht in eine an Di-(C₄-C₁₂-alkyl)ether angereicherte Fraktion und eine an C₄-C₁₂-Monoalkanol angereicherte Fraktion und die an Di-(C₄-C₁₂-alkyl)ether angereicherte Fraktion teilweise oder vollständig ausschleust.

8. Verfahren nach Anspruch 7, wobei man einen Teil der organischen Phase einer Auftrennung unterzieht in eine an Di-(C₄-C₁₂-alkyl)ether angereicherte Fraktion und eine an C₄-C₁₂-Monoalkanol angereicherte Fraktion und einen anderen Teil der organischen Phase als Rücklauf in die thermische Aufreinigung des Rohesters zurückführt.

9. Verfahren nach Anspruch 7 oder 8, wobei man die an C₄-C₁₂-Monoalkanol angereicherte Fraktion teilweise oder vollständig als Rücklauf in die thermische Aufreinigung des Rohesters zurückführt.

10. Verfahren nach einem der Ansprüche 7 bis 9, wobei man die an C₄-C₁₂-Monoalkanol angereicherte Fraktion teilweise oder vollständig in die Veresterung der Benzolpolycarbonsäure mit dem wenigstens einen C₄-C₁₂-Monoalkanol zurückführt.

11. Verfahren nach einem der vorhergehenden Ansprüche, wobei der zur Aufarbeitung eingesetzte Rohester
- 91 bis 99,8 Gew.-% wenigstens eines Esters einer Benzolpolycarbonsäure mit wenigstens einem C₄-C₁₂-Monoalkanol,
- 0,05 bis 1 Gew.-% wenigstens eines Di-(C₄-C₁₂-alkyl)ethers,
- 0,1 bis 5 Gew.-% wenigstens eines C₄-C₁₂-Monoalkanols, und
- 0,05 bis 3 Gew.-% Wasser,
enthält.

12. Verfahren nach einem der vorhergehenden Ansprüche, wobei man zur thermischen Aufreinigung wenigstens eine Kolonne einsetzt, die
- einen seitlichen Zulauf für den Rohester,
- einen oberhalb der Zulaufstelle für den Rohester gelegenen Verstärkungsteil,
- einen oberhalb des Verstärkungsteils gelegenen Rücklauf für wenigstens einen Teil des kondensierten Brüdens,
- eine Zuleitung für den wasserdampfhaltigen Gasstrom im Bereich des Sumpfs der Kolonne,
aufweist.

13. Verfahren nach Anspruch 12, wobei der oberhalb der Zulaufstelle für den Rohester gelegene Verstärkungsteil 0 bis 10 theoretische Trennstufen, bevorzugt 0 bis 5 theoretische Trennstufen, insbesondere 0 bis 2 theoretische Trennstufen, aufweist.

14. Verfahren zur Herstellung eines Cyclohexanpolycarbonsäureesters, bei dem man
i) einen Rohesters aus der Veresterung einer Benzolpolycarbonsäure mit wenigstens einem C₄-C₁₂-Monoalkanol bereitstellt,
ii) den in Schritt i) bereitgestellten Rohester einer Aufarbeitung unterzieht, wie in einem der Ansprüche 1 bis 13 definiert, wobei ein gegenüber dem Rohester an Di-(C₄-C₁₂-alkyl)ethern abgereicherter Benzolpolycarbonsäureester erhalten wird,
iii) den in Schritt ii) erhaltenen, an Di-(C₄-C₁₂-alkyl)ethern abgereicherten, Benzolpolycarbonsäureester einer Hydrierung mit einem wasserstoffhaltigen Gas in Gegenwart eines Hydrierkatalysators unterzieht.

15. Verfahren nach Anspruch 14, wobei der in Schritt iii) eingesetzte Hydrierkatalysator ausgewählt ist unter
- Katalysatoren, die als Aktivmetall wenigstens ein Metall der VIII. Nebengruppe des Periodensystems der Elemente aufgebracht auf einem Träger umfassen, wobei jeweils 5 bis 50 % des Porenvolumens des Trägers von Makroporen mit einem Porendurchmesser im Bereich von 50 nm bis 10000 nm und 50 bis 95 % des Porenvolumens des Trägers von Mesoporen mit einem Porendurchmesser im Bereich von 2 bis weniger als 50 nm gebildet werden, wobei sich die Summe der Porenvolumina zu 100 % addiert, und
- Schalenkatalysatoren, enthaltend ein Aktivmetall ausgewählt unter Ruthenium, Rhodium, Palladium, Platin und Mischungen davon, aufgebracht auf ein Trägermaterial, enthaltend Siliziumdioxid, wobei das Porenvolumen des Trägermaterials 0,6 bis 1,0 mL/g, bestimmt durch Hg-Porosimetrie, beträgt, die BET-Oberfläche 280 bis 500 m²/g beträgt, und mindestens 90 % der vorhandenen Poren einen Durchmesser von 6 bis 12 nm aufweisen.

## Claims

1. A process for workup of a crude ester from the esterification of a benzenepolycarboxylic acid with at least one C₄-C₁₂ monoalkanol, wherein the crude ester additionally comprises
- at least one di-(C₄-C₁₂-alkyl) ether from the etherification of the at least one C₄-C₁₂ monoalkanol,
- optionally the at least one C₄-C₁₂ monoalkanol, and
- optionally water,
in which the crude ester is subjected to a thermal purification in at least one mass transfer apparatus by introducing a steam-containing gas stream in the region of the bottom of the mass transfer apparatus to obtain a bottom product enriched in the at least one benzenepolycarboxylic ester and depleted of the at least one di-(C₄-C₁₂-alkyl) ether and a vapor enriched in the at least one di-(C₄-C₁₂-alkyl) ether,
wherein the vapor is at least partly condensed, the condensate is separated into an aqueous phase and an organic phase comprising di-(C₄-C₁₂-alkyl) ether and C₄-C₁₂ monoalkanol, a portion of the organic phase is recycled as reflux stream into the thermal purification of the crude ester, and another portion of the organic phase is discharged.

2. The process according to claim 1, wherein a third portion of the organic phase is recycled into the esterification of the benzenepolycarboxylic acid with the at least one C₄-C₁₂ monoalkanol.

3. The process according to either of claims 1 and 2, wherein the discharge of a portion of the organic phase is batchwise or continuous.

4. The process according to any of the preceding claims, wherein the content in the bottom product of di-(C₄-C₁₂-alkyl) ether is controlled by implementing control interventions on at least one of the following manipulated variables:
- the mass flow of the reflux stream of the organic phase,
- the mass flow of the organic phase recycled into the esterification,
- the mass flow of the organic phase discharged.

5. The process according to claim 4, in which
- a target value for the content in the bottom product of di-(C₄-C₁₂-alkyl) ether and an upper and lower limit for the variance of the actual value from the target value are fixed,
- the actual value of the content in the bottom product of di-(C₄-C₁₂-alkyl) ether is determined,
- on attainment of the upper limit for the variance of the actual value from the target value, control interventions are implemented until the content in the bottom product of di-(C₄-C₁₂-alkyl) ether has fallen to the lower limit for the variance of the actual value from the target value.

6. The process according to claim 5, wherein the target value for the content in the bottom product of di-(C₄-C₁₂-alkyl) ether is not more than 1000 ppm by weight, preferably not more than 800 ppm by weight, particularly not more than 600 ppm by weight, especially not more than 500 ppm by weight.

7. A process for workup of a crude ester from the esterification of a benzenepolycarboxylic acid with at least one C₄-C₁₂ monoalkanol, wherein the crude ester additionally comprises
- at least one di-(C₄-C₁₂-alkyl) ether from the etherification of the at least one C₄-C₁₂ monoalkanol,
- optionally the at least one C₄-C₁₂ monoalkanol, and
- optionally water,
in which the crude ester is subjected to a thermal purification in at least one mass transfer apparatus by introducing a steam-containing gas stream in the region of the bottom of the mass transfer apparatus to obtain a bottom product enriched in the at least one benzenepolycarboxylic ester and depleted of the at least one di-(C₄-C₁₂-alkyl) ether and a vapor enriched in the at least one di-(C₄-C₁₂-alkyl) ether,
wherein the vapor is at least partly condensed, the condensate is separated into an aqueous phase and an organic phase comprising di-(C₄-C₁₂-alkyl) ether and C₄-C₁₂ monoalkanol, at least a portion of the organic phase is subjected to a separation into a di-(C₄-C₁₂-alkyl) ether-enriched fraction and a C₄-C₁₂ monoalkanol-enriched fraction, and the di-(C₄-C₁₂-alkyl) ether-enriched fraction is partly or fully discharged.

8. The process according to claim 7, wherein a portion of the organic phase is subjected to a separation into a di-(C₄-C₁₂-alkyl) ether-enriched fraction and a C₄-C₁₂ monoalkanol-enriched fraction, and another portion of the organic phase is recycled as reflux stream into the thermal purification of the crude ester.

9. The process according to claim 7 or 8, wherein the C₄-C₁₂ monoalkanol-enriched fraction is partly or fully recycled as reflux stream into the thermal purification of the crude ester.

10. The process according to any of claims 7 to 9, wherein the C₄-C₁₂ monoalkanol-enriched fraction is partly or fully recycled into the esterification of the benzenepolycarboxylic acid with the at least one C₄-C₁₂ monoalkanol.

11. The process according to any of the preceding claims, wherein the crude ester used for workup comprises
- 91% to 99.8% by weight of at least one ester of a benzenepolycarboxylic acid with at least one C₄-C₁₂ monoalkanol,
- 0.05% to 1% by weight of at least one di-(C₄-C₁₂-alkyl) ether,
- 0.1% to 5% by weight of at least one C₄-C₁₂ monoalkanol, and
- 0.05% to 3% by weight of water.

12. The process according to any of the preceding claims, wherein thermal purification is accomplished using at least one column having
- a side feed for the crude ester,
- a rectifying section above the feed point for the crude ester,
- a reflux stream for at least a portion of the condensed vapor above the rectifying section,
- a feed for the steam-containing gas stream in the region of the bottom of the column.

13. The process according to claim 12, wherein the rectifying section above the feed point for the crude ester has 0 to 10 theoretical plates, preferably 0 to 5 theoretical plates, particularly 0 to 2 theoretical plates.

14. A process for preparing a cyclohexanepolycarboxylic ester, in which
i) a crude ester from the esterification of a benzenepolycarboxylic acid with at least one C₄-C₁₂ monoalkanol is provided,
ii) the crude ester provided in step i) is subjected to a workup as defined in any of claims 1 to 13 to obtain a benzenepolycarboxylic ester depleted of di-(C₄-C₁₂-alkyl) ethers compared to the crude ester,
iii) the benzenepolycarboxylic ester depleted of di-(C₄-C₁₂-alkyl) ethers which is obtained in step ii) is subjected to a hydrogenation with a hydrogen-containing gas in the presence of a hydrogenation catalyst.

15. The process according to claim 14, wherein the hydrogenation catalyst used in step iii) is selected from
- catalysts comprising, as active metal, at least one metal from transition group VIII of the Periodic Table of the Elements applied to a support, where 5% to 50% of the pore volume of the support in each case is formed by macropores having a pore diameter in the range from 50 nm to 10 000 nm and 50% to 95% of the pore volume of the support by mesopores having a pore diameter in the range from 2 to less than 50 nm, wherein the sum total of the pore volumes adds up to 100%, and
- eggshell catalysts comprising an active metal selected from ruthenium, rhodium, palladium, platinum and mixtures thereof, applied to a support material comprising silicon dioxide, where the pore volume of the support material is 0.6 to 1.0 mL/g, determined by Hg porosimetry, the BET surface area is 280 to 500 m²/g, and at least 90% of the pores present have a diameter of 6 to 12 nm.

## Revendications

1. Procédé pour le traitement d'un ester brut provenant de l'estérification d'un acide benzènepolycarboxylique avec au moins un C₄-C₁₂-monoalcanol, l'ester brut contenant en outre
- au moins un di-(C₄-C₁₂-alkyl)éther provenant de l'éthérification dudit au moins un C₄-C₁₂-monoalcanol,
- le cas échéant ledit au moins un C₄-C₁₂-monoalcanol et
- le cas échéant de l'eau
dans lequel on soumet l'ester brut à une purification thermique dans au moins un appareil d'échange de matière par introduction d'un flux gazeux contenant de la vapeur d'eau dans la zone du fond de l'appareil d'échange de matière, avec obtention d'un produit de fond enrichi en ledit au moins un ester d'acide benzènepolycarboxylique et appauvri en ledit au moins un di-(C₄-C₁₂-alkyl) éther et de vapeurs enrichies en ledit au moins un di-(C₄-C₁₂-alkyl)éther,
dans lequel on condense au moins partiellement les vapeurs, on sépare le condensat en une phase aqueuse et en une phase organique, contenant du di-(C₄-C₁₂-alkyl)éther et du C₄-C₁₂-monoalcanol, on recycle une partie de la phase organique comme reflux dans la purification thermique de l'ester brut et on soutire une autre partie de la phase organique.

2. Procédé selon la revendication 1, dans lequel on recycle une troisième partie de la phase organique dans l'estérification de l'acide benzènepolycarboxylique avec ledit au moins un C₄-C₁₂-monoalcanol.

3. Procédé selon l'une des revendications 1 ou 2, dans lequel le soutirage d'une partie de la phase organique est réalisé de manière discontinue ou continue.

4. Procédé selon l'une des revendications précédentes, dans lequel on régule la teneur du produit de fond en di-(C₄-C₁₂-alkyl) éther en ce qu'on effectue des interventions de réglage sur au moins l'une des grandeurs de réglage suivantes :
- le flux massique du reflux de la phase organique,
- le flux massique de la phase organique recyclée dans l'estérification,
- le flux massique de la phase organique soutirée.

5. Procédé selon la revendication 4, dans lequel on
- fixe une valeur cible pour la teneur du produit de fond en di-(C₄-C₁₂-alkyl) éther et une valeur limite supérieure et inférieure pour l'écart de la valeur réelle par rapport à la valeur cible,
- détermine la valeur réelle de la teneur du produit de fond en di-(C₄-C₁₂-alkyl) éther,
- effectue, après avoir atteint la valeur limite supérieure pour l'écart de la valeur réelle par rapport à la valeur cible, des interventions de réglage jusqu'à ce que la teneur du produit de fond en di-(C₄-C₁₂-alkyl)éther soit abaissée à la valeur limite inférieure pour l'écart de la valeur réelle par rapport à la valeur cible.

6. Procédé selon la revendication 5, dans lequel la valeur cible pour la teneur du produit de fond en di-(C₄-C₁₂-alkyl)éther se situe à au maximum 1000 ppm en poids, de préférence à au maximum 800 ppm en poids, en particulier à au maximum 600 ppm en poids, spécialement à au maximum 500 ppm en poids.

7. Procédé pour le traitement d'un ester brut provenant de l'estérification d'un acide benzènepolycarboxylique avec au moins un C₄-C₁₂-monoalcanol, l'ester brut contenant en outre
- au moins un di-(C₄-C₁₂-alkyl)éther provenant de l'éthérification dudit au moins un C₄-C₁₂-monoalcanol,
- le cas échéant ledit au moins un C₄-C₁₂-monoalcanol et
- le cas échéant de l'eau
dans lequel on soumet l'ester brut à une purification thermique dans au moins un appareil d'échange de matière par introduction d'un flux gazeux contenant de la vapeur d'eau dans la zone du fond de l'appareil d'échange de matière, avec obtention d'un produit de fond enrichi en ledit au moins un ester d'acide benzènepolycarboxylique et appauvri en ledit au moins un di-(C₄-C₁₂-alkyl) éther et de vapeurs enrichies en ledit au moins un di-(C₄-C₁₂-alkyl)éther,
dans lequel on condense au moins partiellement les vapeurs, on sépare le condensat en une phase aqueuse et en une phase organique, contenant du di-(C₄-C₁₂-alkyl)éther et du C₄-C₁₂-monoalcanol, on soumet au moins une partie de la phase organique à une séparation en une fraction enrichie en di-(C₄-C₁₂-alkyl) éther et en une fraction enrichie en C₄-C₁₂-monoalcanol et on soutire partiellement ou complètement la fraction enrichie en di-(C₄-C₁₂-alkyl) éther.

8. Procédé selon la revendication 7, dans lequel on soumet une partie de la phase organique à une séparation en une fraction enrichie en di-(C₄-C₁₂-alkyl)éther et en une fraction enrichie en C₄-C₁₂-monoalcanol et on recycle une autre partie de la phase organique comme reflux dans la purification thermique de l'ester brut.

9. Procédé selon la revendication 7 ou 8, dans lequel on recycle partiellement ou complètement la fraction enrichie en C₄-C₁₂-monoalcanol comme reflux dans la purification thermique de l'ester brut.

10. Procédé selon l'une des revendications 7 à 9, dans lequel on recycle partiellement ou complètement la fraction enrichie en C₄-C₁₂-monoalcanol dans l'estérification de l'acide benzènepolycarboxylique avec ledit au moins un C₄-C₁₂-monoalcanol.

11. Procédé selon l'une des revendications précédentes, dans lequel l'ester brut utilisé pour le traitement contient
- 91 à 99,8% en poids d'au moins un ester d'un acide benzènepolycarboxylique avec au moins un C₄-C₁₂-monoalcanol,
- 0,05 à 1% en poids d'au moins un di-(C₄-C₁₂-alkyl)éther,
- 0,1 à 5% en poids d'au moins un C₄-C₁₂-monoalcanol et
- 0,05 à 3% en poids d'eau

12. Procédé selon l'une des revendications précédentes, dans lequel on utilise, pour la purification thermique, au moins une colonne qui présente
- une alimentation latérale pour l'ester brut,
- une partie d'amplification située au-dessus du site d'alimentation pour l'ester brut,
- un reflux situé au-dessus de la partie d'amplification pour au moins une partie des vapeurs condensées,
- une conduite d'alimentation pour le flux gazeux contenant la vapeur d'eau dans la zone du fond de la colonne

13. Procédé selon la revendication 12, dans lequel la partie d'amplification située au-dessus du site d'alimentation pour l'ester brut présente 0 à 10 plateaux théoriques de séparation, de préférence 0 à 5 plateaux théoriques de séparation, en particulier 0 à 2 plateaux théoriques de séparation.

14. Procédé de préparation d'un ester de l'acide cyclohexanepolycarboxylique, dans lequel on
i) met à disposition un ester brut provenant de l'estérification d'un acide benzènepolycarboxylique avec au moins un C₄-C₁₂-monoalcanol,
ii) soumet l'ester brut mis à disposition dans l'étape i) à un traitement tel que défini dans l'une des revendications 1 à 13, un ester de l'acide benzènepolycarboxylique appauvri en di-(C₄-C₁₂-alkyl)éthers par rapport à l'ester brut étant obtenu,
iii) soumet l'ester de l'acide benzènepolycarboxylique appauvri en di-(C₄-C₁₂-alkyl) éthers obtenu dans l'étape ii) à une hydrogénation avec un gaz contenant de l'hydrogène en présence d'un catalyseur d'hydrogénation.

15. Procédé selon la revendication 14, le catalyseur d'hydrogénation utilisé dans l'étape iii) étant choisi parmi
- les catalyseurs qui comprennent, comme métal actif, au moins un métal du VIIIème groupe secondaire du système périodique des éléments appliqué sur un support, à chaque fois 5 à 50% du volume des pores du support étant formés par des macropores présentant un diamètre de pore dans la plage de 50 nm à 10.000 nm et 50 à 95% du volume des pores du support étant formés par des mésopores présentant un diamètre de pore dans la plage de 2 à moins de 50 nm, la somme des volumes des pores s'additionnant à 100% et
- les catalyseurs enrobés, contenant un métal actif choisi parmi le ruthénium, le rhodium, le palladium, le platine et leurs mélanges, appliqué sur un matériau support, contenant du dioxyde de silicium, le volume des pores du matériau support représentant 0,6 à 1,0 ml/g, déterminé par porosimétrie de Hg, la surface BET représentant 280 à 500 m²/g et au moins 90% des pores présents présentant un diamètre de 6 à 12 nm.
